# EUROPEAN PATENT APPLICATION

(11) **EP 4 692 083 A1**
(43) Date of publication of application: **11.02.2026**
(21) Application number: 24781317.3
(22) Date of filing: 29.03.2024
(51) Int. Cl.: C07D 413/14, A61K 31/506

(54) **NOVEL AZABICYCLE DERIVATIVES AND PHARMACEUTICAL COMPOSITION FOR INHIBITING AUTOTAXIN, COMPRISING SAME**

(30) Priority: 31.03.2023 KR 20230043184
(71) Applicant: Nextgen Bioscience Co., Ltd., Seongnam-si, Gyeonggi-do 13487 (KR)
(72) Inventor: LEE, Bong Yong, Seoul 06625 (KR); SHIN, Young Ah, Seongnam-si, Gyeonggi-do 13639 (KR); KIM, Shin Ae, Suwon-si, Gyeonggi-do 16509 (KR); LIM, Eun Jin, Seongnam-si, Gyeonggi-do 13489 (KR); KANG, Soo Sung, Bucheon-si, Gyeonggi-do 14535 (KR); KIM, Ji Ho, Bucheon-si, Gyeonggi-do 14424 (KR); YANG, Su Jae, Incheon 21998 (KR); JEONG, Min Jeong, Seoul 04137 (KR)
(74) Representative: Graf von Stosch Patentanwaltsgesellschaft mbH
(86) International application number: PCT/KR2024/004088
(87) International publication number: WO 2024/205327

(57) **Abstract**

The present invention provides: a novel azabicycle derivative compound, a hydrate thereof, a solvate thereof, or a pharmaceutically acceptable salt thereof; and a pharmaceutical composition for prevention or treatment of autotaxin-related disease, comprising same as an active ingredient. The azabicycle derivative compound of the present invention exhibits excellent inhibitory activity against autotaxin and thus may be usefully employed for the treatment and prevention of diseases related to autotaxin inhibition, such as fibrotic disease, inflammatory disease, autoimmune disease, respiratory disease, cardiovascular disease, metabolic disease, cancer and cancer metastasis, ocular disease, cholestatic and other forms of chronic pruritus, and acute or chronic organ transplant rejection.

## Description

### TECHNICAL FIELD

This application claims priority to Korean Patent Application No. 10-2023-0043184, filed on March 31, 2023. The entire disclosure in the specification of which is incorporated herein by reference. The present invention relates to novel azabicycle derivatives, and more particularly, to novel azabicycle derivatives having autotaxin inhibitory activity.

### BACKGROUND ART

Autotaxin (ATX), also known as ectonucleotide pyrophosphatase/phosphodiesterase family member 2 (ENPP2), is a secreted enzyme essential for the production of the lipid signaling molecule lysophosphatidic acid (LPA). Autotaxin exhibits lysophospholipase D activity, which converts lysophosphatidylcholine (LPC) to LPA. Therefore, LPA levels in plasma and ascites are correlated with ATX activity.

Plasma LPA is a bioactive lipid that influences the migration, proliferation, and survival of various cell types. In addition, the ATX-LPA signaling process is involved in the physiological and pathophysiological functions of various diseases, including nervous system function, vascular development, cardiovascular physiology, tissue regeneration, immune system function, chronic inflammation, tumor metastasis and progression, organ fibrosis, and obesity and/or other metabolic diseases (e.g., diabetes mellitus).

Therefore, increased ATX activity and increased LPA levels, altered LPA receptor expressions, and altered responses to LPA may be associated with the initiation, progression, and/or outcome of various pathophysiological diseases involving the ATX/LPA signaling pathway. In particular, it is known to be associated with cancer, lymphocyte homing, chronic inflammation, neuropathic pain, fibrotic diseases (e.g., idiopathic pulmonary fibrosis, IPF), and thrombosis. Accordingly, lowering the levels of LPA and/or autotaxin (ATX), which induces it, is necessary to treat these diseases.

### DISCLOSURE

### OBJECT OF THE INVENTION

The problem to be addressed by the present invention is to provide novel structured autotaxin inhibitory compounds that exhibit excellent inhibitory activity against autotaxin.

Further, the problem to be addressed by the present invention is to provide a pharmaceutical composition for preventing or treating an autotaxin-related disease, comprising an autotaxin inhibitory compound having novel structure.

Further, the problem to be addressed by the present invention is to provide a method for preventing or treating an autotaxin-related disease which comprises administering a therapeutically effective amount of an autotaxin inhibitory compound having novel structure to a subject in need thereof.

Further, the problem to be addressed by the present invention is to provide a use of the novel structured autotaxin inhibitory compound as an effective ingredient in the manufacture of a medicament for preventing or treating autotaxin-related diseases.

The problems to be addressed by the present invention are not limited to the problems mentioned above, and other technical problems not mentioned can be clearly understood by a person having ordinary skill in the technical field to which the present invention belongs from the description below.

### Technical Solution

In order to address the above problem, according to one aspect of the present invention, there is provided an azabicycle derivative compound represented by the following Formula 1, a hydrate thereof, a solvate thereof or a pharmaceutically acceptable salt thereof:

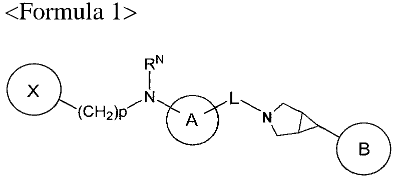

wherein,
X is C₅₋₇ aryl; 5- or 6-membered heteroaryl having 1 to 3 N; or a fused bicyclic ring in which C₅₋₇ aryl ring is fused with a 5- or 6-membered cycloalkyl ring having 0 to 3 O, wherein X is optionally substituted with one or more independent R^{x} ,
wherein R ^{x} is C₁₋₄ alkyl, C₁₋₄ alkoxy, cyano, halogen or C₁₋₄ haloalkyl,
p is an integer from 0 to 3,
R^{N} is hydrogen or C₁₋₄ alkyl,
A is a 5- or 6-membered heteroaryl having 1 to 3 heteroatoms selected from N and S, wherein A is optionally substituted with one or more independent R^{A},
wherein R^{A} is C₁₋₄ alkyl,
L is -(CH₂)ₘC(O)-, -CHCHC(O)-, or a 5- or 6-membered aromatic or non-aromatic heterocycle having 1 to 4 heteroatoms selected from N and O, wherein m is an integer from 1 to 5, and
B is -(CH₂)ₙC(O)OH, or a 5- or 6-membered heteroaryl having 1 to 4 N, wherein n is an integer from 1 to 3, wherein B is optionally substituted with one or more independent R^{B},
wherein R^{B}, is C₁₋₄ alkyl.

According to another aspect of the present invention, there is provided a pharmaceutical composition for preventing or treating an autotaxin-related disease, comprising an azabicycle derivative compound represented by the Formula 1, a hydrate thereof, a solvate thereof or a pharmaceutically acceptable salt thereof, as an active ingredient.

According to another aspect of the present invention, there is provided a method for preventing or treating an autotaxin-related disease which comprises administering a therapeutically effective amount of an azabicycle derivative compound represented by the Formula 1, a hydrate thereof, a solvate thereof or a pharmaceutically acceptable salt thereof, to a subject in need thereof.

According to another aspect of the present invention, there is provided a use of an azabicycle derivative compound represented by the Formula 1, a hydrate thereof, a solvate thereof or a pharmaceutically acceptable salt thereof, to a subject in need thereof, as an effective ingredient for the manufacture of a medicine for preventing or treating an autotaxin-related disease.

According to another aspect of the present invention, there is provided a pharmaceutical composition comprising an azabicycle derivative compound represented by the Formula 1, a hydrate thereof, a solvate thereof or a pharmaceutically acceptable salt thereof, and a pharmaceutically acceptable additive.

### Effects of the Invention

It was confirmed that the novel azabicycle derivative compound of the present invention exhibits excellent autotaxin inhibitory activity, and thus it was found that it can be used for the prevention or treatment of autotaxin-related diseases.

Therefore, the novel structural azabicycle derivative compound of the present invention can be usefully used in the fields of medicine and pharmacy for the treatment and prevention of autotaxin-related diseases such as fibrotic diseases, inflammatory diseases, autoimmune diseases, respiratory diseases, cardiovascular diseases, metabolic diseases, cancer and cancer metastasis, ocular diseases, cholestatic forms and other forms of chronic pruritus, and acute or chronic organ transplant rejection.

The effects of the present invention are not limited to the effects described above, and should be understood to include all effects that can be inferred from the composition of the invention described in the description or claims of the present invention.

### DETAILED DESCRIPTION OF THE INVENTION

In this specification, autotaxin (ATX) is a secreted enzyme that plays an important role in the production of lysophosphatidic acid (LPA), also referred to as ectonucleotide pyrophosphatase/phosphodiesterase family member 2 (ENPP2). Autotaxin exhibits lysophospholipase D activity, which converts lysophosphatidylcholine (LPC) to LPA. Therefore, LPA levels in plasma and ascites are associated with ATX activity.

The present invention provides an azabicycle derivative compound represented by the following Formula 1, a hydrate thereof, a solvate thereof or a pharmaceutically acceptable salt thereof:

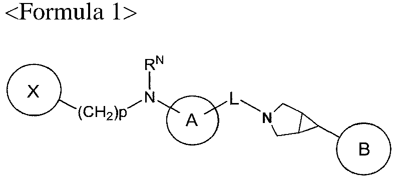

wherein,
X is C₅₋₇ aryl; 5- or 6-membered heteroaryl having 1 to 3 N; or a fused bicyclic ring in which C₅₋₇ aryl ring is fused with a 5- or 6-membered cycloalkyl ring having 0 to 3 O, wherein X is optionally substituted with one or more independent R^{x} ,
wherein R ^{x} is C₁₋₄ alkyl, C₁₋₄ alkoxy, cyano, halogen or C₁₋₄ haloalkyl,
p is an integer from 0 to 3,
R^{N} is hydrogen or C₁₋₄ alkyl,
A is a 5- or 6-membered heteroaryl having 1 to 3 heteroatoms selected from N and S, wherein A is optionally substituted with one or more independent R^{A},
wherein R^{A} is C₁₋₄ alkyl,
L is -(CH₂)ₘC(O)-, -CHCHC(O)-, or a 5- or 6-membered aromatic or non-aromatic heterocycle having 1 to 4 heteroatoms selected from N and O, wherein m is an integer from 1 to 5, and
B is -(CH₂)ₙC(O)OH, or a 5- or 6-membered heteroaryl having 1 to 4 N, wherein n is an integer from 1 to 3, wherein B is optionally substituted with one or more independent R^{B},
wherein R^{B}, is C₁₋₄ alkyl.

In one embodiment, X may be phenyl, pyridinyl, dihydroindenyl or benzodioxolyl.

In one embodiment, R^{x} may be methyl, methoxy, cyano, fluoro, chloro, bromo, difluoromethyl or trifluoromethyl.

In one embodiment, p may be 0, 1 or 2.

In one embodiment, R^{N} may be hydrogen or methyl.

In one embodiment, A may be pyridinyl, pyrimidinyl, pyrazinyl or thiadiazolyl.

In one embodiment, R^{A} may be methyl.

In one embodiment, L may be -(CH₂)₂C(O)-, -(CH₂)₃C(O)-, -CHCHC(O)-, dihydroisoxazolyl or oxadiazolyl.

In one embodiment, B may be -CH₂C(O)OH or triazolyl.

In one embodiment, R^{B} may be methyl.

Representative examples of the azabicycle derivative compound according to the present invention are as follows:
[1] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-benzylpyrimidin-2-amine,
[2] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-chlorobenzyl)pyrimidin-2-amine,
[3] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorobenzyl)pyrimidin-2-amine,
[4] 5-(5-((1*R*,5*R*,6*r*)-6-(1*H*-1,2,3-Triazol-5-yl)-3-azabicyclo[3.1.0]hexan-3-yl)-1,3,4-oxadiazol-2-yl)-*N*-(3,4-difluorobenzyl)pyrimidin-2-amine,
[5] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-difluorobenzyl)pyrimidin-2-amine,
[6] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,4-dichlorobenzyl)pyrimidin-2-amine,
[7] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-dichlorobenzyl)pyrimidin-2-amine,
[8] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-dibromobenzyl)pyrimidin-2-amine,
[9] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(benzo[d][1,3]dioxol-5-ylmethyl)pyrimidin-2-amine,
[10] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-bis(trifluoromethyl)benzyl)pyrimidin-2-amine,
[11] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-phenethylpyrimidin-2-amine,
[12] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-fluorophenethyl)pyrimidin-2-amine,
[13] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-fluorophenethyl)pyrimidin-2-amine,
[14] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-chlorophenethyl)pyrimidin-2-amine,
[15] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)pyrimidin-2-amine,
[16] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2,3-dihydro-1*H*-inden-2-yl)pyrimidin-2-amine,
[17] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(5,6-difluoro-2,3-dihydro-1*H*-inden-2-yl)pyrimidin-2-amine,
[18] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(5-bromo-2,3-dihydro-1*H*-inden-2-yl)pyrimidin-2-amine,
[19] 1-((1*R*,5*S*,6*r*)-6-(1*H*-1,2,3-Triazol-5-yl)-3-azabicyclo[3.1.0]hexan-3-yl)-3-(2-((2,3-dihydro-1*H*-inden-2-yl)amino)pyrimidin-5-yl)propan-1-one,
[20] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-(trifluoromethyl)benzyl)pyrimidin-2-amine,
[21] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-(trifluoromethyl)benzyl)pyrimidin-2-amine,
[22] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2-(trifluoromethyl)benzyl)pyrimidin-2-amine,
[23] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-(difluoromethyl)benzyl)pyrimidin-2-amine,
[24] 4-(((5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)pyrimidin-2-yl)amino)methyl)benzonitrile,
[25] 3-(((5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)pyrimidin-2-yl)amino)methyl)benzonitrile,
[26] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-(trifluoromethyl)phenethyl)pyrimidin-2-amine,
[27] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-bromophenethyl)pyrimidin-2-amine,
[28] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2-chlorophenethyl)pyrimidin-2-amine,
[29] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-dichlorophenethyl)pyrimidin-2-amine,
[30] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-methoxyphenethyl)pyrimidin-2-amine,
[31] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-methylphenethyl)pyrimidin-2-amine,
[32] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2-(pyridin-2-yl)ethyl)pyrimidin-2-amine,
[33] 4-(2-((5-(5-((1*R*,5*S,*6*r*)-6-(1*H*-1,2,3-Triazol-5-yl)-3-azabicyclo[3.1.0]hexan-3-yl)-1,3,4-oxadiazol-2-yl)pyrimidin-2-yl)amino)ethyl)benzonitrile,
[34] 5-(5-((1*R*,5*S*,6*R*)-6-(1*H*-1,2,3-Triazol-5-yl)-3-azabicyclo[3.1.0]hexan-3-yl)-1,3,4-oxadiazol-2-yl)-*N*-((R)-2,3-dihydro-1*H*-inden-1-yl)pyrimidin-2-amine,
[35] 5-(5-((1*R*,5*S*,6*S*)-6-(1*H*-1,2,3-Triazol-5-yl)-3-azabicyclo[3.1.0]hexan-3-yl)-1,3,4-oxadiazol-2-yl)-*N*-((S)-2,3-dihydro-1*H*-inden-1-yl)pyrimidin-2-amine,
[36] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-phenylpyrimidin-2-amine,
[37] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-N-(4-chlorophenethyl)-N-methylpyrimidin-2-amine,
[38] 1-((1*R*,5*S*,6*r*)-6-(1*H*-1,2,3-Triazol-5-yl)-3-azabicyclo[3.1.0]hexan-3-yl)-4-(5-((2,3-dihydro-1*H*-inden-2-yl)amino)-1,3,4-thiadiazol-2-yl)butan-1-one,
[39] 1-((1*R*,5*S*,6*r*)-6-(1*H*-1,2,3-Triazol-5-yl)-3-azabicyclo[3.1.0]hexan-3-yl)-3-(5-((2,3-dihydro-1*H*-inden-2-yl)amino)-1,3,4-thiadiazol-2-yl)propan-1-one,
[40] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)-4-methylpyrimidin-2-amine,
[41] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)pyridin-2-amine,
[42] 5-(5-((1*R*,5*S*,6r)-6-(1*H*-1,2,3-Triazol-5-yl)-3-azabicyclo[3.1.0]hexan-3-yl)-1,3,4-oxadiazol-2-yl)-*N*-(4-chlorophenethyl)pyrazin-2-amine,
[43] 1-((1*R*,5*S*,6*r*)-6-(1*H*-1,2,3-Triazol-5-yl)-3-azabicyclo[3.1.0]hexan-3-yl)-3-(2-((4-chlorophenethyl)amino)pyrimidin-5-yl)propan-1-one,
[44] 1-((1*R*,5*S*,6*r*)-6-(1*H*-1,2,3-Triazol-5-yl)-3-azabicyclo[3.1.0]hexan-3-yl)-4-(2-((3,5-dichlorobenzyl)amino)pyrimidin-5-yl)butan-1-one,
[45] (*E*)-1-((1*R*,5*S*,6*r*)-6-(1*H*-1,2,3-Triazol-5-yl)-3-azabicyclo[3.1.0]hexan-3-yl)-3-(2-((4-chlorophenethyl)amino)pyrimidin-5-yl)prop-2-en-1-one,
[46] (*E*)-1-((1*R*,5*S*,6*r*)-6-(1*H*-1,2,3-Triazol-5-yl)-3-azabicyclo[3.1.0]hexan-3-yl)-3-(2-((2,3-dihydro-1*H*-inden-2-yl)amino)pyrimidin-5-yl)prop-2-en-1-one,
[47] *N*-(4-Chlorophenethyl)-5-(5-((1*R*,5*S*,6*r*)-6-(1-methyl-1*H*-1,2,3-triazol-5-yl)-3-azabicyclo[3.1.0]hexan-3-yl)-1,3,4-oxadiazol-2-yl)pyrimidin-2-amine,
[48] *N*-(4-Chlorophenethyl)-5-(5-((1*R*,5*S*,6*r*)-6-(4-methyl-1*H*-1,2,3-triazol-5-yl)-3-azabicyclo[3.1.0]hexan-3-yl)-1,3,4-oxadiazol-2-yl)pyrimidin-2-amine,
[49] 5-(3-((1*R*,5*S*,6*r*)-6-(1*H*-1,2,3-Triazol-5-yl)-3-azabicyclo[3.1.0]hexan-3-yl)-4,5-dihydroisoxazol-5-yl)-*N*-(4-chlorophenethyl)pyrimidin-2-amine,
[50] 5-(3-((1*R*,5S*,*6*r*)-6-(1*H*-1,2,3-Triazol-5-yl)-3-azabicyclo[3.1.0]hexan-3-yl)-4,5-dihydroisoxazol-5-yl)-*N*-(2,3-dihydro-1*H*-inden-2-yl)pyrimidin-2-amine, and
[51] 2-((1*R*,5*S*,6*s*)-3-(5-(2-((4-Chlorophenethyl)amino)pyrimidin-5-yl)-1,3,4-oxadiazol-2-yl)-3-azabicyclo[3.1.0]hexan-6-yl)acetic acid.

This specification uses the following definitions when defining the compound of Formula 1 unless specifically defined.

The term "alkyl" refers to a straight-chain or branched-chain saturated hydrocarbon, preferably C ₁₋₁₀ alkyl. Examples of alkyl include, but are not limited to, methyl, ethyl, *n*-propyl, *iso*-propyl, *n*-butyl, *iso*-butyl, *tert*-butyl, *n*-pentyl, *iso*-pentyl, *n-*hexyl, 3-methylhexyl, 2,2-dimethylpentyl, 2,3-dimethylpentyl, *n*-heptyl, *n*-octyl, *n*-nonyl, *n*-decyl, etc..

The term "cycloalkyl" refers to a partially or fully saturated single or fused ring hydrocarbon, preferably C₃₋₁₀ monocyclic, bicyclic or tricyclic functional group. Examples of monocyclic functional groups in cycloalkyl include cyclopropyl, cyclobutyl, cyclopentyl, cyclohexyl, cyclohexynyl and the like. Bicyclic functional groups in cycloalkyl include bicycloalkyl and spiro functional groups, and specific examples include bicyclo[1.1.1]pentanyl, bicyclo[3.1.0]hexanyl, bicyclo[2.1.1]hexanyl, bicyclo[2.2.1]heptanyl, spiro[2.3]hexanyl, spiro[3.3]heptanyl and the like.

The term "hydroxy" or "hydroxyl" is defined as -OH, and the term "alkoxy", unless otherwise defined, means alkyloxy, a radical in which the hydrogen atoms of the hydroxy group are replaced by C₁₋₁₀ alkyl.

The term "halogen" or "halo" refers to fluorine (F), chlorine (Cl), bromine (Br), or iodine (I).

The terms "haloalkyl" and "haloalkoxy" mean alkyl or alkoxy substituted with one or more halogen atoms.

The term "heteroatom" means N, O, or S.

The term "aryl" means an aromatic hydrocarbon, including a polycyclic aromatic ring system in which a carbocyclic aromatic ring or a heteroaryl ring is fused with one or more other rings. Preferably it is C₅₋₁₂ aryl, more preferably C₅₋₁₀ aryl. For example, the aryl includes, but is not limited to, phenyl, naphthyl, tetrahydronaphthyl, and the like.

The term "heteroaryl" or "aromatic heterocycle" means a 3- to 12-membered, more preferably 5- to 10-membered aromatic hydrocarbon forming a single or fused ring that contains one or more heteroatoms selected from N, O and S as ring atoms, and that can be fused with a benzo or C₃-₈ cycloalkyl. For example, heteroaryl includes, but are not limited to, pyrrolyl, imidazolyl, pyrazolyl, triazolyl, pyridyl, pyrazinyl, pyrimidyl, pyridazinyl, triazinyl, oxadiazolyl, isoxadiazolyl, tetrazolyl, indolyl, indazolyl, isoxazolyl, oxazolyl, thiazolyl, isothiazolyl, furanyl, benzofuranyl, thiophenyl, benzothiophenyl, benzothiazolyl, benzooxazolyl, benzimidazolyl, quinolinyl, isoquinolinyl, etc..

Additionally, the heteroaryl includes groups in which the heteroaryl ring is fused to a cycloalkyl or a non-aromatic heterocyclic ring, for example, dihydrocyclopentapyrazinyl, and the like.

The term "heterocycle" or "heterocycloalkyl" means is a non-aromatic carbocyclic ring containing one or more heteroatoms, such as N, O or S, within the ring. The ring may be 5, 6, 7 or 8-membered and/or may be fused to another ring, such as a cycloalkyl or an aromatic ring. Examples of such compounds include pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, oxathiolanyl, dithiolanyl, piperidinyl, tetrahydropyranyl, thianyl, piperazinyl, morpholino, and dioxanyl.

The compound represented by the Formula 1 according to the present invention can be made and used in the form of a prodrug, a hydrate, a solvate, and a pharmaceutically acceptable salt in order to promote *in vivo* absorption or increase solubility, and therefore the prodrug, hydrate, solvate, and pharmaceutically acceptable salt also fall within the scope of the present invention. Furthermore, the compound represented by the Formula 1 has a chiral carbon, and thus its stereoisomers exist, and such stereoisomers are also included within the scope of the present invention.

The term "prodrug" refers to a substance that is transformed *in vivo* into the parent drug. Prodrugs are often used because, in some cases, they are easier to administer than the parent drug. For example, they may be bioactive by oral administration, whereas the parent drug may not be. Prodrugs may also have improved solubility in pharmaceutical compositions than the parent drug. For example, prodrugs may be *in vivo* hydrolysable esters of compounds according to the invention and pharmaceutically acceptable salts thereof. Another example of a prodrug may be a short peptide (polyamino acid) that is linked to an acid group that is metabolized to reveal the active site of the peptide.

The term "hydrate" refers to a compound of the present invention or a salt thereof having a stoichiometric or non-stoichiometric amount of water bound by non-covalent intermolecular forces.

The term "solvate" refers to a compound of the present invention or a salt thereof having a stoichiometric or non-stoichiometric amount of solvent bound by non-covalent intermolecular forces. Preferred solvents therefor include solvents that are volatile, non-toxic, and/or suitable for administration to humans.

The term "isomer" refers to a compound of the present invention or a salt thereof having the same formula or molecular formula but structurally or sterically different. Such isomers include structural isomers such as tautomers, and stereoisomers such as R or S isomers having an asymmetric carbon center, and geometric isomers (trans, cis). All of these isomers and mixtures thereof are also included in the scope of the present invention. In particular, when the compound of Formula 1 has optical isomers, stereoisomers, regioisomers, or rotamers, these are also included in the compound of Formula 1, and can be obtained as a single product according to synthetic and separation methods known in the art. For example, when the compound of Formula 1 includes optical isomers, optical isomers resolved from this compound are also included in the compound of Formula 1. Optical isomers can be prepared by methods known per se.

The term "pharmaceutically acceptable salt" means a salt form of a compound which does not cause serious irritation to the organism to which the compound is administered and does not impair the biological activity and physical properties of the compound. The pharmaceutical salts include acid addition salts formed by acids which form non-toxic acid addition salts containing pharmaceutically acceptable anions, for example, inorganic acids such as hydrochloric acid, sulfuric acid, nitric acid, phosphoric acid, hydrobromic acid, hydroiodic acid, etc., organic carboxylic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc., sulfonic acids such as methanesulfonic acid, ethanesulfonic acid, benzenesulfonic acid, p-toluenesulfonic acid, etc.. For example, pharmaceutically acceptable carboxylic acid salts include metal salts or alkaline earth metal salts formed by lithium, sodium, potassium, calcium, magnesium, etc.; amino acid salts such as lysine, arginine, guanidine, etc.; organic salts such as dicyclohexylamine, N-methyl-D-glucamine, tris(hydroxymethyl)methylamine, diethanolamine, choline, and triethylamine. The compound of Formula 1 according to the present invention can be converted into its salt by a conventional method.

In addition, the present invention provides a method for preparing the compound of Formula 1. The synthetic methods of Examples 1 to 51 are exemplified as a method for preparing the compound of Formula 1 of the present invention, and the synthetic methods of Examples 1 to 51 do not limit the method for preparing the compound of Formula 1 according to the present invention. The synthetic methods of Examples 1 to 51 are merely examples, and it is obvious that they can be easily modified by those skilled in the art depending on a specific substituent.

The present invention also provides a pharmaceutical composition for preventing or treating an autotaxin-related disease, comprising an azabicycle derivative compound represented by the Formula 1, a hydrate thereof, a solvate thereof or a pharmaceutically acceptable salt thereof, as an active ingredient.

The present invention also provides a method for preventing or treating an autotaxin-related disease which comprises administering a therapeutically effective amount of an azabicycle derivative compound represented by the Formula 1, a hydrate thereof, a solvate thereof or a pharmaceutically acceptable salt thereof, to a subject in need thereof.

The present invention also provides a use of an azabicycle derivative compound represented by the Formula 1, a hydrate thereof, a solvate thereof or a pharmaceutically acceptable salt thereof as an effective ingredient for the manufacture of a medicine for preventing or treating an autotaxin-related disease.

As a result of measuring the autotaxin protein inhibitory activity of the azabicycle derivative compound of the present invention, it was confirmed that excellent autotaxin inhibitory activity was exhibited even at a very low concentration (nM level) of the compound, and thus it can be used for the treatment and prevention of autotaxin-related diseases.

In one embodiment, the autotaxin-related disease may be selected from the group consisting of fibrotic diseases, inflammatory diseases, autoimmune diseases, respiratory diseases, cardiovascular diseases, metabolic diseases, cancer and cancer metastasis, ocular diseases, cholestatic form and other forms of chronic pruritus, and acute or chronic organ transplant rejection.

The above fibrotic diseases include, but are not limited to, idiopathic pulmonary fibrosis (IPF), interstitial lung disease, liver fibrosis, liver cirrhosis, non-alcoholic steatohepatitis, radiation-induced fibrosis, renal fibrosis, cutaneous fibrosis, glomerulosclerosis, myocardial and vascular fibrosis.

The above inflammatory diseases include, but are not limited to, rheumatoid arthritis, osteoarthritis, atopic dermatitis, inflammatory bowel disease, inflammatory airway disease, chronic obstructive pulmonary disease (COPD) and asthma.

The above autoimmune diseases include, but are not limited to, multiple sclerosis and scleroderma.

The above respiratory diseases include, but are not limited to, asbestos-induced pulmonary fibrosis and acute respiratory distress syndrome (ARDS).

The above cardiovascular diseases include, but are not limited to, arteriosclerosis, myocardial infarction, arterial and pulmonary hypertension, cardiac arrhythmias, stroke and other vascular damage.

The above metabolic diseases include, but are not limited to, obesity and diabetes.

The above cancers and cancer metastases include, but are not limited to, breast cancer, ovarian cancer, lung cancer, prostate cancer, mesothelioma, glioma, hepatocarcinoma, gastrointestinal cancer, pancreatic cancer, and their progression and metastatic invasion.

The above ocular diseases include, but are not limited to, proliferative and nonproliferative retinopathy, diabetic retinopathy, dry and wet age-related macular degeneration (AMD), macular edema, central artery/vein occlusion, traumatic injury, and glaucoma.

The present invention also provides a pharmaceutical composition or formulation comprising an azabicycle derivative compound represented by the Formula 1, a hydrate thereof, a solvate thereof or a pharmaceutically acceptable salt thereof, and a pharmaceutically acceptable additive.

The above additives may include pharmaceutically acceptable carriers such as commonly used excipients, disintegrants, sweeteners, lubricants or flavoring agents, and may be formulated into oral preparations such as tablets, capsules, powders, granules and suspensions, emulsions or syrups according to conventional methods; or parenteral preparations such as external solutions, external suspensions, external emulsions, gels (ointments, etc.), inhalants, sprays and injections. The preparations may be formulated into various forms, for example, single-dose or multiple-dose dosage forms.

The subject to which the pharmaceutical composition of the present invention is administered may be, for example, a human, a monkey, a cow, a horse, a sheep, a pig, a chicken, a turkey, a cat, a dog, a mouse, a rat, a rabbit or a guinea pig, and may be, for example, a mammal, for example, a human, but is not limited thereto.

In addition, the pharmaceutical composition of the present invention can be administered orally or parenterally, and in case of parenteral administration, it can be administered by routes such as skin, transdermally, ocularly, intraperitoneally, rectal, or intravenous, intramuscular, subcutaneous, intrauterine epidural, intracerebroventricular injection, or topical administration. Topical ocular administration includes, for example, direct administration into the eye, or administration around the eye, behind the eye, subretinal, central retinal, outside the fovea, subconjunctival, intravitreous, intracameral, or suprachoroidal. The pharmaceutical composition can be administered through an insertion device.

The dosage of the active ingredient contained in the pharmaceutical composition of the present invention varies depending on the patient's condition and weight, the degree of the disease, the form of the active ingredient, the route and period of administration, and may be appropriately adjusted depending on the patient. For example, the active ingredient may be administered at a dosage of 0.0001 to 1000 mg/kg per day, preferably 0.001 to 100 mg/kg, and the dose may be administered once a day or divided into several times. In addition, the pharmaceutical composition of the present invention may contain the active ingredient at a weight percentage of 0.001 to 90% based on the total weight of the composition.

Hereinafter, the present invention will be described in more detail through Synthetic examples, Examples, and Test examples. However, the following Synthetic examples, Examples, and Tst examples are intended to illustrate the present invention, and the scope of the present invention is not limited thereto.

### Synthetic method

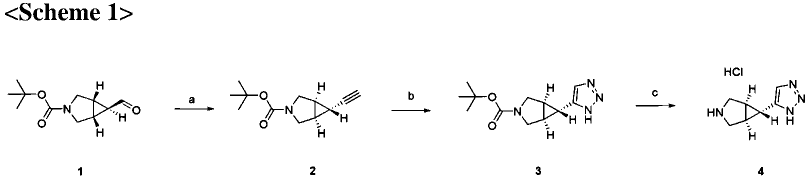

Reagents and conditions: (a) Bestmann-Ohira reagent, K₂CO₃, MeOH, r,t.; (b) Me₃SiN₃, CuI, DMF, 100 °C, 12 h; (c) dioxane containing 4N HCl, r.t., 2 h

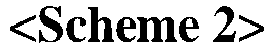

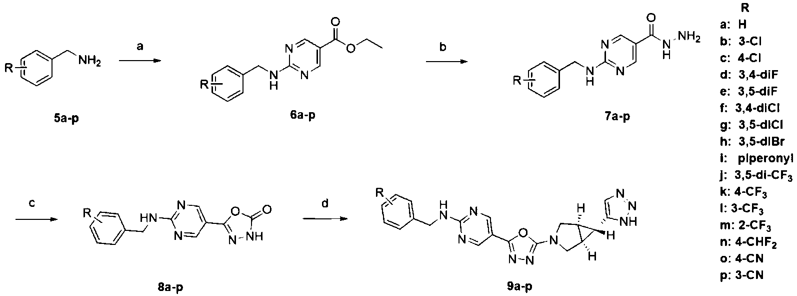

Reagents and conditions: (a) ethyl 2-chloropyrimidine-5-carboxylate, DIEA, DMF, 100 °C, 3 h or ethyl 2-chloropyrimidine-5-carboxylate, DIEA, 1,4-dioxane, 100 °C, 2 h; (b) N₂H₄·H₂O, EtOH, reflux, 8~15 h; (c) triphosgene, TEA, THF, DMF, r.t., 2 h or 1,1'-carbonyldiimidazole (CDI), THF, 0°C(15min)->r.t., 20 h; (d) (1*R*,5*S*,6*r*)-6-(1*H*-1,2,3-triazol-5-yl)-3-azabicyclo[3.1.0]hexane hydrochloride, BOP reagent, DIEA, DMF, r.t., 8~22 h

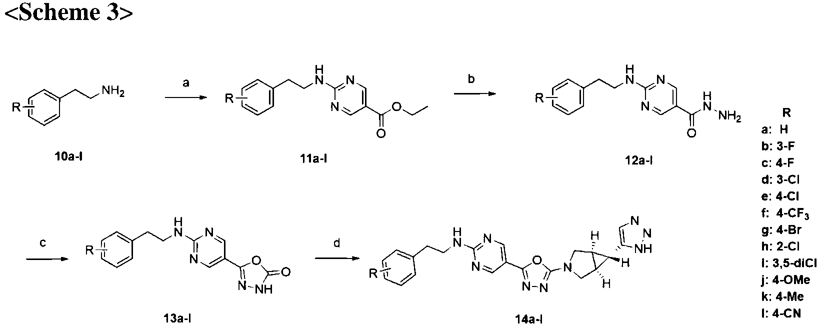

Reagents and conditions: (a) ethyl 2-chloropyrimidine-5-carboxylate, DIEA, DMF, 100 °C, 3 h or ethyl 2-chloropyrimidine-5-carboxylate, DIEA, 1,4-dioxane, 100 °C, 2 h; (b) N₂H₄· H₂O, EtOH, reflux, 8~15 h; (c) triphosgene, TEA, THF, DMF, r.t., 2 h or 1,1'-carbonyldiimidazole (CDI), THF, 0°C(15min)->r.t., 20 h; (d) (1*R*,5*S*,6*r*)-6-(1*H*-1,2,3-triazol-5-yl)-3-azabicyclo[3.1.0]hexane hydrochloride, BOP reagent, DIEA, DMF, r.t., 8~22 h

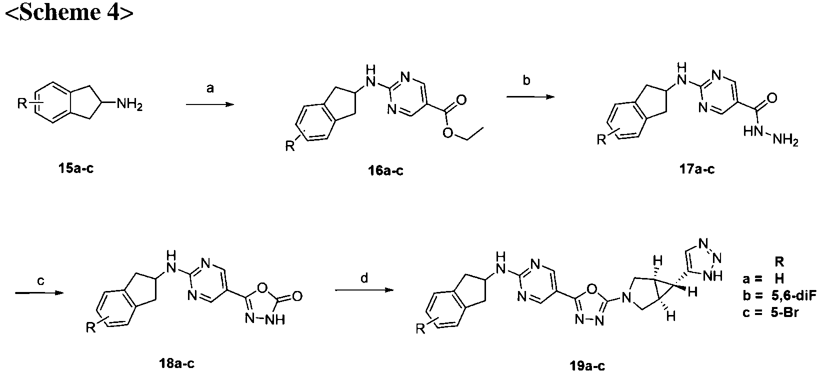

Reagents and conditions: (a) ethyl 2-chloropyrimidine-5-carboxylate, DIEA, DMF, 100 °C, 3 h;(b) N₂H₄· H₂O, EtOH, reflux, 8 h; (c) triphosgene, TEA, THF, DMF, r.t., 2 h; (d) (1*R*,5*S*,6*r*)-6-(1*H*-1,2,3-triazol-5-yl)-3-azabicyclo[3.1.0]hexane hydrochloride, BOP reagent, DIEA, DMF, r.t., 8 h

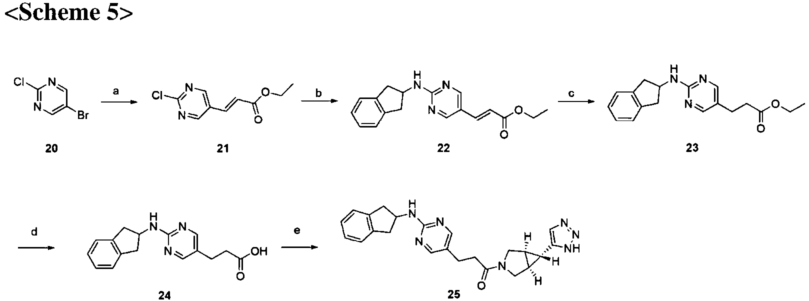

Reagents and conditions: (a) ethyl acrylate, Pd(OAc)₂, tri(o-tolyl)phosphine, DIEA, DMF, reflux, 4 h; (b) 2-aminoindane, Et₃N, dioxane, 100 °C, 6 h; (c) H₂, Pd/C, EA, MeOH, EtOH, DCM, r.t., 6 h; (d) LiOH, THF, MeOH, H₂O, r.t., 1 h; (e) (1*R*,5*S*,6*r*)-6-(1*H-*1,2,3-triazol-5-yl)-3-azabicyclo[3.1.0]hexane hydrochloride, HATU, DIEA, DMF, r.t., 2 h

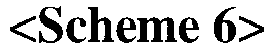

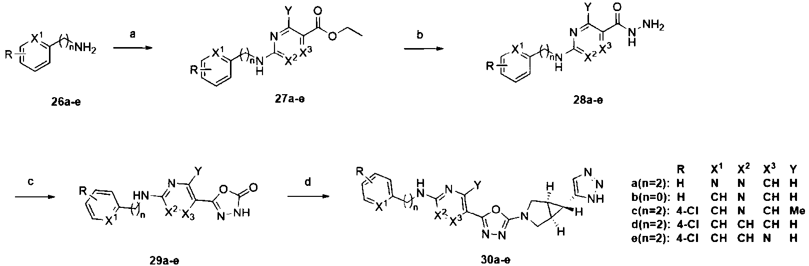

Reagents and conditions: (a) ethyl 2-chloropyrimidine-5-carboxylate, DIEA, 1,4-dioxane, 100 °C, 2 h; (b) N₂H₄·H₂O, EtOH, reflux, 15 h; (c) 1,1'-carbonyldiimidazole, TEA, THF, 0°C (15 min)->r.t., 20 h; (d) (1R,5S,6r)-6-(1H-1,2,3-triazol-5-yl)-3-azabicyclo[3.1.0]hexane hydrochloride, BOP reagent, DIEA, DMF, 0°C->r.t., 22 h

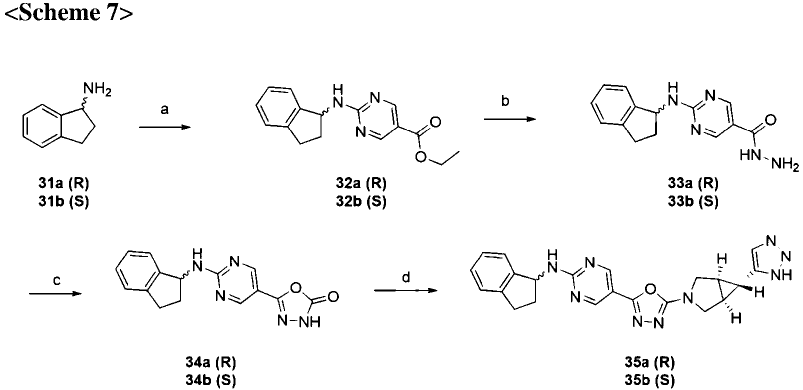

Reagents and conditions: (a) ethyl 2-chloropyrimidine-5-carboxylate, DIEA, 1,4-dioxane, 100 °C, 2 h; (b) N₂H₄·H₂O, EtOH, reflux, 115 h; (c) 1,1'-carbonyldiimidazole, TEA, THF, 0°C(15min)->r.t., 20 h; (d) (1R,5S,6r)-6-(1H-1,2,3-triazol-5-yl)-3-azabicyclo[3.1.0]hexane hydrochloride, BOP reagent, DIEA, DMF, 0°C->r.t., 22 h

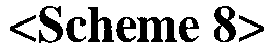

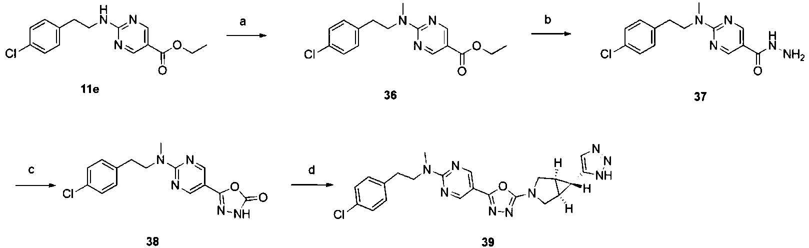

Reagents and conditions: (a) iodomethane, NaH, DMF, 0°C->r.t., 6.5 h; (b) N₂H₄· H₂O, EtOH, reflux, 15 h; (c)1,1'-carbonyldiimidazole, TEA, THF, 0°C(15min)->r.t., 20 h; (d) (1R,5S,6r)-6-(1H-1,2,3-triazol-5-yl)-3-azabicyclo[3.1.0]hexane hydrochloride, BOP reagent, DIEA, DMF, 0°C->r.t., 22h

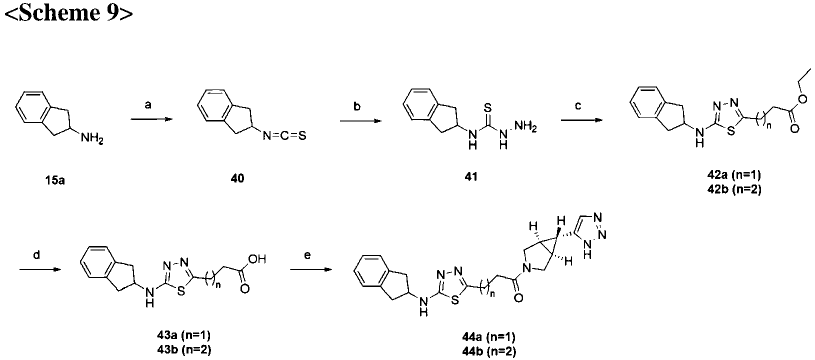

Reagents and conditions: (a) O,O'-Di-2-pyridyl thiocarbonate, DIEA, DCM, r.t., 12h; (b) N₂H₄·H₂O, EtOH, r.t., 12 h; (c) ethyl 3-cyanopropanoate or ethyl 4-cyanobutanoate, TFA, 80°C, 12h; (d) LiOH, THF, MeOH, H₂O, r.t., 3 h; (e) (1*R*,5*S*,6*r*)-6-(1*H*-1,2,3-triazol-5-yl)-3-azabicyclo[3.1.0]hexane hydrochloride, HATU, DIEA, DCM, r.t., 12h

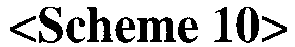

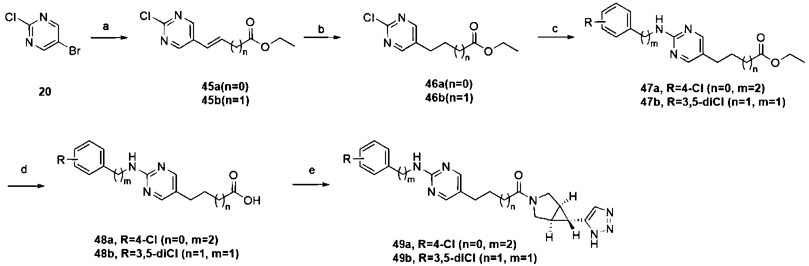

Reagents and conditions: (a) ethyl acrylate or ethyl but-3-enoate, Pd(OAc)₂, tri(o-tolyl)phosphine, DIEA, DMF, reflux, 4 h; (b) H₂, Pd/C, EtOAc, r.t., 12 h; (c) 2-(4-chlorophenyl)ethan-1-amine or (3,5-dichlorophenyl)methanamine, DIEA, n-butanol, microwave, 150 °C, 2 h; (d) LiOH, THF, MeOH, H₂O, r.t., 3 h; (e) (1*R*,5*S*,6*r*)-6-(1*H*-1,2,3-triazol-5-yl)-3-azabicyclo[3.1.0]hexane hydrochloride, HATU, DIEA, DCM, r.t., 12 h

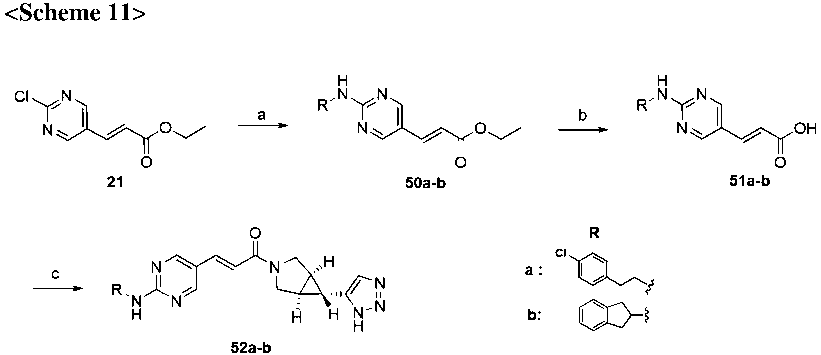

Reagents and conditions: (a) 2-(4-chlorophenyl)ethan-1-amine or (3,5-dichlorophenyl)methanamine, DIEA, n-butanol, microwave, 150 °C, 2 h; (d) LiOH, THF, MeOH, H₂O, r.t., 3 h; (e) (1*R*,5*S*,6*r*)-6-(1*H*-1,2,3-triazol-5-yl)-3-azabicyclo[3.1.0]hexane hydrochloride, HATU, DIEA, DCM, r.t., 12 h

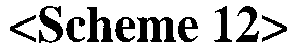

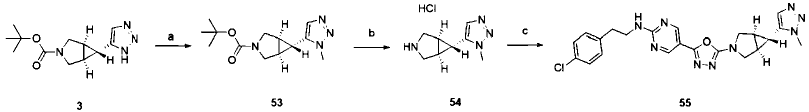

Reagents and conditions: (a) iodomethane, NaH, DMF, 0°C->r.t., 15h; (b) dioxane containing 4N HCl, 1,4-dioxane, r.t., 3 h; (c) 2-(4-chlorophenyl)ethan-1-amine, DIEA, DMF, 0°C(30min)-> BOP reagent, r.t., 15h

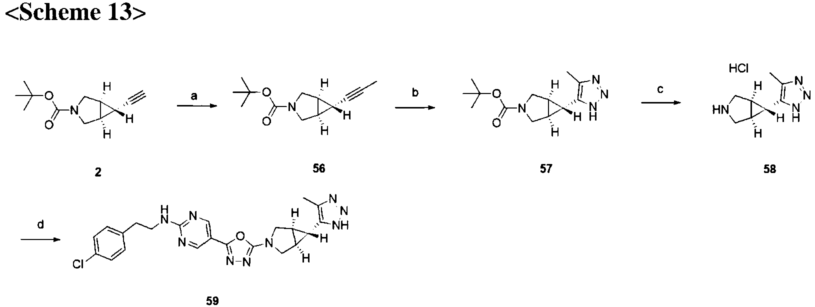

Reagents and conditions: (a) i) n-BuLi(2.5M in hexane), THF, -78°C, 30min ii) iodomethane, r.t., 12h; (b) Me₃SiN₃, microwave, 200 °C, 7 h; (c) dioxane containing 4N HCl, 1,4-dioxane, r.t., 3 h; (d) 2-(4-chlorophenyl)ethan-1-amine, BOP reagent, DIEA, DMF, r.t., 5h

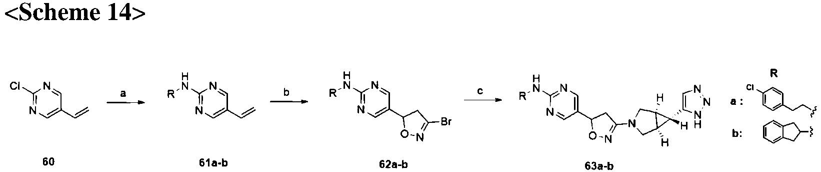

Reagents and conditions: (a) 2-(4-chlorophenyl)ethan-1-amine or 2,3-dihydro-1H-inden-2-amine, DIEA, n-butanol, microwave, 150 °C, 2 h; (b) i)1,1-dibromoformaldoxime, -10°C, DMF ii) KHCO₃, H₂O, r.t., 1 h; (c) (1*R*,5*S*,6*r*)-6-(1*H*-1,2,3-triazol-5-yl)-3-azabicyclo[3.1.0]hexane hydrochloride, DIEA, tert-butanol, microwave, 200°C, 3 h

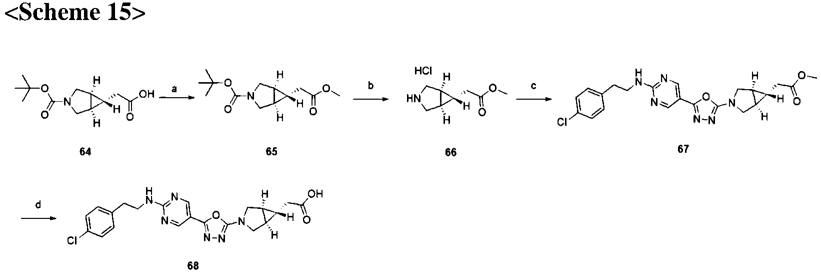

Reagents and conditions: (a) iodomethane, K₂CO₃, DMF, r.t., 15h; (b) dioxane containing 4N HCl, 1,4-dioxane, r.t., 5 h; (c) 2-(4-chlorophenyl)ethan-1-amine, DIEA, DMF, 0°C(30min)-> BOP reagent, r.t., 15 h; (d) 1N NaOH, THF, r.t., 1.5h

### Synthetic example 1. tert-Butyl (1R,5S,6s)-6-ethynyl-3-azabicyclo[3.1.0]hexane-3-carboxylate (2)

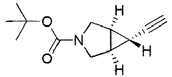

*tert*-Butyl (1*R*,5*S*,6*r*)-6-formyl-3-azabicyclo[3.1.0]hexane-3-carboxylate (1 mmol) and K₂CO₃ (2 mmol) in MeOH was stirred. Then, a mixture of dimethyl (1-diazo-2-oxopropyl)phosphonate (1.2 mmol) dissolved in ACN was slowly added dropwise. The solvent was evaporated under reduced pressure and extracted with CH₂Cl₂. The combined organic solution was dried over Na₂SO₄ and concentrated. The solvent was removed under reduced pressure, and the residue was purified by column chromatography (SiO₂, hexane: ethyl acetate) to give compound (2) (400 mg, Yield: 64.3%); ¹H NMR (400 MHz, CD₃OD) *δ* 3.59 - 3.52 (m, 2H), 3.39 - 3.32 (m, 2H), 2.18 (d, *J =* 2.1 Hz, 1H), 1.87 - 1.80 (m, 2H), 1.43 (s, 9H), 1.06 - 1.02 (m, 1H).

### Synthetic example 2. tert-Butyl (1R,5S,6r)-6-(1H-1,2,3-triazol-5-yl)-3-azabicyclo[3.1.0]hexane-3-carboxylate (3)

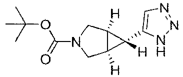

*tert-Butyl* (1R,5S,6s)-6-ethynyl-3-azabicyclo[3.1.0]hexane-3-carboxylate (1) (1.03 mmol), trimethylsilyl azide (1.15 mmol) and copper(I) iodide (0.05 mmol) were dissolved in DMF. The mixture was stirred at 100 °C for 12 h under argon atmosphere. Then the mixture was filtered through a pad of Celite, rinsed with ethyl acetate and washed with water. The combined organic solution was dried over Na₂SO₄ and concentrated. The solvent was removed under reduced pressure, and the residue was purified by column chromatography (SiO₂, hexane: ethyl acetate) to give compound **(3)** (181 mg, Yield: 70 %); ¹H NMR (400 MHz, CDCl₃) *δ* 13.41 (s, 1H), 7.45 (s, 1H), 3.82 - 3.68 (m, 2H), 3.52 - 3.44 (m, 2H), 1.98 - 1.93 (m, 2H), 1.86 - 1.83 (m, 1H), 1.47 (s, 9H).

### Synthetic example 3. (1R,5S,6r)-6-(1H-1,2,3-Triazol-5-yl)-3-azabicyclo[3.1.0]hexane·HCl (4)

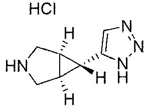

4.0 M HCl in dioxane was added to *tert*-butyl 6-(1*H*-1,2,3-triazol-5-yl)-3-azabicyclo[3.1.0]hexane-3-carboxylate (3, 0.68 mmol) in dioxane. The reaction mixture was stirred at room temperature for 2 h. The solvent was evaporated to dryness to give compound **(4)** (quantitative); ¹H NMR (400 MHz, CD₃OD) *δ* 8.36 (s, 1H), 3.73 - 3.59 (m, 4H), 2.57 - 2.43 (m, 3H).; MS calculated for C₇H₁₁N₄ [M+H]⁺ 151.15, found 151.15.

### General procedure for the synthesis of 6a-j, 11a-e and 16a-c (I)

A mixture of ethyl 2-chloropyrimidine-5-carboxylate (1.4 mmol), various substituted amines (1.68 mmol) and DIEA (3 mmol) in DMF (5 mL) was stirred at 100 °C for 3 h. After cooling, the solvent was evaporated and the crude product was extracted with ethyl acetate and washed with brine. The organic layer was dried over anhydrous sodium sulfate and concentrated. The products were obtained by column chromatography (hexane/ethyl acetate) to obtain **6a-j, 11a-e, and 16a-c.**

### Synthetic example 4. Ethyl 2-(benzylamino)pyrimidine-5-carboxylate (6a)

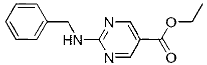

Yield: 78%; ¹H NMR (400 MHz, DMSO-d₆) *δ* 8.73 (d, *J* = 1.6 Hz, 2H), 8.63 (t, *J* = 6.4 Hz, 1H), 7.34 - 7.19 (m, 5H), 4.58 (d, *J* = 6.4 Hz, 2H), 4.25 (q, *J* = 7.1 Hz, 2H), 1.28 (t, *J =* 7.1 Hz, 3H); MS calculated for C₁₄H₁₅N₃O₂ [M+H]⁺ 258.12, found 258.10.

### Synthetic example 5. Ethyl 2-((3-chlorobenzyl)amino)pyrimidine-5-carboxylate (6b)

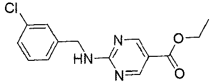

Yield: 95%; ¹H NMR (400 MHz, CDCl₃) δ 9.00 - 8.72 (m, 2H), 7.35 - 7.32 (m, 1H), 7.30 - 7.26 (m, 1H), 7.26 - 7.20 (m, 2H), 6.00 (s, 1H), 4.70 (dd, *J* = 6.2, 0.8 Hz, 2H), 4.35 (q, *J =* 7.1 Hz, 2H), 1.37 (t, *J* = 7.2 Hz, 3H); MS calculated for C₁₄H₁₅ClN₃O₂ [M+H]⁺ 292.08, found 292.00.

### Synthetic example 6. Ethyl 2-((4-chlorobenzyl)amino)pyrimidine-5-carboxylate (6c)

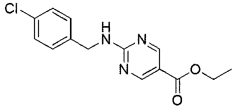

Yield: 74%; ¹H NMR (400 MHz, CDCl₃) *δ* 8.95 - 8.68 (m, 2H), 7.33 - 7.26 (m, 4H), 6.11 (s, 1H), 4.67 (d, *J =* 6.1 Hz, 2H), 4.35 (q, *J =* 7.1 Hz, 2H), 1.37 (t, *J =* 7.1 Hz, 3H); MS calculated for C₁₄H₁₆ClN₃O₂ [M+H]⁺ 292.08, found 292.05.

### Synthetic example 7. Ethyl 2-((3,4-difluorobenzyl)amino)pyrimidine-5-carboxylate (6d)

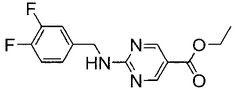

Yield: 74%; ¹H NMR (400 MHz, CDCl₃) *δ* 8.86 (s, 2H), 7.22 - 7.03 (m, 3H), 5.88 (s, 1H), 4.67 (dq, J = 6.3, 0.8 Hz, 2H), 4.35 (q, J = 7.1 Hz, 2H), 1.37 (t, J = 7.1 Hz, 3H); MS calculated for C₁₄H₁₄F₂N₃O₂ [M+H]⁺ 294.10, found 294.15.

### Synthetic example 8. Ethyl 2-((3,5-difluorobenzyl)amino)pyrimidine-5-carboxylate (6e)

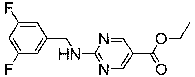

Yield: 51%; ¹H NMR (400 MHz, CDCl₃) *δ* 8.86 (s, 2H), 6.89 - 6.82 (m, 2H), 6.71 (tt, J = 8.9, 2.3 Hz, 1H), 6.02 (s, 1H), 4.71 (dd, J = 6.4, 0.7 Hz, 2H), 4.35 (q, J = 7.1 Hz, 2H), 1.37 (t, J = 7.2 Hz, 3H); MS calculated for C₁₄H₁₄F₂N₃O₂ [M+H]⁺ 294.10, found 294.15.

### Synthetic example 9. Ethyl 2-((3,4-dichlorobenzyl)amino)pyrimidine-5-carboxylate (6f)

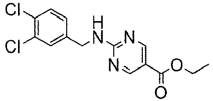

Yield: 94%; ¹H NMR (400 MHz, CDCl₃) *δ* 8.86 (s, 2H), 7.46 - 7.37 (m, 2H), 7.17 (ddt, J = 8.2, 2.1, 0.7 Hz, 1H), 5.92 (s, 1H), 4.67 (dt, J = 6.3, 0.8 Hz, 2H), 4.35 (q, J = 7.1 Hz, 2H), 1.37 (t, J = 7.1 Hz, 3H); MS calculated for C₁₄H₁₄Cl₂N₃O₂ [M+H]⁺ 326.05, found 326.05.

### Synthetic example 10. Ethyl 2-((3,5-dichlorobenzyl)amino)pyrimidine-5-carboxylate (6g)

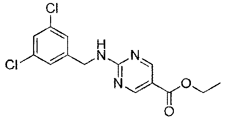

Yield: 96%; ¹H NMR (400 MHz, CDCl₃) *δ* 8.90 (s, 2H), 7.32 - 7.30 (m, 2H), 7.27 - 7.26 (m, 1H), 6.09 (d, *J* = 6.4 Hz, 1H), 4.72 (d, *J* = 6.3 Hz, 2H), 4.40 (q, *J* = 7.1 Hz, 2H), 1.42 (t, *J* = 7.1 Hz, 3H); MS calculated for C₁₄H₁₄Cl₂N₃O₂ [M+H]⁺ 326.05, found 326.05.

### Synthetic example 11. Ethyl 2-((3,5-dibromobenzyl)amino)pyrimidine-5-carboxylate (6h)

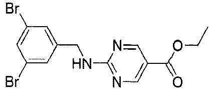

Yield: 82%; ¹H NMR (400 MHz, CDCl₃) *δ* 8.92 - 8.71 (m, 2H), 7.57 (t, J = 1.8 Hz, 1H), 7.44 - 7.40 (m, 2H), 6.33 - 6.22 (m, 1H), 4.69 - 4.65 (m, 2H), 4.36 (q, J = 7.1 Hz, 2H), 1.38 (t, J = 7.1 Hz, 3H).; MS calculated for C₁₄H₁₄C₁₂N₅O₂ [M+H]⁺ 413.94, found 415.95.

### Synthetic example 12. Ethyl 2-((benzo[d][1,3]dioxol-5-ylmethyl)amino)pyrimidine-5-carboxylate (6i)

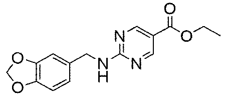

Yield: 87%; ¹H NMR (400 MHz, CDCl₃) *δ* 8.99 - 8.64 (m, 2H), 6.86 - 6.74 (m, 3H), 5.98 (s, 1H), 5.95 (s, 2H), 4.60 (dd, *J =* 6.0, 0.6 Hz, 2H), 4.35 (q, *J =* 7.1 Hz, 2H), 1.37 (t, *J* = 7.1 Hz, 3H);MS calculated for C₁₄H₁₆N₃O₄ [M+H]⁺ 302.11, found 302.10.

### Synthetic example 13. Ethyl 2-((3,5-bis(trifluoromethyl)benzyl)amino)pyrimidine-5-carboxylate (6j)

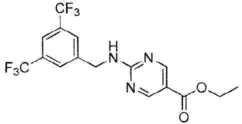

Yield: 93%; ¹H NMR (400 MHz, CDCl₃) δ 8.88 (s, 2H), 7.80 (s, 3H), 6.02 (t, *J* = 4.7 Hz, 1H), 4.84 (d, *J* = 6.4 Hz, 2H), 4.36 (q, *J* = 7.1 Hz, 2H), 1.38 (t, *J* = 7.1 Hz, 3H); MS calculated for C₁₆H₁₃ F₆N₃O₂ [M+H]⁺ 394.10, found 394.05.

### Synthetic example 14. Ethyl 2-(phenethylamino)pyrimidine-5-carboxylate (11a)

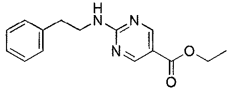

Yield: 80 %; ¹H NMR (400 MHz, CDCl₃) δ 8.95 - 8.68 (m, 2H), 7.34 - 7.28 (m, 2H), 7.25 - 7.18 (m, 3H), 5.75 (d, *J* = 6.5 Hz, 1H), 4.35 (q, *J* = 7.1 Hz, 2H), 3.76 (td, *J* = 7.0, 6.0 Hz, 2H), 2.93 (t, *J* = 7.0 Hz, 2H), 1.37 (t, *J* = 7.1 Hz, 3H).; MS calculated for C₁₅H₁₈ N₃O₂ [M+H]⁺ 272.14, found 272.05.

### Synthetic example 15. Ethyl 2-((3-fluorophenethyl)amino)pyrimidine-5-carboxylate (11b)

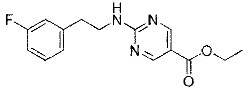

Yield: 82%; ¹H NMR (400 MHz, DMSO-d₆) *δ* 8.79 - 8.65 (m, 2H), 8.18 (t, *J* = 5.8 Hz, 1H), 7.31 (td, *J =* 8.1, 6.3 Hz, 1H), 7.12 - 6.98 (m, 3H), 4.25 (q, *J* = 7.1 Hz, 2H), 3.58 (dt, *J* = 7.5, 6.4 Hz, 2H), 2.88 (t, *J =* 7.2 Hz, 2H), 1.28 (t, *J* = 7.1 Hz, 3H); MS calculated for C₁₅H₁₆FN₃O₂ [M+H]⁺ 290.13, found 290.00.

### Synthetic example 16. Ethyl 2-((4-fluorophenethyl)amino)pyrimidine-5-carboxylate (11c)

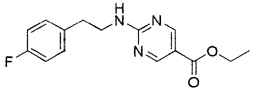

Yield: 75%; ¹H NMR (400 MHz, DMSO- d₆) δ 8.77 - 8.64 (m, 2H), 8.16 (t, *J =* 5.8 Hz, 1H), 7.29 - 7.23 (m, 2H), 7.12 - 7.06 (m, 2H), 4.25 (q, *J* = 7.1 Hz, 2H), 3.55 (dt, *J* = 7.8, 6.3 Hz, 2H), 2.84 (t, *J* = 7.3 Hz, 2H), 1.28 (t, *J* = 7.1 Hz, 3H); MS calculated for C₁₅H₁₆FN₃O₂ [M+H]⁺ 290.13, found 290.00.

### Synthetic example 17. Ethyl 2-((3-chlorophenethyl)amino)pyrimidine-5-carboxylate (11d)

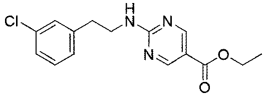

Yield: 83%; ¹H NMR (400 MHz, CDCl₃) δ 8.96 - 8.70 (m, 2H), 7.25 - 7.18 (m, 3H), 7.10 (dt, *J =* 7.5, 1.7 Hz, 1H), 5.73 (s, 1H), 4.35 (q, *J* = 7.1 Hz, 2H), 3.75 (td, *J =* 7.0, 6.1 Hz, 2H), 2.91 (t, *J* = 7.0 Hz, 2H), 1.37 (t, *J* = 7.1 Hz, 3H); MS calculated for C₁₅H₁₆ClN₃O₂ [M+H]⁺ 306.10, found 306.00.

### Synthetic example 18. Ethyl 2-((4-chlorophenethyl)amino)pyrimidine-5-carboxylate (11e)

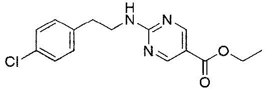

Yield: 82%; ¹H NMR (400 MHz, CDCl₃) δ 8.96 - 8.68 (m, 2H), 7.27 (d, *J* = 6.3 Hz, 1H), 7.25 (s, 1H), 7.17 - 7.13 (m, 2H), 5.66 (s, 1H), 4.35 (q, *J* = 7.1 Hz, 2H), 3.74 (td, *J* = 7.0, 6.1 Hz, 2H), 2.90 (t, *J* = 7.0 Hz, 2H), 1.37 (t, *J* = 7.1 Hz, 3H).; MS calculated for C₁₅H₁₆ClN₃O₂ [M+H]⁺ 306.10, found 305.95.

### Synthetic example 19. Ethyl 2-((2,3-dihydro-1H-inden-2-yl)amino)pyrimidine-5-carboxylate (16a)

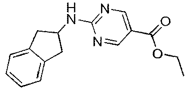

Yield: 64 %; ¹H NMR (400 MHz, CD₃OD) *δ* 8.92 - 8.64 (m, 2H), 7.24 - 7.09 (m, 4H), 4.83 - 4.77 (m, 1H), 4.33 (q, *J =* 7.1 Hz, 2H), 3.37 - 3.31 (m, 2H), 2.93 (dd, *J =* 15.7, 6.4 Hz, 2H), 1.36 (t, *J* = 7.1 Hz, 3H); MS calculated for C₁₆H₁₈N₃O₂ [M+H]+ 284.14, found 284.10.

### Synthetic example 20. Ethyl 2-((5,6-difluoro-2,3-dihydro-1H-inden-2-yl)amino)pyrimidine-5-carboxylate (16b)

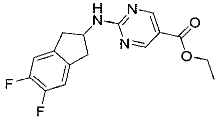

Yield: 86%; ¹H NMR (400 MHz, CDCl₃) *δ* 8.85 (d, *J* = 35.7 Hz, 2H), 7.02 (t, *J =* 8.8 Hz, 2H), 5.82 (s, 1H), 4.90 (qt, *J* = 7.2, 5.2 Hz, 1H), 4.36 (q, *J* = 7.1 Hz, 2H), 3.36 (dd, *J =* 16.0, 7.0 Hz, 2H), 2.85 (dd, *J =* 16.0, 5.2 Hz, 2H), 1.38 (t, *J* = 7.1 Hz, 3H); MS calculated for C₁₆H₁₆F₂N₃O₂ [M+H]⁺ 320.12, found 320.10.

### Synthetic example 21. Ethyl 2-((5-bromo-2,3-dihydro-1H-inden-2-yl)amino)pyrimidine-5-carboxylate (16c)

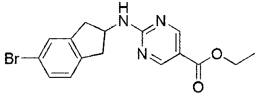

Yield: 63%; ¹H NMR (400 MHz, CDCl₃) *δ* 9.00 - 8.75 (m, 2H), 7.40 (d, *J* = 1.8 Hz, 1H), 7.37 - 7.31 (m, 1H), 7.13 (d, *J* = 8.0 Hz, 1H), 5.76 (d, *J* = 8.0 Hz, 1H), 4.91 (qt, *J* = 7.2, 5.1 Hz, 1H), 4.38 (q, *J* = 7.1 Hz, 2H), 3.45 - 3.32 (m, 2H), 2.95 - 2.83 (m, 2H), 1.40 (t, *J =* 7.1 Hz, 3H).; MS calculated for C₁₆H₁₇BrN₃O₂ [M+H]⁺ 362.05, found 364.00.

### General procedure for the synthesis of 7a-j, 12a-e, and 17a-c (II)

**Compounds 6a-j, 11a-e,** and **16a-c** (5 mmol) and hydrazine monohydrate (50 mmol) were dissolved in EtOH and refluxed for 3 h. After the reaction was completed, the reaction mixture was evaporated under vacuum to obtain **7a-j, 12a-e,** and **17a-c.**

### Synthetic example 22. 2-(Benzylamino)pyrimidine-5-carbohydrazide (7a)

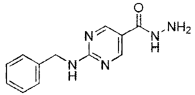

Yield: Quantitative.; ¹H NMR (400 MHz, DMSO-d₆) *δ* 9.55 (s, 1H), 8.66 (s, 2H), 8.27 (t, *J =* 6.4 Hz, 1H), 7.30 - 7.20 (m, 5H), 4.54 (d, *J =* 6.3 Hz, 2H), 4.40 (s, 2H); MS calculated for C₁₂H₁₄N₅O₂ [M+H]⁺ 244.12, found 244.15.

### Synthetic example 23. 2-((3-Chlorobenzyl)amino)pyrimidine-5-carbohydrazide (7b)

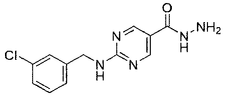

Yield: Quantitative.; ¹H NMR (400 MHz, DMSO-d₆) *δ* 9.58 (s, 1H), 8.68 (s, 2H), 8.32 (t, *J* = 6.4 Hz, 1H), 7.37 - 7.33 (m, 2H), 7.31 - 7.26 (m, 2H), 4.56 (d, *J =* 6.3 Hz, 2H), 4.49 (d, *J* = 6.0 Hz, 1H); MS calculated for C₁₂H₁₃ClN₅O₂ [M+H]⁺ 278.08, found 278.10.

### Synthetic example 24. 2-((4-Chlorobenzyl)amino)pyrimidine-5-carbohydrazide (7c)

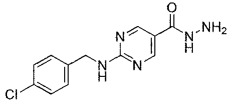

Yield: Quantitative.; ¹H NMR (400 MHz, DMSO-d₆) *δ* 9.56 (s, 1H), 8.66 (s, 2H), 8.30 (t, *J* = 6.4 Hz, 1H), 7.38 - 7.30 (m, 4H), 4.52 (d, *J =* 6.3 Hz, 2H), 4.47 - 4.32 (m, 2H); MS calculated for C₁₂H₁₃ClN₃O₂ [M+H]⁺ 278.08, found 278.10.

### Synthetic example 25. 2-((3,4-Difluorobenzyl)amino)pyrimidine-5-carbohydrazide (7d)

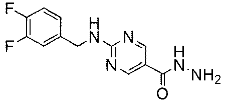

Yield: Quantitative.; ¹H NMR (400 MHz, CD₃OD) *δ* 8.67 (s, 2H), 7.26 - 7.16 (m, 2H), 7.15 - 7.11 (m, 1H), 4.60 (s, 2H); MS calculated for C₁₂H₁₂F₂N₅O₂ [M+H]⁺ 280.10, found 280.15.

### Synthetic example 26. 2-((3,5-Difluorobenzyl)amino)pyrimidine-5-carbohydrazide (7e)

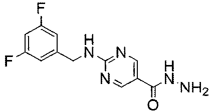

Yield: Quantitative.; ¹H NMR (400 MHz, MeOD) *δ* 8.67 (s, 2H), 6.95 - 6.89 (m, 2H), 6.78 (tt, *J* = 9.1, 2.4 Hz, 1H), 4.63 (s, 2H); MS calculated for C₁₂H₁₂F₂N₅O₂ [M+H]⁺ 280.10, found 280.10.

### Synthetic example 27. 2-((3,4-Dichlorobenzyl)amino)pyrimidine-5-carbohydrazide (7f)

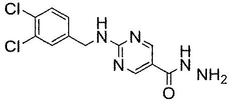

Yield: Quantitative.; ¹H NMR (400 MHz, CD₃OD) *δ* 8.67 (s, 2H), 7.49 - 7.43 (m, 2H), 7.28 - 7.24 (m, 1H), 4.60 (s, 2H); MS calculated for C₁₂H₁₂Cl₂N₃O₂ [M+H]⁺ 312.04, found 312.10.

### Synthetic example 28. 2-((3,5-Dichlorobenzyl)amino)pyrimidine-5-carbohydrazide (7g)

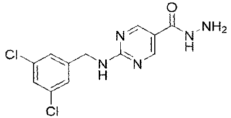

Yield: Quantitative.; ¹H NMR (400 MHz, DMSO-d₆) *δ* 9.59 (s, 1H), 8.68 (s, 2H), 8.32 (t, *J =* 6.4 Hz, 1H), 7.46 (t, *J =* 1.9 Hz, 1H), 7.34 (d, *J =* 1.9 Hz, 2H), 4.54 (d, *J =* 6.3 Hz, 2H), 3.99 (s, 2H); MS calculated for C₁₂H₁₂Cl₂N₅O₂ [M+H]⁺ 312.04, found 312.05.

### Synthetic example 29. 2-((3,5-Dibromobenzyl)amino)pyrimidine-5-carbohydrazide (7h)

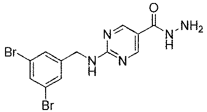

Yield: Quantitative.; ¹H NMR (400 MHz, DMSO-d₆) *δ* 10.29 (s, 1H), 8.71 (s, 2H), 8.37 (s, 1H), 7.70 (t, *J* = 1.8 Hz, 1H), 7.54 - 7.51 (m, 2H), 4.58 - 4.53 (m, 2H); MS calculated for C₁₂H₁₂Br₂N₅O₂ [M+H]⁺ 399.94, found 401.95.

### Synthetic example 30. 2-((Benzo[d][1,3]dioxol-5-ylmethyl)amino)pyrimidine-5-carbohydrazide (7i)

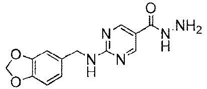

Yield: Quantitative.; ¹H NMR (400 MHz, DMSO-d₆) *δ* 9.56 (s, 1H), 8.66 (s, 2H), 8.20 (t, *J* = 6.3 Hz, 1H), 6.87 - 6.81 (m, 2H), 6.79 - 6.76 (m, 1H), 5.96 (s, 2H), 4.44 (d, *J =* 6.4 Hz, 2H), 4.39 (s, 2H) ;MS calculated for C₁₃H₁₄N₃O₃ [M+H]⁺ 288.11, found 288.15.

### Synthetic example 31. 2-((3,5-Bis(trifluoromethyl)benzyl)amino)pyrimidine-5-carbohydrazide (7j)

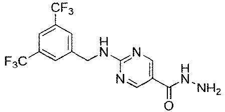

Yield: Quantitative.; ¹H NMR (400 MHz, DMSO-d₆) *δ* 9.59 (s, 1H), 8.68 (s, 2H), 8.40 (t, *J =* 6.4 Hz, 1H), 7.99 (dd, *J* = 10.3, 2.0 Hz, 3H), 4.72 (d, *J =* 6.3 Hz, 2H), 4.41 (s, 2H); MS calculated for C₁₄H₁₂F₆N₆O [M+H]⁺ 380.09, found 380.10.

### Synthetic example 32. 2-(Phenethylamino)pyrimidine-5-carbohydrazide (12a)

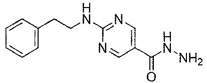

Yield: Quantitative.; ¹H NMR (400 MHz, DMSO-d₆) *δ* 9.56 (s, 1H), 8.72 - 8.56 (m, 2H), 7.79 (t, *J* = 5.8 Hz, 1H), 7.32 - 7.15 (m, 5H), 4.39 (s, 2H), 3.53 (ddd, *J* = 8.6, 7.5, 6.0 Hz, 2H), 2.84 (dd, *J* = 8.3, 6.6 Hz, 2H); MS calculated for C₁₃H₁₆N₅O [M+H]⁺ 258.30, found 258.10.

### Synthetic example 33. 2-((3-Fluorophenethyl)amino)pyrimidine-5-carbohydrazide (12b)

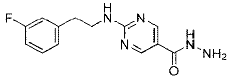

Yield: Quantitative.; ¹H NMR (400 MHz, DMSO-d₆) *δ* 9.56 (s, 1H), 8.66 (d, *J* = 16.1 Hz, 2H), 7.80 (t, *J* = 5.8 Hz, 1H), 7.32 (td, *J* = 8.1, 6.4 Hz, 1H), 7.11 - 6.97 (m, 3H), 4.40 (s, 2H), 3.55 (dt, *J* = 7.6, 6.3 Hz, 2H), 2.87 (t, *J* = 7.2 Hz, 2H); MS calculated for C₁₃H₁₅FN₅O [M+H]⁺ 276.29, found 276.05.

### Synthetic example 34. 2-((4-Fluorophenethyl)amino)pyrimidine-5-carbohydrazide (12c)

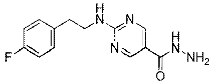

Yield: Quantitative.; ¹H NMR (400 MHz, DMSO-d₆) *δ* 9.55 (s, 1H), 8.66 (d, *J =* 15.9 Hz, 2H), 7.79 (t, *J =* 5.8 Hz, 1H), 7.30 - 7.23 (m, 2H), 7.14 - 7.06 (m, 2H), 4.39 (s, 2H), 3.52 (dt, *J=* 7.9, 6.3 Hz, 2H), 2.83 (t, *J* = 7.3 Hz, 2H); MS calculated for C₁₃H₁₅FN₅O [M+H]⁺ 276.29, found 276.05.

### Synthetic example 35. 2-((3-Chlorophenethyl)amino)pyrimidine-5-carbohydrazide (12d)

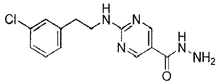

Yield: Quantitative.; ¹H NMR (400 MHz, DMSO-d₆) *δ* 9.57 (s, 1H), 8.66 (d, *J* = 16.0 Hz, 2H), 7.80 (t, *J* = 5.8 Hz, 1H), 7.33 - 7.23 (m, 3H), 7.19 (dt, *J* = 7.5, 1.5 Hz, 1H), 4.40 (s, 2H), 3.54 (dt, *J* = 7.6, 6.3 Hz, 2H), 2.85 (t, *J* = 7.2 Hz, 2H); MS calculated for C₁₃H₁₅Cl₅O [M+H]⁺ 292.75, found 292.00.

### Synthetic example 36. 2-((4-Chlorophenethyl)amino)pyrimidine-5-carbohydrazide (12e)

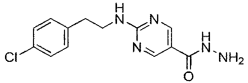

Yield: Quantitative.; ¹H NMR (400 MHz, DMSO-d₆) *δ* 9.56 (s, 1H), 8.66 (d, *J =* 16.6 Hz, 2H), 7.79 (t, *J* = 5.8 Hz, 1H), 7.35 - 7.30 (m, 2H), 7.28 - 7.22 (m, 2H), 4.40 (s, 2H), 3.52 (dt, *J* = 7.7, 6.2 Hz, 2H), 2.83 (t, *J* = 7.2 Hz, 2H); MS calculated for C₁₃H₁₅ClN₅O [M+H]⁺ 292.75, found 292.00.

### Synthetic example 37. 2-((2,3-Dihydro-1H-inden-2-yl)amino)pyrimidine-5-carbohydrazide (17a)

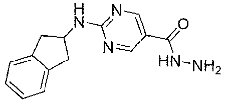

Yield: Quantitative.; ¹H NMR (400 MHz, DMSO-d₆) *δ* 9.58 (s, 1H), 8.69 (s, 2H), 8.06 (d, *J* = 6.9 Hz, 1H), 7.26 - 7.09 (m, 4H), 4.72 - 4.61 (m, 1H), 4.41 (s, 2H), 3.28 - 3.22 (m, 2H), 2.95 - 2.86 (m, 2H); MS calculated for C₁₄H₁₆N₅O [M+H]⁺ 270.13, found 270.15.

### Synthetic example 38. 2-((2,3-Dihydro-1H-inden-2-yl)amino)pyrimidine-5-carbohydrazide (17b)

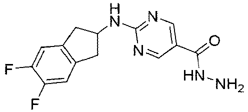

Yield: Quantitative.; ¹H NMR (400 MHz, DMSO-d₆) *δ* 9.60 (s, 1H), 8.69 (d, *J* = 14.2 Hz, 2H), 8.12 (d, *J* = 6.9 Hz, 1H), 7.28 (t, *J* = 9.3 Hz, 2H), 4.73 - 4.61 (m, 1H), 4.42 (s, 2H), 3.23 (dd, *J* = 16.0, 7.5 Hz, 2H), 2.86 (dd, *J* = 16.0, 6.6 Hz, 2H).;MS calculated for C₁₄H₁₄F₂N₃O [M+H]⁺ 306.12, found 306.10

### Synthetic example 39. 2-((5-Bromo-2,3-dihydro-1H-inden-2-yl)amino)pyrimidine-5-carbohydrazide (17c)

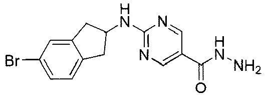

Yield: Quantitative.; ¹H NMR (400 MHz, DMSO-d₆) *δ* 9.58 (s, 1H), 8.69 (s, 2H), 8.08 (dd, *J =* 6.9, 2.9 Hz, 1H), 7.42 (d, *J =* 1.9 Hz, 1H), 7.32 (dd, *J* = 8.0, 2.0 Hz, 1H), 7.18 (d, *J =* 8.0 Hz, 1H), 4.66 (h, *J =* 7.0 Hz, 1H), 4.41 (s, 2H), 3.29 - 3.18 (m, 2H), 2.95 - 2.82 (m, 2H).; MS calculated for C₁₄H₁₂F₆N₆O [M+H]⁺ 380.09, found 380.10.

### General procedure for the synthesis of 8a-j, 13a-e, and 18a-c (III)

**Compounds 7a-j, 12a-e,** and **17a-c** (0.1 mmol) dissolved in DMF (5 mL) and TEA (0.5 mmol) was slowly added to a solution of triphosgene (0.04 mmol) in THF (5 mL) at 0 °C. The reaction mixture was stirred at room temperature for 2 h. After the reaction was completed, the solvent was evaporated to dryness. The product was extracted with ethyl acetate and washed with brine. The organic layer was dried over anhydrous sodium sulfate and concentrated. The residue was purified by column chromatography (EA/Hex) to give **8a-j, 13a-e,** and **18a-c.**

### Synthetic example 40. 5-(2-(Benzylamino)pyrimidin-5-yl)-1,3,4-oxadiazol-2(3H)-one (8a)

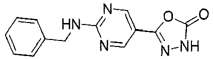

Yield: 10%; ¹H NMR (400 MHz, DMSO-d₆) *δ* 12.43 (s, 1H), 8.63 (s, 2H), 8.50 (t, *J =* 6.3 Hz, 1H), 7.31 (d, *J* = 4.8 Hz, 4H), 7.26 - 7.20 (m, 1H), 4.57 (d, *J* = 6.3 Hz, 2H); MS calculated for C₁₃H₁₂N₅O₂ [M+H]⁺ 270.10, found 270.05.

### Synthetic example 41. 5-(2-((3-Chlorobenzyl)amino)pyrimidin-5-yl)-1,3,4-oxadiazol-2(3H)-one (8b)

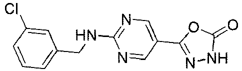

Yield: 36.9%; ¹H NMR (400 MHz, DMSO-d₆) *δ* 12.44 (s, 1H), 8.64 (d, *J =* 4.0 Hz, 2H), 8.52 (t, *J* = 6.4 Hz, 1H), 7.37 - 7.26 (m, 4H), 4.57 (d, *J* = 6.4 Hz, 2H); MS calculated for C₁₃H₁₁ClN₅O₂ [M+H]⁺ 304.06, found 304.05.

### Synthetic example 42. 5-(2-((4-Chlorobenzyl)amino)pyrimidin-5-yl)-1,3,4-oxadiazol-2(3H)-one (8c)

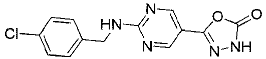

Yield: 10 %; ¹H NMR (400 MHz, DMSO-d₆) *δ* 12.44 (s, 1H), 8.63 (s, 2H), 8.51 (t, *J =* 6.3 Hz, 1H), 7.39 - 7.36 (m, 2H), 7.32 (d, *J* = 8.6 Hz, 2H), 4.55 (d, *J* = 6.3 Hz, 2H); MS calculated for C₁₃H₁₁ClN₅O₂ [M+H]⁺ 304.06, found 304.05.

### Synthetic example 43. 5-(2-((3,4-Difluorobenzyl)amino)pyrimidin-5-yl)-1,3,4-oxadiazol-2(3H)-one (8d)

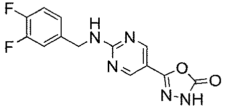

Yield: 41%; ¹H NMR (400 MHz, MeOD) *δ* 8.67 (s, 2H), 7.28 - 7.13 (m, 3H), 4.60 (s, 2H); MS calculated for C₁₃H₁₀F₂N₅O₂ [M+H]⁺ 306.08, found 306.05.

### Synthetic example 44. 5-(2-((3,5-Difluorobenzyl)amino)pyrimidin-5-yl)-1,3,4-oxadiazol-2(3H)-one (8e)

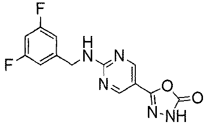

Yield: 47%; ¹H NMR (400 MHz, CD₃OD) *δ* 8.68 (s, 2H), 6.97 - 6.90 (m, 2H), 6.79 (tt, *J* = 9.2, 2.4 Hz, 1H), 4.64 (s, 2H); MS calculated for C₁₃H₁₀F₂N₅O₂ [M+H]⁺ 306.08, found 306.10.

### Synthetic example 45.5-(2-((3,4-Dichlorobenzyl)amino)pyrimidin-5-yl)-1,3,4-oxadiazol-2(3H)-one (8f)

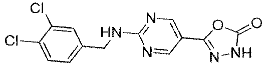

Yield: 20%; ¹H NMR (400 MHz, CD₃OD) *δ* 8.68 (s, 2H), 7.49 (d, *J* = 2.0 Hz, 1H), 7.45 (d, *J* = 8.3 Hz, 1H), 7.30 - 7.25 (m, 1H), 4.61 (s, 2H); MS calculated for C₁₃H₁₀Cl₂N₅O₂ [M+H]⁺ 338.02, found 339.05.

### Synthetic example 46.5-(2-((3,5-Dichlorobenzyl)amino)pyrimidin-5-yl)-1,3,4-oxadiazol-2(3H)-one (8g)

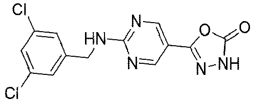

Yield: 15%; ¹H NMR (400 MHz, DMSO-d₆) *δ* 12.48 (s, 1H), 8.66 (s, 2H), 8.53 (t, *J =* 6.4 Hz, 1H), 7.49 (t, *J* = 2.0 Hz, 1H), 7.36 (d, *J =* 1.9 Hz, 2H), 4.58 (d, *J =* 6.3 Hz, 2H); MS calculated for C₁₃H₁₀Cl₂N₅O₂ [M+H]⁺ 338.02, found 338.00.

### Synthetic example 47. 5-(2-((3,5-Dibromobenzyl)amino)pyrimidin-5-yl)-1,3,4-oxadiazol-2(3H)-one (8h)

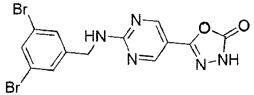

Yield: 65%; ¹H NMR (400 MHz, DMSO-d₆) *δ* 12.46 (s, 1H), 8.69 - 8.63 (m, 2H), 8.52 (t, *J* = 6.4 Hz, 1H), 7.71 (t, *J* = 1.8 Hz, 1H), 7.53 (d, *J* = 1.8 Hz, 2H), 4.57 (d, *J* = 6.2 Hz, 2H); MS calculated for C₁₃H₁₀Br₂N₅O₂ [M+H]⁺ 425.92, found 427.95.

### Synthetic example 48. 5-(2-((Benzo[d][1,3]dioxol-5-ylmethyl)amino)pyrimidin-5-yl)-1,3,4-oxadiazol-2(3H)-one (8i)

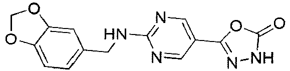

Yield: 33%; ¹H NMR (400 MHz, MeOD) *δ* 8.66 (s, 2H), 6.85 - 6.80 (m, 2H), 6.76 - 6.72 (m, 1H), 5.90 (s, 2H), 4.53 (s, 2H); MS calculated for C₁₄H₁₂N₅O₄ [M+H]⁺ 314.09, found 314.15.

### Synthetic example 49. 5-(2-((3,5-Bis(trifluoromethyl)benzyl)amino)pyrimidin-5-yl)-1,3,4-oxadiazol-2(3H)-one (8j)

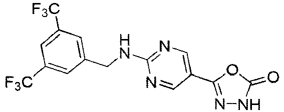

Yield: 10%; ¹H NMR (400 MHz, DMSO-d₆) *δ* 12.46 (s, 1H), 8.71 - 8.51 (m, 3H), 8.02 - 7.95 (m, 3H), 4.78 - 4.70 (m, 2H); MS calculated for C₁₅H₁₀F₆N₆O₂ [M+H]⁺ 406.07, found 405.95.

### Synthetic example 50. 5-(2-(Phenethylamino)pyrimidin-5-yl)-1,3,4-oxadiazol-2(3H)-one (13a)

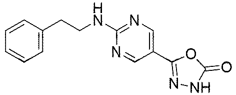

Yield: 7%; ¹H NMR (400 MHz, DMSO-d₆) *δ* 12.43 (s, 1H), 8.74 - 8.43 (m, 2H), 8.15 - 8.02 (m, 1H), 7.31 - 7.17 (m, 5H), 3.59 - 3.51 (m, 2H), 2.88 - 2.80 (m, 2H); MS calculated for C₁₄H₁₄N₅O₂ [M+H]⁺ 284.11, found 284.05.

### Synthetic example 51. 5-(2-((3-Fluorophenethyl)amino)pyrimidin-5-yl)-1,3,4-oxadiazol-2(3H)-one (13b)

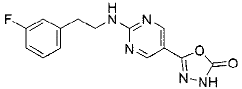

Yield: 10%; ¹H NMR (400 MHz, DMSO-d₆) *δ* 12.43 (s, 1H), 8.62 (d, *J* = 14.7 Hz, 2H), 8.05 (t, *J* = 5.8 Hz, 1H), 7.32 (td, *J* = 8.1, 6.3 Hz, 1H), 7.10 - 6.99 (m, 3H), 3.58 (dt, *J* = 7.7, 6.3 Hz, 2H), 2.88 (t, *J* = 7.2 Hz, 2H);MS calculated for C₁₄H₁₃FN₅O₂ [M+H]⁺ 302.10, found 302.05.

### Synthetic example 52. 5-(2-((4-Fluorophenethyl)amino)pyrimidin-5-yl)-1,3,4-oxadiazol-2(3H)-one (13c)

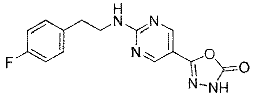

Yield: 11%; ¹H NMR (400 MHz, DMSO-d₆) *δ* 12.43 (s, 1H), 8.62 (d, *J* = 15.0 Hz, 2H), 8.04 (t, *J* = 5.8 Hz, 1H), 7.30 - 7.23 (m, 2H), 7.13 - 7.07 (m, 2H), 3.55 (dt, *J* = 7.8, 6.3 Hz, 2H), 2.85 (t, *J* = 7.3 Hz, 2H); MS calculated for C₁₄H₁₃FN₃O₂ [M+H]⁺ 302.10, found 302.05.

### Synthetic example 53. 5-(2-((3-Chlorophenethyl)amino)pyrimidin-5-yl)-1,3,4-oxadiazol-2(3H)-one (13d)

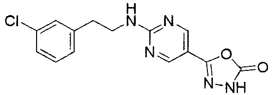

Yield: 18%; ¹H NMR (400 MHz, DMSO-d₆) *δ* 12.43 (s, 1H), 8.62 (d, *J* = 14.2 Hz, 2H), 8.05 (t, *J* = 5.8 Hz, 1H), 7.33 - 7.29 (m, 2H), 7.27 - 7.24 (m, 1H), 7.20 (dt, *J* = 7.4, 1.5 Hz, 1H), 3.57 (dt, *J* = 7.5, 6.4 Hz, 2H), 2.87 (t, *J* = 7.2 Hz, 2H); MS calculated for C₁₄H₁₃ClN₅O₂ [M+H]⁺ 318.08, found 318.00.

### Synthetic example 54. 5-(2-((4-Chlorophenethyl)amino)pyrimidin-5-yl)-1,3,4-oxadiazol-2(3H)-one (13e)

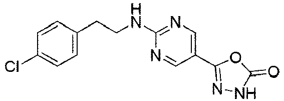

Yield: 27%; ¹H NMR (400 MHz, DMSO-d₆) *δ* 12.43 (s, 1H), 8.62 (d, *J* = 15.7 Hz, 2H), 8.04 (t, *J* = 5.8 Hz, 1H), 7.36 - 7.31 (m, 2H), 7.27 - 7.23 (m, 2H), 3.55 (dt, *J* = 7.7, 6.3 Hz, 2H), 2.85 (t, *J* = 7.2 Hz, 2H); MS calculated for C₁₄H₁₃ClN₅O₂ [M+H]⁺ 318.08, found 318.05.

### Synthetic example 55. 5-(2-((2,3-Dihydro-1H-inden-2-yl)amino)pyrimidin-5-yl)-1,3,4-oxadiazol-2(3H)-one (18a)

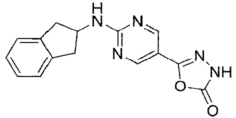

Yield: 30%; ¹H NMR (400 MHz, DMSO-d₆) δ 12.45 (s, 1H), 8.76 - 8.55 (m, 2H), 8.32 (d, *J =* 6.9 Hz, 1H), 7.28 - 7.09 (m, 4H), 4.74 - 4.63 (m, 1H), 3.30 - 3.24 (m, 2H), 2.97 - 2.87 (m, 2H); MS calculated for C₁₅H₁₄N₅O₂ [M+H]⁺ 296.11, found 296.10.

### Synthetic example 56. 5-(2-((5,6-Difluoro-2,3-dihydro-1H-inden-2-yl)amino)pyrimidin-5-yl)-1,3,4-oxadiazol-2(3H)-one (18b)

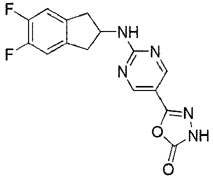

Yield: 42%; ¹H NMR (400 MHz, CD₃OD) *δ* 8.67 (s, 2H), 7.11 (t, J = 9.1 Hz, 2H), 4.82 - 4.78 (m, 2H), 3.36 - 3.33 (m, 1H), 2.92 (dd, J = 15.9, 6.2 Hz, 2H); MS (ESI, m/z) calculated for C₁₅H₁₂F₂N₅O₂ [M+H]⁺ 332.09, found 332.01.

### Synthetic example 57. 5-(2-((5-Bromo-2,3-dihydro-1H-inden-2-yl)amino)pyrimidin-5-yl)-1,3,4-oxadiazol-2(3H)-one (18c)

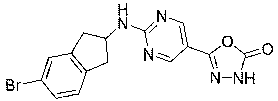

Yield: 15%; ¹H NMR (400 MHz, DMSO-d₆) *δ* 12.45 (s, 1H), 8.66 (d, *J* = 14.5 Hz, 2H), 8.32 (d, *J* = 6.8 Hz, 1H), 7.43 (d, *J* = 1.9 Hz, 1H), 7.33 (dd, *J* = 8.0, 2.0 Hz, 1H), 7.19 (d, *J* = 8.1 Hz, 1H), 4.68 (h, *J* = 6.9 Hz, 1H), 3.28 - 3.18 (m, 2H), 2.91 (td, *J =* 16.1, 8.0 Hz, 2H); MS calculated for C₁₅H₁₃BrN₅O₂ [M+H]⁺ 374.02, found 374.00.

### General procedure for the synthesis of 9a-j, 14a-e, and 19a-c (IV)

**Compounds 8a-j, 13a-e and 18a-c** (1 mmol), (1*R*,5*S*,6*r*)-6-(1*H*-1,2,3-triazol-5-yl)-3-azabicyclo[3.1.0]hexane (3, 1.2 mmol), DIEA (3.5 mmol) and BOP reagent (1.2 mmol) were dissolved in DMF (5 mL). The reaction mixture was stirred at room temperature for 8 h. After the reaction was completed, the solvent was evaporated to dryness. The crude product was extracted with ethyl acetate and washed with brine. The organic layer was dried over anhydrous sodium sulfate and concentrated. The products were separated by column chromatography (DCM/MeOH) to give **9a-j, 14a-e and 19a-c.**

### Example 1. 5-(5-((1R,5S,6r)-6-(1H-1,2,3-Triazol-5-yl)-3-azabicyclo[3.1.0]hexan-3-yl)-1,3,4-oxadiazol-2-yl)-N-benzylpyrimidin-2-amine (9a)

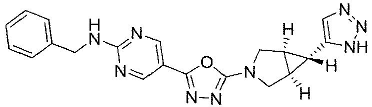

Yield: 36%; ¹H NMR (400 MHz, Acetone-d₆) δ 13.84 (s, 1H), 8.73 (s, 2H), 7.59 (s, 1H), 7.47 - 7.38 (m, 3H), 7.33 - 7.29 (m, 2H), 7.24 (td, *J* = 5.5, 2.5 Hz, 1H), 4.72 - 4.70 (m, 2H), 3.92 (d, *J =* 10.2 Hz, 2H), 3.74 - 3.71 (m, 2H), 2.81 (s, 1H), 2.16 (ddd, *J* = 3.7, 2.5, 1.3 Hz, 2H); ¹³C NMR (100 MHz, Acetone-d₆) δ 162.83, 162.50, 155.73, 155.03, 147.04, 139.77, 130.67, 128.29, 127.37, 126.84, 109.30, 50.01, 44.59, 25.75 (d, *J* = 5.7 Hz), 17.77; HRMS (ESI, m/z) calculated for C₂₀H₂₀N₉O⁺ [M+H]⁺ 402.1785, found 402.1785.

### Example 2. 5-(5-((1R,5S,6r)-6-(1H-1,2,3-Triazol-5-yl)-3-azabicyclo[3.1.0]hexan-3-yl)-1,3,4-oxadiazol-2-yl)-N-(3-chlorobenzyl)pyrimidin-2-amine (9b)

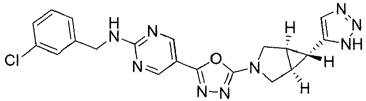

Yield: 21%; ¹H NMR (400 MHz, acetone-d₆) δ 8.75 (s, 2H), 7.59 (s, 1H), 7.52 (t, *J* = 6.5 Hz, 1H), 7.44 - 7.41 (m, 1H), 7.36 - 7.32 (m, 2H), 7.30 - 7.25 (m, 1H), 4.72 (dd, *J* = 6.5, 0.7 Hz, 2H), 3.93 - 3.90 (m, 2H), 3.74 - 3.71 (m, 2H), 2.18 - 2.14 (m, 2H), 2.02 (t, *J* = 3.5 Hz, 1H); ¹³C NMR (100 MHz, DMSO-d₆) δ 163.72, 163.53, 156.65, 156.00, 148.02, 143.45, 134.66, 131.67, 130.99, 128.28, 127.84, 126.88, 110.57, 51.01, 45.07, 26.71, 18.78; HRMS (ESI, m/z) calculated for C₂₀H₁₉ClN₉O⁺ [M+H]⁺ 436.1396, found 436.1395.

### Example 3. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-chlorobenzyl)pyrimidin-2-amine (9c)

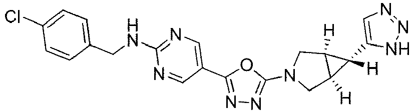

Yield: 24.4%; ¹H NMR (400 MHz, Acetone-d₆) *δ* 13.84 (s, 1H), 8.74 (s, 2H), 7.59 (s, 1H), 7.49 (dd, *J* = 11.8, 5.4 Hz, 1H), 7.43 - 7.40 (m, 2H), 7.36 - 7.32 (m, 2H), 4.70 (d, *J* = 6.4 Hz, 2H), 3.92 (d, *J* = 10.2 Hz, 2H), 3.74 - 3.70 (m, 2H), 2.16 (ddd, *J* = 3.7, 2.6, 1.3 Hz, 2H), 2.02 (t, *J* = 3.6 Hz, 1H); ¹³C NMR (100 MHz, Acetone-d₆) δ 163.71, 163.50, 156.65, 156.01, 148.01, 139.81, 133.05, 131.65, 130.13, 129.27, 110.45, 51.00, 44.93, 26.70, 18.79.; HRMS (ESI, m/z) calculated for C₂₀H₁₉ClN₉O⁺ [M+H]⁺ 436.1396, found 436.1390.

### Example 4. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,4-difluorobenzyl)pyrimidin-2-amine (9d)

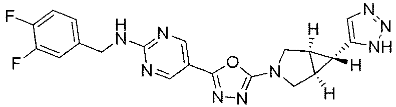

Yield: 31%; ¹H NMR (400 MHz, DMSO-d₆) *δ* 14.58 (s, 1H), 8.76 (s, 2H), 8.47 (t, *J* = 6.4 Hz, 1H), 7.61 (s, 1H), 7.39 (tt, *J* = 7.9, 2.8 Hz, 2H), 7.21 - 7.18 (m, 1H), 4.58 (d, *J* = 6.2 Hz, 2H), 3.86 - 3.82 (m, 2H), 3.72 - 3.67 (m, 2H), 2.14 - 2.10 (m, 2H), 1.97 (t, *J =* 3.6 Hz, 1H); ¹³C NMR (100 MHz, DMSO-d₆) *δ* 162.15, 162.07, 155.62, 155.27, 155.11, 150.00 (dd, *J* = 91.9, 12.6 Hz), 147.56 (dd, *J* = 90.3, 12.6 Hz), 146.46, 137.50 (dd, *J* = 5.3, 3.6 Hz), 130.88, 129.64, 123.74 (dd, *J* = 6.5, 3.4 Hz), 116.66 (dt, *J* = 105.5, 17.3 Hz), 108.54, 49.96, 43.15, 25.39, 17.60.; HRMS (ESI, m/z) calculated for C₂₀H₁₈F₂N₉O⁺ [M+H]⁺ 438.1597, found 438.1599.

### Example 5. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,5-difluorobenzyl)pyrimidin-2-amine (9e)

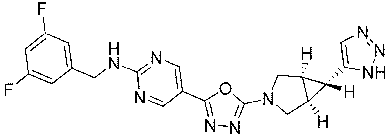

Yield: 35% ¹H NMR (400 MHz, CDCl₃) *δ* 12.91 (s, 1H), 8.80 (s, 2H), 7.49 (s, 1H), 6.90 - 6.84 (m, 2H), 6.71 (tt, *J* = 8.9, 2.4 Hz, 1H), 6.21 (t, *J* = 6.3 Hz, 1H), 4.70 (d, *J* = 6.4 Hz, 2H), 3.97 (d, *J* = 10.3 Hz, 2H), 3.79 - 3.74 (m, 2H), 2.17 (td, *J* = 3.1, 1.3 Hz, 2H), 1.98 (t, *J* = 3.6 Hz, 1H); ¹³C NMR (100 MHz, Acetone-d₆) *δ* 163.99 (dd, *J* = 246.6, 12.8 Hz), 163.62, 163.48, 156.56, 156.01, 147.97, 145.88 (t, *J* = 8.6 Hz), 131.61, 110.98 (dt, *J* = 11.9, 6.4 Hz), 110.70, 102.81 (t, *J* = 25.8 Hz), 50.97, 44.92 (t, *J* = 2.2 Hz), 26.67, 18.73.; HRMS (ESI, m/z) calculated for C₂₀H₁₈F₂N₉O⁺ [M+H]⁺ 438.1597, found 438.1596.

### Example 6. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,4-dichlorobenzyl)pyrimidin-2-amine (9f)

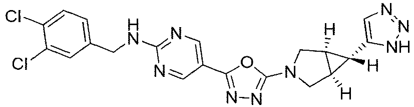

Yield: 25%; ¹H NMR (400 MHz, DMSO-d₆) *δ* 8.71 (d, *J* = 5.7 Hz, 2H), 8.46 (q, *J* = 7.8 Hz, 1H), 7.64 - 7.53 (m, 3H), 7.31 (dd, *J =* 8.3, 2.1 Hz, 1H), 4.58 - 4.51 (m, 2H), 3.81 (d, *J* = 10.2 Hz, 2H), 3.66 (dt, *J =* 10.2, 1.9 Hz, 2H), 2.11 - 2.06 (m, 2H), 1.94 (t, *J =* 3.6 Hz, 1H); ¹³C NMR (100 MHz, DMSO-d₆) *δ* 162.11, 162.07, 155.63, 155.24, 155.10, 144.90, 141.00, 130.84, 130.49, 129.23, 129.07, 127.48, 108.62, 49.95, 43.07, 25.36, 17.29; HRMS (ESI, m/z) calculated for C₂₀H₁₈Cl₂N₉O⁺ [M+H]⁺ 470.1006, found 470.1007.

### Example 7. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,5-dichlorobenzyl)pyrimidin-2-amine (9g)

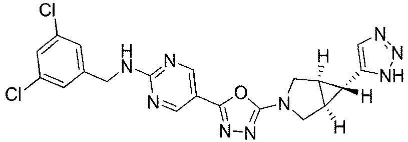

Yield: 15%; ¹H NMR (400 MHz, DMSO-d₆) *δ* 14.66 (s, 1H), 8.73 (s, 2H), 8.45 (t, *J* = 6.4 Hz, 1H), 7.62 (s, 1H), 7.48 (t, *J =* 2.0 Hz, 1H), 7.36 (d, *J* = 1.9 Hz, 2H), 4.57 (d, *J* = 6.3 Hz, 2H), 3.84 - 3.79 (m, 2H), 3.68 - 3.64 (m, 2H), 2.11 - 2.07 (m, 2H), 1.94 (t, *J =* 3.6 Hz, 1H).; ¹³C NMR (100 MHz, DMSO-d₆) *δ* 162.08, 155.74, 155.22, 144.23, 133.93, 129.64, 126.51 - 126.32 (m), 125.84, 108.75, 54.91, 49.96, 43.23, 25.36.; HRMS (ESI, m/z) calculated for C₂₀H₁₈Cl₂N₉O⁺ [M+H]⁺ 470.1006, found 470.1006.

### Example 8. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,5-dibromobenzyl)pyrimidin-2-amine (9h)

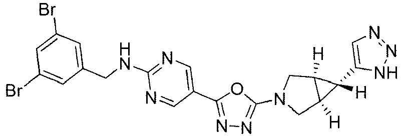

Yield: 22%; ¹H NMR (400 MHz, CDCl₃) δ 12.06 (s, 1H), 8.83 (s, 2H), 7.60 (t, *J* = 1.8 Hz, 1H), 7.52 (s, 1H), 7.46 (dd, *J =* 1.6, 0.8 Hz, 2H), 6.03 (t, *J* = 6.3 Hz, 1H), 4.70 - 4.67 (m, 2H), 4.00 (d, *J* = 10.3 Hz, 2H), 3.79 (dt, *J* = 10.2, 1.9 Hz, 2H), 2.19 (ddd, *J* = 3.7, 2.5, 1.2 Hz, 2H), 2.01 (t, *J* = 3.5 Hz, 1H ¹³C NMR (100 MHz, DMSO-d₆) δ 162.07, 162.04, 155.66, 155.20, 155.10, 144.69, 131.69, 131.69, 129.07, 129.03, 122.36, 108.74, 49.95, 43.08, 25.34, 17.28. HRMS (ESI, m/z) calculated for C₂₀H₁₈Br₂N₉O⁺ [M+H]⁺ 557.9996, found 559.9979.

### Example 9. 5-(5-((1R,5S,6r)-6-(1H-1,2,3-Triazol-5-yl)-3-azabicyclo[3.1.0]hexan-3-yl)-1,3,4-oxadiazol-2-yl)-N-(benzo[d][1,3]dioxol-5-ylmethyl)pyrimidin-2-amine (9i)

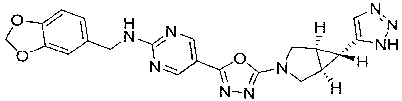

Yield: 35%; ¹H NMR (400 MHz, DMSO-d₆) *δ* 14.66 (s, 1H), 8.71 (s, 2H), 8.34 (t, *J* = 6.4 Hz, 1H), 7.62 (s, 1H), 6.88 (d, *J* = 1.6 Hz, 1H), 6.85 - 6.82 (m, 1H), 6.80 - 6.77 (m, 1H), 5.96 (s, 2H), 4.46 (d, *J* = 6.4 Hz, 2H), 3.83 - 3.78 (m, 2H), 3.69 - 3.64 (m, 2H), 2.11 - 2.07 (m, 2H), 1.94 (t, *J* = 3.5 Hz, 1H); ¹³C NMR (100 MHz, DMSO-d₆) δ 162.16, 162.02, 155.51, 155.33, 155.08, 147.19, 146.02, 144.43, 133.44, 129.62, 120.32, 108.20, 108.00, 107.80, 100.76, 49.95, 43.81, 25.35, 17.40; HRMS (ESI, m/z) calculated for C₂₁H₂₀N₉O₃ [M+H]⁺ 446.1684, found 446.1685.

### Example 10. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,5-bis(trifluoromethyl)benzyl)pyrimidin-2-amine (9j)

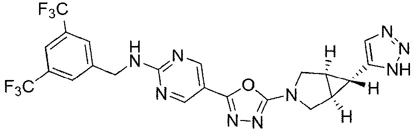

Yield: 43%; ¹H NMR (400 MHz, DMSO-d₆) *δ* 8.75 (d, *J* = 7.7 Hz, 2H), 8.54 (t, *J* = 6.3 Hz, 1H), 8.03 - 7.97 (m, 3H), 7.62 (s, 1H), 4.75 (d, *J* = 6.3 Hz, 2H), 3.81 (d, *J* = 10.2 Hz, 2H), 3.66 (dt, *J* = 10.2, 2.0 Hz, 2H), 2.08 (td, *J* = 3.1, 1.3 Hz, 2H), 1.93 (t, *J* = 3.5 Hz, 1H); ¹³C NMR (100 MHz, DMSO-d₆) *δ* 162.58, 162.56, 156.22, 155.65, 145.35, 143.84, 130.62 (q, *J* = 32.5 Hz), 128.43 (d, *J* = 4.0 Hz), 123.85 (q, *J* = 272.8 Hz), 121.26 - 120.97 (m), 109.36, 50.43, 43.90, 25.83, 17.77; HRMS (ESI, m/z) calculated for C₂₂H₁₈F₆N₉O⁺ [M+H]⁺ 538.1533, found 538.1531.

### Example 11. 5-(5-((1R,5S,6r)-6-(1H-1,2,3-Triazol-5-yl)-3-azabicyclo[3.1.0]hexan-3-yl)-1,3,4-oxadiazol-2-yl)-N-phenethylpyrimidin-2-amine (14a)

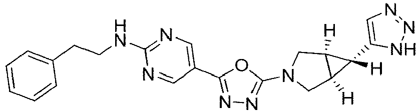

Yield: 36%; ¹H NMR (400 MHz, DMSO-d₆) *δ* 14.69 (s, 1H), 8.71 (d, *J* = 17.3 Hz, 2H), 7.96 (t, *J* = 5.8 Hz, 1H), 7.64 (s, 1H), 7.32 - 7.27 (m, 2H), 7.26 - 7.23 (m, 2H), 7.22 - 7.18 (m, 1H), 3.82 (d, *J* = 10.2 Hz, 2H), 3.67 (dt, *J* = 10.1, 1.9 Hz, 2H), 3.56 (ddd, *J* = 8.6, 7.5, 6.0 Hz, 2H), 2.87 (dd, *J* = 8.3, 6.5 Hz, 2H), 2.12 - 2.07 (m, 2H), 1.95 (t, *J* = 3.5 Hz, 1H); ¹³C NMR (100 MHz, DMSO-d₆) *δ* 162.17, 162.01, 155.41, 155.11, 145.80, 139.44, 131.04, 128.68, 128.31, 126.07, 107.85, 49.96, 42.38, 34.77, 25.38, 17.39; HRMS (ESI, m/z) calculated for C₂₁H₂₂N₉0⁺ [M+H]⁺ 416.1942, found 416.1942.

### Example 12. 5-(5-((1R,5S,6r)-6-(1H-1,2,3-Triazol-5-yl)-3-azabicyclo[3.1.0]hexan-3-yl)-1,3,4-oxadiazol-2-yl)-N-(3-fluorophenethyl)pyrimidin-2-amine (14b)

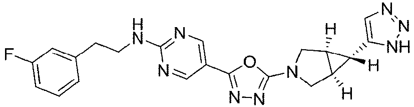

Yield: 41%; ¹H NMR (400 MHz, DMSO-d₆) *δ* 14.66 - 14.62 (m, 1H), 8.70 (d, *J* = 13.6 Hz, 2H), 7.97 (t, *J* = 5.8 Hz, 1H), 7.64 (s, 1H), 7.32 (td, *J* = 8.1, 6.4 Hz, 1H), 7.11 - 7.07 (m, 2H), 7.04 - 6.99 (m, 1H), 3.82 (d, *J* = 10.2 Hz, 2H), 3.67 (dt, *J* = 10.1, 1.9 Hz, 2H), 3.58 (dt, *J* = 7.7, 6.2 Hz, 2H), 2.89 (t, *J* = 7.2 Hz, 2H), 2.11 - 2.07 (m, 2H), 1.95 (t, *J* = 3.6 Hz, 1H); ¹³C NMR (100 MHz, DMSO-d₆) *δ* 162.66 (d, *J* = 242.8 Hz), 162.65, 162.50, 155.87, 155.56, 145.41, 142.94 (d, *J* = 7.5 Hz), 130.59 (d, *J* = 8.3 Hz), 128.82, 125.36 (d, *J* = 2.5 Hz), 115.87 (d, *J* = 20.7 Hz), 113.32 (d, *J* = 20.7 Hz), 108.39, 50.44, 42.45, 34.80 (d, *J* = 1.7 Hz), 25.85, 17.77.; HRMS (ESI, m/z) calculated for C₂₁H₂₁FN₉O⁺ [M+H]⁺ 434.1848, found 434.1847.

### Example 13. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-fluorophenethyl)pyrimidin-2-amine (14c)

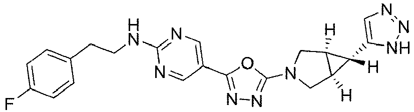

Yield: 33%; ¹H NMR (400 MHz, DMSO-d₆) *δ* 14.70 (s, 1H), 8.70 (d, *J* = 13.3 Hz, 2H), 7.96 (t, *J* = 5.8 Hz, 1H), 7.64 (s, 1H), 7.30 - 7.25 (m, 2H), 7.13 - 7.08 (m, 2H), 3.82 (d, *J* = 10.2 Hz, 2H), 3.67 (dt, *J* = 10.1, 1.9 Hz, 2H), 3.55 (dt, *J* = 7.9, 6.2 Hz, 2H), 2.86 (t, *J* = 7.3 Hz, 2H), 2.11 - 2.08 (m, 2H), 1.95 (t, *J* = 3.6 Hz, 1H); ¹³C NMR (100 MHz, DMSO-d₆) *δ* 162.17, 161.21 (d, *J* = 240.1 Hz), 155.40, 155.08, 145.24, 135.59 (d, *J* = 3.0 Hz), 130.50, 130.43, 129.26, 115.06, 114.85, 107.87, 49.96, 42.36, 33.85, 25.38, 17.30; HRMS (ESI, m/z) calculated for C₂₁H₂₁FN₉O⁺ [M+H]⁺ 434.1848, found 434.1847.

### Example 14. 5-(5-((1R,5S,6r)-6-(1H-1,2,3-Triazol-5-yl)-3-azabicyclo[3.1.0]hexan-3-yl)-1,3,4-oxadiazol-2-yl)-N-(3-chlorophenethyl)pyrimidin-2-amine (14d)

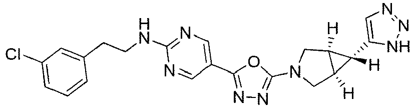

Yield: 28%; ¹H NMR (400 MHz, DMSO-d₆) *δ* 14.68 (s, 1H), 8.70 (d, *J* = 11.7 Hz, 2H), 7.96 (t, *J =* 5.8 Hz, 1H), 7.63 (s, 1H), 7.33 - 7.19 (m, 4H), 3.81 (d, *J =* 10.1 Hz, 2H), 3.66 (dt, *J* = 10.1, 1.9 Hz, 2H), 3.60 - 3.54 (m, 2H), 2.87 (t, *J =* 7.1 Hz, 2H), 2.09 (t, *J* = 2.6 Hz, 2H), 1.94 (t, *J* = 3.5 Hz, 1H); ¹³C NMR (100 MHz, DMSO-d₆) *δ* 162.61, 162.45, 155.82, 155.51, 145.81, 142.55, 133.31, 130.53, 130.07, 129.03, 127.95, 126.50, 108.36, 50.40, 42.42, 34.66, 25.81, 17.77; HRMS (ESI, m/z) calculated for C₂₁H₂₁ClN₉O⁺ [M+H]⁺ 450.1552, found 450.1554.

### Example 15. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-chlorophenethyl)pyrimidin-2-amine (14e)

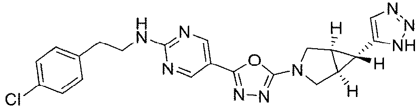

Yield: 56%; ¹H NMR (400 MHz, DMSO-d₆) *δ* 14.66 (s, 1H), 8.66 (d, *J* = 13.0 Hz, 2H), 7.91 (t, *J =* 5.8 Hz, 1H), 7.60 (s, 1H), 7.32 - 7.28 (m, 2H), 7.24 - 7.20 (m, 2H), 3.78 (d, *J* = 10.2 Hz, 2H), 3.63 (dt, *J* = 10.1, 1.9 Hz, 2H), 3.52 (dt, *J* = 7.7, 6.3 Hz, 2H), 2.82 (t, *J* = 7.2 Hz, 2H), 2.07 - 2.04 (m, 2H), 1.91 (t, *J* = 3.5 Hz, 1H); ¹³C NMR (100 MHz, DMSO-d₆) *δ* 162.60, 162.45, 155.83, 155.51, 145.55, 138.94, 131.15, 131.04, 128.69 - 128.58 (m), 108.35, 50.40, 42.56, 34.40, 25.81, 17.74; HRMS (ESI, m/z) calculated for C₂₁H₂₁ClN₉O⁺ [M+H]⁺ 450.1552, found 450.1552.

### Example 16. 5-(5-((1R,5S,6r)-6-(1H-1,2,3-Triazol-5-yl)-3-azabicyclo[3.1.0]hexan-3-yl)-1,3,4-oxadiazol-2-yl)-N-(2,3-dihydro-1H-inden-2-yl)pyrimidin-2-amine (19a)

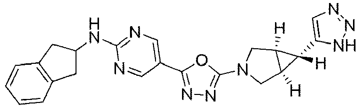

Yield: 48%; ¹H NMR (400 MHz, CDCl₃+CD₃OD) *δ* 8.76 (s, 2H), 7.50 (s, 1H), 7.28 - 7.15 (m, 4H), 4.84 (tt, *J* = 7.1, 5.5 Hz, 1H), 3.96 (d, *J* = 10.2 Hz, 2H), 3.82 - 3.77 (m, 2H), 3.41 (dd, *J* = 15.9, 7.2 Hz, 2H), 2.96 (dd, *J* = 15.9, 5.6 Hz, 2H), 2.22 - 2.18 (m, 2H), 2.00 - 1.97 (m, 1H); ¹³C NMR (100 MHz, CD₃OD) δ 162.48, 162.26, 156.46, 156.06, 146.06, 141.25, 130.70, 127.03, 125.02, 108.72, 52.96, 50.63, 40.01, 29.95, 25.96; HRMS (ESI, m/z) calculated for C₂₂H₂₂N₉O⁺ [M+H]⁺ 428.1942, found 428.1943.

### Example 17. 5-(5-((1R,5S,6r)-6-(1H-1,2,3-Triazol-5-yl)-3-azabicyclo[3.1.0]hexan-3-yl)-1,3,4-oxadiazol-2-yl)-N-(5,6-difluoro-2,3-dihydro-1H-inden-2-yl)pyrimidin-2-amine (19b)

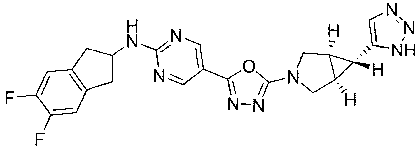

Yield: 76%; ¹H NMR (400 MHz, CDCl₃+CD₃OD) *δ* 8.77 (s, 2H), 7.50 (s, 1H), 7.05 (t, *J =* 8.9 Hz, 2H), 4.89 - 4.83 (m, 1H), 4.04 (s, 1H), 3.97 - 3.94 (m, 2H), 3.82 - 3.78 (m, 2H), 3.39 (d, *J =* 7.2 Hz, 1H), 3.34 (s, 1H), 2.92 (dd, *J =* 16.0, 5.8 Hz, 2H), 2.22 - 2.19 (m, 2H), 1.99 (t, *J* = 3.6 Hz, 1H); ¹³C NMR (100 MHz, CD₃OD) δ 162.57, 162.30, 156.50, 156.13, 150.01 (dd, *J* = 246.8, 14.9 Hz), 137.69 - 137.19 (m), 130.07, 113.65 (dd, *J* = 12.1, 6.6 Hz), 108.92, 53.41, 50.70, 39.58, 29.99, 26.02; HRMS (ESI, m/z) calculated for C₂₂H₂₀F₂N₉O⁺ [M+H]⁺ 464.1753, found 464.1755.

### Example 18. 5-(5-((1R,5S,6r)-6-(1H-1,2,3-Triazol-5-yl)-3-azabicyclo[3.1.0]hexan-3-yl)-1,3,4-oxadiazol-2-yl)-N-(5-bromo-2,3-dihydro-1H-inden-2-yl)pyrimidin-2-amine (19c)

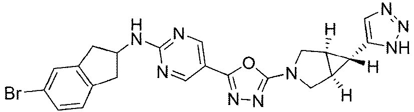

Yield: 30%; ¹H NMR (400 MHz, DMSO-d₆) *δ* 14.71 (s, 1H), 8.74 (s, 2H), 8.23 (d, *J* = 6.8 Hz, 1H), 7.63 (s, 1H), 7.42 (d, *J* = 1.9 Hz, 1H), 7.36 - 7.31 (m, 1H), 7.19 (d, *J* = 8.0 Hz, 1H), 4.68 (h, *J* = 6.9 Hz, 1H), 3.82 (d, *J* = 10.2 Hz, 2H), 3.67 (dt, *J* = 10.1, 1.9 Hz, 2H), 3.28 - 3.21 (m, 2H), 2.97 - 2.85 (m, 2H), 2.11 - 2.07 (m, 2H), 1.95 (t, *J* = 3.5 Hz, 1H); ¹³C NMR (100 MHz, DMSO-d₆) δ 162.05, 161.90, 155.39, 155.12, 144.36, 140.80, 139.77, 129.16, 127.43, 126.61 - 126.49 (m), 119.26, 108.15, 66.36, 54.92, 52.23, 49.98, 38.72, 38.30, 25.39, 17.32; HRMS (ESI, m/z) calculated for C₂₂H₂₁BrN₉O⁺ [M+H]⁺ 506.1047, found 506.1047.

### Synthetic example 58. Ethyl (E)-3-(2-chloropyrimidin-5-yl)acrylate (21)

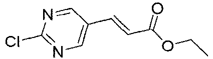

A mixture of 5-bromo-2-chloropyrimidine (0.19 g, 1.00 mmol), ethyl acrylate (0.42 mL, 4.00 mmol), palladium(II) diacetate (8.98 mg, 0.04 mmol), and tri(o-tolyl)phosphine (30.43 mg, 0.10 mmol) in dimethylformamide (2 mL) and diisopropylethylamine (1 mL) was heated to reflux for 4 h, then cooled to room temperature. The solvent was evaporated to dryness, and extracted with ethyl acetate. The extract was washed with brine, dried over sodium sulfate, and concentrated. The residue was purified by column chromatography (ethyl acetate/hexane) to give the title product; Yield: 74 %; ¹H NMR (400 MHz, CDCl₃) δ 8.76 (d, *J* = 0.5 Hz, 2H), 7.58 (dd, *J* = 16.2, 0.6 Hz, 1H), 6.58 (d, *J = 16.2* Hz, 1H), 4.30 (d, *J* = 7.1 Hz, 2H), 1.35 (t, *J =* 7.2 Hz, 3H).

### Synthetic example 59. Ethyl (E)-3-(2-((2,3-dihydro-1H-inden-2-yl)amino)pyrimidin-5-yl)acrylate (22)

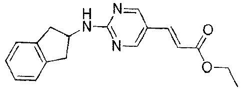

Compound **22** was synthesized according to General Procedure I using compound 21 and 2-aminoindane; Yield: 82%; ¹H NMR (400 MHz, DMSO-d₆) *δ* 8.69 (s, 2H), 8.09 (d, *J =* 6.9 Hz, 1H), 7.50 (d, *J =* 16.1 Hz, 1H), 7.25 - 7.12 (m, 4H), 6.53 (d, *J =* 16.1 Hz, 1H), 4.66 (h, *J =* 7.1 Hz, 1H), 4.17 (q, *J =* 7.1 Hz, 2H), 3.26 (dd, *J =* 15.8, 7.6 Hz, 2H), 2.91 (dd, *J* = 15.8, 6.8 Hz, 2H), 1.25 (t, *J* = 7.1 Hz, 3H).

### Synthetic example 60. Ethyl 3-(2-((2,3-dihydro-1H-inden-2-yl)amino)pyrimidin-5-yl)propanoate (23)

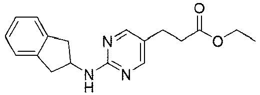

Compound 23 (1 mmol) was dissolved in EtOH (5 mL) / DCM (5 ml), and palladium on carbon was added to the mixture at room temperature. The reaction mixture was stirred at room temperature under hydrogen atmosphere for 6 h. The mixture was filtered through Celite, and the filtrate was concentrated. The residue was purified by column chromatography (ethyl acetate/hexane) to give the title compound; Yield: 61%; ¹H NMR (400 MHz, CDCl₃) δ 8.01 (s, 2H), 7.22 - 7.15 (m, 4H), 6.12 (t, *J* = 6.9 Hz, 1H), 4.78 (dqd, *J* = 7.2, 5.0, 2.4 Hz, 1H), 4.13 (q, *J =* 7.1 Hz, 2H), 3.37 (dd, *J* = 16.0, 7.1 Hz, 2H), 2.86 (dd, *J =* 16.0, 4.9 Hz, 2H), 2.71 (t, *J =* 7.5 Hz, 2H), 2.54 - 2.49 (m, 2H), 1.24 (t, *J* = 7.1 Hz, 3H).

### Synthetic example 61. 3-(2-((2,3-Dihydro-1H-inden-2-yl)amino)pyrimidin-5-yl)propanoic acid (24)

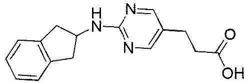

A solution of lithium hydroxide (0.12 g, 5.00 mmol) in water (2 mL) was added to a solution of compound **23** in methanol. The reaction mixture was stirred for 1 h. After the solvent was evaporated, the mixture was dissolved in water again and acidified (pH 4-5) with 1 M hydrochloric acid, and a white solid precipitated. The precipitate was filtered to obtain the title product; Yield: 89%; ¹H NMR (400 MHz, DMSO-d₆) *δ* 12.15 (s, 1H), 8.19 (s, 2H), 7.29 (d, *J =* 6.8 Hz, 1H), 7.20 (dd, *J =* 5.4, 3.4 Hz, 2H), 7.16 - 7.10 (m, 2H), 4.57 (h, *J* = 7.1 Hz, 1H), 3.22 (dd, *J* = 15.7, 7.5 Hz, 2H), 2.86 (dd, *J* = 15.7, 7.0 Hz, 2H), 2.62 (t, *J* = 7.3 Hz, 2H).

### Example 19. 1-((1R,5S,6r)-6-(1H-1,2,3-Triazol-5-yl)-3-azabicyclo[3.1.0]hexan-3-yl)-3-(2-((2,3-dihydro-1H-inden-2-yl)amino)pyrimidin-5-yl)propan-1-one (25)

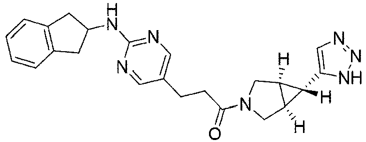

Compound **24** (1 mmol), **4** (1.2 mmol), DIEA (3.5 mmol), and HATU (1.2 mmol) were dissolved in DCM (5 mL). The reaction mixture was stirred at room temperature for 2 h. After the reaction was completed, the solvent was evaporated to dryness. The crude product was extracted with ethyl acetate and washed with brine. The organic layer was dried over anhydrous sodium sulfate and concentrated. The product was separated by column chromatography (DCM/MeOH) to obtain the title compound; Yield: 50%; ¹H NMR (400 MHz, CDCl₃) δ 8.18 (s, 2H), 7.42 (s, 1H), 7.23 - 7.13 (m, 4H), 5.77 (d, *J =* 7.7 Hz, 1H), 4.83 - 4.68 (m, 1H), 3.98 (d, *J =* 12.2 Hz, 1H), 3.67 - 3.58 (m, 2H), 3.54 - 3.48 (m, 1H), 3.37 (dd, *J* = 15.9, 7.1 Hz, 2H), 2.90 - 2.78 (m, 4H), 2.57 - 2.42 (m, 2H), 2.05 - 1.96 (m, 2H), 1.71 (t, *J* = 3.5 Hz, 1H); ¹³C NMR (100 MHz, CDCl₃) δ 171.21, 160.90, 158.02, 146.17, 141.26, 129.70, 126.61, 124.78, 122.55, 52.54, 49.16, 48.33, 40.21, 36.23, 25.98, 24.88, 24.67, 18.47; HRMS (ESI, m/z) calculated for C₂₃H₂₆N₇O⁺ [M+H]⁺416.2193, found 416.2193.

### Example 20. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-(trifluoromethyl)benzyl)pyrimidin-2-amine (9k)

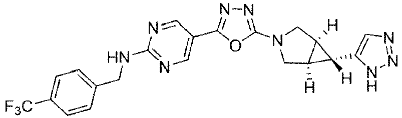

### Step 1: ethyl 2-((4-(trifluoromethyl)benzyl)amino)pyrimidine-5-carboxylate (6k)

After (4-(trifluoromethyl)phenyl)methanamine (110 mg, 0.63 mmol) was dissolved in anhydrous 1,4-dioxane (2.1 mL), DIPEA (214 uL, 1.26 mmol) and ethyl 2-chloropyrimidin-5-carboxylate (106 mg, 0.57 mmol) were added sequentially, and the mixture was stirred at 100 °C for 2 hours. The reaction mixture was cooled to room temperature, DW (6 mL) was added, and the mixture was stirred for 30 minutes. The resulting solid was filtered, washed with DW, and dried in vacuo to give compound 6k (144 mg, yield: 78%) as a yellow solid.
¹H NMR (400 MHz, CDCl3) δ 8.85 (s, 2H), 7.59 (d, J = 8.0 Hz, 2H), 7.45 (d, J = 7.9 Hz, 2H), 6.05 (s, 1H), 4.78 (d, J = 6.2 Hz, 2H), 4.35 (q, J = 7.1 Hz, 2H), 1.37 (t, J = 7.0 Hz, 3H); LCMS *m*/*z* 326[M+H]⁺

### Step 2: 2-((4-(trifluoromethyl)benzyl)amino)pyrimidine-5-carbohydrazide (7k)

A reaction mixture of compound 6k (142 mg, 0.44 mmol), hydrazine monohydrate (0.49 mL, 6.55 mmol), and EtOH (4.9 mL) was stirred at 100°C for 15 h. After cooling to room temperature, DW was added, and the mixture was stirred for 30 min. The resulting solid was filtered and dried under vacuum to obtain compound 7k (107 mg, yield: 79%) as an off-white solid.
¹H NMR (400 MHz, CDCl3) δ 8.68 (s, 2H), 7.60 (d, J = 8.3 Hz, 2H), 7.45 (d, J = 8.1 Hz, 2H), 7.08 (s, 1H), 5.86 (s, 1H), 4.77 (d, J = 6.2 Hz, 2H), 4.06 (s, 2H); LCMS *m*/*z* 312[M+H]⁺

### Step 3: 5-(2-((4-(trifluoromethyl)benzyl)amino)pyrimidin-5-yl)-1,3,4-oxadiazol-2(3H)-one (8k)

Compound 7k (105 mg, 0.34 mmol) was dissolved in THF (1.8 mL) and cooled to 0 °C. TEA (47 uL, 0.34 mmol) and CDI (65.6 mg, 0.41 mmol) were slowly added, and the mixture was stirred at 0 °C for 15 minutes. After stirring for 20 hours at room temperature, DW was added and stirred for 30 minutes. The resulting solid was filtered and dried in vacuo to obtain compound 8k (107 mg, yield: 79%) as a white solid.
LCMS *m*/*z* 338[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-(trifluoromethyl)benzyl)pyrimidin-2-amine (9k)

Compound 8k (30.1 mg, 0.09 mmol) and (1R,5S,6r)-6-(1H-1,2,3-triazol-5-yl)-3-azabicyclo[3.1.0]hexane hydrochloride (20 mg, 0.11 mmol) were dissolved in DMF (1.8 mL) and cooled to 0 °C. DIPEA (76 uL, 0.45 mmol) was added and stirred for 10 minutes. After that, BOP reagent (47.6 mg, 0.11 mmol) was added and stirred at room temperature for 22 hours. DW was added to the reaction mixture, extracted with EtOAc, and the organic layer was washed with brine. The organic layer was dried over MgSO₄, filtered, and concentrated, and the residue was purified by silica gel column chromatography (0-1% MeOH/DCM). The obtained residue was developed on prep TLC (5% MeOH/DCM -> 7% MeOH/DCM) to obtain compound 9k (6.5 mg, yield: 15%) as a white solid.
¹H NMR (400 MHz, DMSO-d₆) δ 14.66 (s, 1H), 8.72 (s, 2H), 8.48 (t, *J =* 6.3 Hz, 1H), 7.68 (d, *J* = 8.2 Hz, 2H), 7.62 (s, 1H), 7.53 (d, *J* = 8.0 Hz, 2H), 4.65 (d, *J* = 6.2 Hz, 2H), 3.81 (d, *J* = 10.2 Hz, 2H), 3.66 (d, *J* = 10.0 Hz, 2H), 2.09 (s, 2H), 1.93 (t, *J* = 3.5 Hz, 1H); LCMS *m*/*z* 470[M+H]⁺

### Example 21. 5-(5-((1R,5S,6r)-6-(1H-1,2,3-Triazol-5-yl)-3-azabicyclo[3.1.0]hexan-3-yl)-1,3,4-oxadiazol-2-yl)-N-(3-(trifluoromethyl)benzyl)pyrimidin-2-amine (9l)

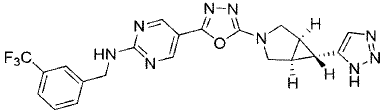

The synthesis was performed in the same manner as Example 20, except that (3-(trifluoromethyl)phenyl)methanamine was used instead of (4-(trifluoromethyl)phenyl)methanamine.

### Step 1: ethyl 2-((3-(trifluoromethyl)benzyl)amino)pyrimidine-5-carboxylate (6l)

Off-white solid (150 mg, yield: 81 %); LCMS *m*/*z* 326[M+H]⁺

### Step 2: 2-((3-(trifluoromethyl)benzyl)amino)pyrimidine-5-carbohydrazide (7l)

White solid (93 mg, yield: 65 %); LCMS *m*/*z* 312[M+H]⁺

### Step 3: 5-(2-((3-(trifluoromethyl)benzyl)amino)pyrimidin-5-yl)-1,3,4-oxadiazol-2(3H)-one (8l)

White solid (90 mg, yield: 89 %); LCMS *m*/*z* 338[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(3-(trifluoromethyl)benzyl)pyrimidin-2-amine (9l)

Yellow solid (20 mg, yield: 38 %); ¹H NMR (400 MHz, DMSO-d₆) δ 14.73 (d, J = 168.1 Hz, 1H), 8.72 (s, 2H), 8.47 (t, J = 6.3 Hz, 1H), 7.68 - 7.53 (m, 5H), 4.66 (d, J = 6.3 Hz, 2H), 3.81 (d, J = 10.2 Hz, 2H), 3.66 (d, J = 10.0 Hz, 2H), 2.08 (s, 2H), 1.94 (s, 1H); LCMS *m*/*z* 470[M+H]⁺

### Example 22. 5-(5-((1R,5S,6r)-6-(1H-1,2,3-Triazol-5-yl)-3-azabicyclo[3.1.0]hexan-3-yl)-1,3,4-oxadiazol-2-yl)-N-(2-(trifluoromethyl)benzyl)pyrimidin-2-amine (9m)

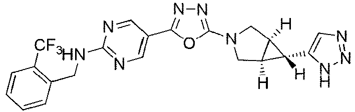

The synthesis was performed in the same manner as Example 20, except that (2-(trifluoromethyl)phenyl)methanamine was used instead of (4-(trifluoromethyl)phenyl)methanamine.

### Step 1: ethyl 2-((2-(trifluoromethyl)benzyl)amino)pyrimidine-5-carboxylate (6m)

Off-white solid (250 mg, yield: 71 %); LCMS *m*/*z* 326[M+H]⁺

### Step 2: 2-((2-(trifluoromethyl)benzyl)amino)pyrimidine-5-carbohydrazide (7m)

White solid (194 mg, yield: 81 %); ¹H NMR (400 MHz, DMSO-d₆) δ 9.55 (s, 1H), 8.68 (s, 2H), 8.27 (t, J = 6.2 Hz, 1H), 7.72 (d, J = 7.7 Hz, 1H), 7.61 (t, J = 7.5 Hz, 1H), 7.51 - 7.41 (m, 2H), 4.75 (d, J = 6.1 Hz, 2H), 4.41 (s, 2H); LCMS *m*/*z* 312[M+H]⁺

### Step 3: 5-(2-((2-(trifluoromethyl)benzyl)amino)pyrimidin-5-yl)-1,3,4-oxadiazol-2(3H)-one (8m)

Off-white solid (141 mg, quantitative); LCMS *m*/*z* 338[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(2-(trifluoromethyl)benzyl)pyrimidin-2-amine (9m)

Off-white solid (21 mg, yield: 51 %); ¹H NMR (400 MHz, DMSO-d₆) δ 14.65 (broad s, 1H), 8.72 (broad s, 2H), 8.42 (t, J = 6.1 Hz, 1H), 7.73 (d, J = 7.8 Hz, 1H), 7.63 (t, J = 7.6 Hz, 2H), 7.51 (d, J = 7.7 Hz, 1H), 7.46 (t, J = 7.6 Hz, 1H), 4.77 (d, J = 6.0 Hz, 2H), 3.81 (d, J = 10.2 Hz, 2H), 3.66 (d, J = 9.6 Hz, 2H), 2.09 (s, 2H), 1.94 (t, J = 3.4 Hz, 1H); LCMS *m*/*z* 470[M+H]⁺

### Example 23. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-(difluoromethyl)benzyl)pyrimidin-2-amine (9n)

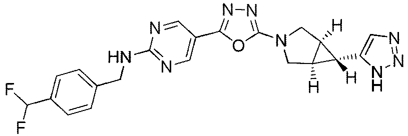

The synthesis was performed in the same manner as Example 20, except that (4-(difluoromethyl)phenyl)methanamine was used instead of (4-(trifluoromethyl)phenyl)methanamine.

### Step 1: ethyl 2-((4-(difluoromethyl)benzyl)amino)pyrimidine-5-carboxylate (6n)

Off-white solid (165 mg, yield: 95 %); ¹H NMR (400 MHz, CDCl₃) δ 8.85 (s, 2H), 7.49 (d, J = 8.1 Hz, 2H), 7.43 (d, J = 8.1 Hz, 2H), 6.63 (t, J = 56.5 Hz, 1H), 5.97 (s, 1H), 4.76 (d, J = 6.2 Hz, 2H), 4.35 (q, J = 7.1 Hz, 2H), 1.37 (t, J = 7.1 Hz, 3H); LCMS *m*/*z* 308[M+H]⁺

### Step 2: 2-((4-(difluoromethyl)benzyl)amino)pyrimidine-5-carbohydrazide (7n)

Off-white solid (124 mg, yield: 80 %); ¹H NMR (400 MHz, CD₃OD) δ 8.67 (s, 2H), 7.46 (q, J = 8.6 Hz, 4H), 6.71 (t, J = 56.3 Hz, 1H), 4.69 (s, 2H); LCMS *m*/*z* 294[M+H]⁺

### Step 3: 5-(2-((4-(difluoromethyl)benzyl)amino)pyrimidin-5-yl)-1,3,4-oxadiazol-2(3H)-one (8n)

Off-white solid (106 mg, yield: 80 %); LCMS *m*/*z* 320[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-(difluoromethyl)benzyl)pyrimidin-2-amine (9n)

Off-white solid (16.5 mg, yield: 41%); ¹H NMR (400 MHz, DMSO-d₆) δ 14.52 (s, 1H), 8.71 (s, 2H), 8.45 (t, J = 6.3 Hz, 1H), 7.57 (s, 1H), 7.52 (d, J = 8.1 Hz, 2H), 7.45 (d, J = 8.0 Hz, 2H), 6.99 (t, J = 56.0 Hz, 1H), 4.62 (d, J = 6.1 Hz, 2H), 3.81 (d, J = 10.2 Hz, 2H), 3.66 (d, J = 9.9 Hz, 2H), 2.08 (s, 2H), 1.94 (t, J = 3.3 Hz, 1H); LCMS *m*/*z* 452[M+H]⁺

### Example 24. 4-(((5-(5-((1R,5S,6r)-6-(1H-1,2,3-Triazol-5-yl)-3-azabicyclo[3.1.0]hexan-3-yl)-1,3,4-oxadiazol-2-yl)pyrimidin-2-yl)amino)methyl)benzonitrile (9o)

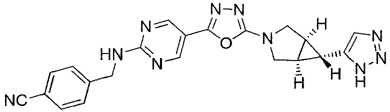

The synthesis was performed in the same manner as Example 20, except that 4-(aminomethyl)benzonitrile was used instead of (4-(trifluoromethyl)phenyl)methanamine.

### Step 1: ethyl 2-((4-cyanobenzyl)amino)pyrimidine-5-carboxylate (6o)

Off-white solid (136 mg, yield: 81 %); LCMS *m*/*z* 283[M+H]⁺

### Step 2: 2-((4-cyanobenzyl)amino)pyrimidine-5-carbohydrazide (7o)

Pink solid (92 mg, yield: 71 %); LCMS *m*/*z* 269[M+H]⁺

### Step 3: 4-(((5-(5-oxo-4,5-dihydro-1,3,4-oxadiazol-2-yl)pyrimidin-2-yl)amino)methyl)benzonitrile (8o)

Pink solid (85 mg, yield: 84 %); LCMS *m*/*z* 295[M+H]⁺

### Step 4: 4-(((5-(5-((1R,5S,6r)-6-(1H-1,2,3-triazol-5-yl)-3-azabicyclo[3.1.0]hexan-3-yl)-1,3,4-oxadiazol-2-yl)pyrimidin-2-yl)amino)methyl)benzonitrile (9o)

Pink solid (23 mg, yield: 49 %); ¹H NMR (400 MHz, DMSO-d₆) δ 14.67 (s, 1H), 8.72 (s, 2H), 8.48 (t, J = 6.3 Hz, 1H), 7.78 (d, J = 8.2 Hz, 2H), 7.62 (s, 1H), 7.50 (d, J = 8.2 Hz, 2H), 4.64 (d, J = 6.3 Hz, 2H), 3.81 (d, J = 10.2 Hz, 2H), 3.66 (d, J = 9.9 Hz, 2H), 2.09 (s, 2H), 1.94 (t, J = 3.5 Hz, 1H); LCMS *m*/*z* 427[M+H]⁺

### Example 25. 3-(((5-(5-((1R,5S,6r)-6-(1H-1,2,3-Triazol-5-yl)-3-azabicyclo[3.1.0]hexan-3-yl)-1,3,4-oxadiazol-2-yl)pyrimidin-2-yl)amino)methyl)benzonitrile (9p)

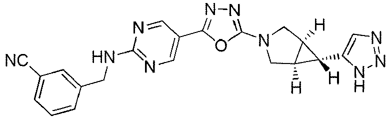

The synthesis was performed in the same manner as Example 20, except that 3-(aminomethyl)benzonitrile was used instead of (4-(trifluoromethyl)phenyl)methanamine.

### Step 1: ethyl 2-((3-cyanobenzyl)amino)pyrimidine-5-carboxylate (6p)

Yellow solid (175 mg, yield: 82 %); LCMS *m*/*z* 283[M+H]⁺

### Step 2: 2-((3-cyanobenzyl)amino)pyrimidine-5-carbohydrazide (7p)

White solid (139 mg, yield: 84 %); LCMS *m*/*z* 269[M+H]⁺

### Step 3: 3-(((5-(5-oxo-4,5-dihydro-1,3,4-oxadiazol-2-yl)pyrimidin-2-yl)amino)methyl)benzonitrile (8p)

Off-white solid (136 mg, yield: 89 %); LCMS *m*/*z* 295[M+H]⁺

### Step 4: 3-(((5-(5-((1R,5S,6r)-6-(1H-1,2,3-triazol-5-yl)-3-azabicyclo[3.1.0]hexan-3-yl)-1,3,4-oxadiazol-2-yl)pyrimidin-2-yl)amino)methyl)benzonitrile (9p)

Yellow solid (28 mg, yield: 59%); ¹H NMR (400 MHz, DMSO-d₆) δ 14.64 (s, 1H), 8.73 (s, 2H), 8.44 (t, J = 6.3 Hz, 1H), 7.75 (s, 1H), 7.73 - 7.58 (m, 3H), 7.54 (t, J = 7.7 Hz, 1H), 4.62 (d, J = 6.3 Hz, 2H), 3.81 (d, J = 10.2 Hz, 2H), 3.66 (d, J = 10.0 Hz, 2H), 2.09 (s, 2H), 1.94 (t, J = 3.5 Hz, 1H); LCMS *m*/*z* 427[M+H]⁺

### Example 26. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-(trifluoromethyl)phenethyl)pyrimidin-2-amine (14f)

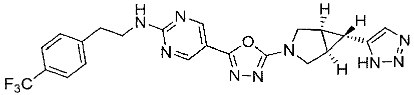

The synthesis was performed in the same manner as Example 20, except that 2-(4-(trifluoromethyl)phenyl)ethan-1-amine was used instead of (4-(trifluoromethyl)phenyl)methanamine.

### Step 1: ethyl 2-((4-(trifluoromethyl)phenethyl)amino)pyrimidine-5-carboxylate (11f)

Light brown solid (245 mg, yield: 77 %); LCMS *m*/*z* 340 [M+H]⁺

### Step 2: 2-((4-(trifluoromethyl)phenethyl)amino)pyrimidine-5-carbohydrazide (12f)

White solid (177 mg, yield: 83 %); LCMS *m*/*z* 326 [M+H]⁺

### Step 3: 5-(2-((4-(trifluoromethyl)phenethyl)amino)pyrimidin-5-yl)-1,3,4-oxadiazol-2(3H)-one (13f)

Yellow solid (123 mg, yield: 65 %); ¹H NMR (400 MHz, DMSO-d₆) δ 12.46 (s, 1H), 8.61 (d, J = 5.9 Hz, 2H), 8.03 (t, J = 5.8 Hz, 1H), 7.64 (d, J = 8.1 Hz, 2H), 7.46 (d, J = 8.0 Hz, 2H), 3.61 (dd, J = 13.4, 6.7 Hz, 2H), 2.96 (t, J = 7.1 Hz, 2H); LCMS *m*/*z* 352 [M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-(trifluoromethyl)phenethyl)pyrimidin-2-amine (14f)

White solid (7.5 mg, yield:18 %); ¹H NMR (400 MHz, DMSO-d₆) δ 15.06 -14.42 (m, 1H), 8.70 (s, 2H), 7.95 (t, J = 5.8 Hz, 1H), 7.64 (d, J = 8.1 Hz, 2H), 7.58 (s, 1H), 7.47 (d, J = 8.0 Hz, 2H), 3.82 (d, J = 10.2 Hz, 2H), 3.67 (d, J = 10.3 Hz, 2H), 3.61 (dd, J = 13.5, 6.7 Hz, 2H), 2.98 (dd, J = 12.5, 5.3 Hz, 2H), 2.08 (s, 2H), 1.94 (s, 1H); LCMS *m*/*z* 484 [M+H]⁺

### Example 27. 5-(5-((1R,58,6r)-6-(1H-1,2,3-Triazol-5-yl)-3-azabicyclo[3.1.0]hexan-3-yl)-1,3,4-oxadiazol-2-yl)-N-(4-bromophenethyl)pyrimidin-2-amine (14g)

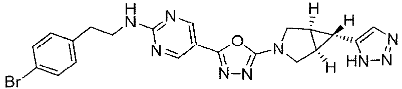

The synthesis was performed in the same manner as in Example 20, except that 2-(4-bromophenyl)ethan-1-amine was used instead of (4-(trifluoromethyl)phenyl)methanamine.

### Step 1: ethyl 2-((4-bromophenethyl)amino)pyrimidine-5-carboxylate (11g)

Off-white solid (150 mg, yield: 86 %); LCMS *m*/*z* 350 [M+H]⁺

### Step 2: 2-((4-bromophenethyl)amino)pyrimidine-5-carbohydrazide (12g)

White solid (97 mg, yield: 67 %); LCMS *m*/*z* 336 [M+H]⁺

### Step 3: 5-(2-((4-bromophenethyl)amino)pyrimidin-5-yl)-1,3,4-oxadiazol-2(3H)-one (13g)

Off-white solid (66 mg, yield: 63 %); LCMS *m*/*z* 362 [M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-bromophenethyl)pyrimidin-2-amine (14g)

Off-white solid (19 mg, yield: 35 %); ¹H NMR (400 MHz, DMSO-d₆) δ 15.08 - 14.43 (m, 1H), 8.69 (s, 2H), 7.91 (t, *J =* 5.8 Hz, 1H), 7.87 - 7.55 (m, 1H), 7.50 - 7.44 (m, 2H), 7.21 (d, *J* = 8.4 Hz, 2H), 3.82 (d, *J* = 10.2 Hz, 2H), 3.67 (d, *J* = 9.8 Hz, 2H), 3.61 - 3.52 (m, 2H), 2.84 (t, *J* = 7.2 Hz, 2H), 2.09 (s, 2H), 1.95 (t, *J* = 3.4 Hz, 1H); LCMS *m*/*z* 494 [M+H]⁺

### Example 28. 5-(5-((1R,5S,6r)-6-(1H-1,2,3-Triazol-5-yl)-3-azabicyclo[3.1.0]hexan-3-yl)-1,3,4-oxadiazol-2-yl)-N-(2-chlorophenethyl)pyrimidin-2-amine (14h)

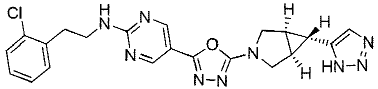

The synthesis was performed in the same manner as in Example 20, except that 2-(2-chlorophenyl)ethan-1-amine was used instead of (4-(trifluoromethyl)phenyl)methanamine.

### Step 1: ethyl 2-((2-chlorophenethyl)amino)pyrimidine-5-carboxylate (11h)

Off-white solid (167 mg, yield:85 %); LCMS *m*/*z* 306 [M+H]⁺

### Step 2: 2-((2-chlorophenethyl)amino)pyrimidine-5-carbohydrazide (12h)

White solid (126 mg, yield: 79 %); LCMS *m*/*z* 292 [M+H]⁺

### Step 3: 5-(2-((2-chlorophenethyl)amino)pyrimidin-5-yl)-1,3,4-oxadiazol-2(3H)-one (13h)

Off-white solid (95 mg, yield: 69 %); LCMS *m*/*z* 318 [M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(2-chlorophenethyl)pyrimidin-2-amine (14h)

Off-white solid (28 mg, yield: 56 %); ¹H NMR (400 MHz, DMSO-d₆) δ 15.21 - 14.31 (m, 1H), 8.70 (s, 2H), 7.96 (t, J = 5.8 Hz, 1H), 7.88 - 7.47 (m, 1H), 7.41 (dd, J = 7.4, 1.8 Hz, 1H), 7.35 (dd, J = 7.2, 2.1 Hz, 1H), 7.29 - 7.21 (m, 2H), 3.82 (d, J = 10.2 Hz, 2H), 3.67 (d, J = 9.9 Hz, 2H), 3.63 - 3.56 (m, 2H), 3.00 (t, J = 7.2 Hz, 2H), 2.09 (s, 2H), 1.95 (t, J = 3.4 Hz, 1H); LCMS *m*/*z* 450 [M+H]⁺

### Example 29. 5-(5-((1R,5S,6r)-6-(1H-1,2,3-Triazol-5-yl)-3-azabicyclo[3.1.0]hexan-3-yl)-1,3,4-oxadiazol-2-yl)-N-(3,5-dichlorophenethyl)pyrimidin-2-amine (14i)

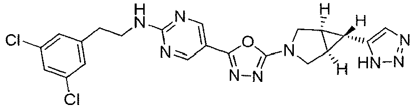

The synthesis was performed in the same manner as in Example 20, except that 2-(3,5-dichlorophenyl)ethan-1-amine was used instead of (4-(trifluoromethyl)phenyl)methanamine.

### Step 1: ethyl 2-((3,5-dichlorophenethyl)amino)pyrimidine-5-carboxylate (11i)

Light brown solid (144 mg, yield: 44 %); ¹H NMR (400 MHz, CDCl₃) δ 8.84 (s, 2H), 7.24 (t, *J =* 1.9 Hz, 1H), 7.12 (d, *J* = 1.9 Hz, 2H), 5.59 (t, *J =* 5.9 Hz, 1H), 4.36 (q, *J* = 7.1 Hz, 2H), 3.75 (dd, *J* = 13.5, 6.9 Hz, 2H), 2.90 (t, *J* = 7.1 Hz, 2H), 1.38 (t, *J* = 7.1 Hz, 3H); LCMS *m*/*z* 340 [M+H]⁺

### Step 2: 2-((3,5-dichlorophenethyl)amino)pyrimidine-5-carbohydrazide (12i)

White solid (120 mg, yield: 86 %); LCMS *m*/*z* 326 [M+H]⁺

### Step 3: 5-(2-((3,5-dichlorophenethyl)amino)pyrimidin-5-yl)-1,3,4-oxadiazol-2(3H)-one (13i)

White solid (108 mg, yield: 84 %); ¹H NMR (400 MHz, DMSO-d₆) δ 12.41 (s, 1H), 8.61 (s, 2H), 8.02 (t, *J* = 5.7 Hz, 1H), 7.41 (t, *J* = 1.8 Hz, 1H), 7.31 (d, *J* = 1.9 Hz, 2H), 3.59 (q, *J* = 6.7 Hz, 2H), 2.88 (t, *J* = 6.9 Hz, 2H); LCMS *m*/*z* 352[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(3,5-dichlorophenethyl)pyrimidin-2-amine (14i)

White solid (7 mg, yield: 17 %); ¹H NMR (400 MHz, DMSO-d₆) δ 14.6 (s, 1H), 8.70 (s, 2H), 7.93 (t, J = 5.8 Hz, 1H), 7.61 (s, 1H), 7.41 (t, J = 1.8 Hz, 1H), 7.32 (d, J = 1.8 Hz, 2H), 3.82 (d, J = 10.2 Hz, 2H), 3.67 (d, J = 10.1 Hz, 2H), 3.59 (dd, J = 12.8, 6.6 Hz, 2H), 2.89 (t, J = 6.8 Hz, 2H), 2.09 (s, 2H), 1.94 (t, J = 3.5 Hz, 1H); LCMS *m*/*z* 483[M+H]⁺

### Example 30. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-methoxyphenethyl)pyrimidin-2-amine (14j)

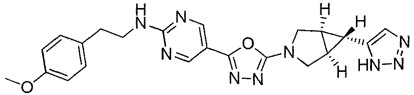

The synthesis was performed in the same manner as in Example 20, except that 2-(4-methoxyphenyl)ethan-1-amine was used instead of (4-(trifluoromethyl)phenyl)methanamine.

### Step 1: ethyl 2-((4-methoxyphenethyl)amino)pyrimidine-5-carboxylate (11j)

Brown solid (122 mg, yield: 72 %); ¹H NMR (400 MHz, CDCl₃) δ 8.83 (d, J = 31.4 Hz, 2H), 7.14 (d, J = 8.4 Hz, 2H), 6.85 (d, J = 8.5 Hz, 2H), 5.56 (s, 1H), 4.35 (q, J = 7.1 Hz, 2H), 3.79 (s, 3H), 3.73 (dd, J = 13.2, 6.7 Hz, 2H), 2.87 (t, J = 6.9 Hz, 2H), 1.37 (t, J = 7.1 Hz, 3H); LCMS *m*/*z* 302[M+H]⁺

### Step 2: 2-((4-methoxyphenethyl)amino)pyrimidine-5-carbohydrazide (12j)

Brown solid (44 mg, yield: 38 %); ¹H NMR (400 MHz, CD₃OD) δ 8.65 (s, 2H), 7.14 (d, J = 8.6 Hz, 2H), 6.83 (d, J = 8.6 Hz, 2H), 3.75 (s, 3H), 3.66 - 3.57 (m, 2H), 2.84 (t, J = 7.3 Hz, 2H); LCMS *m*/*z* 288[M+H]⁺

### Step 3: 5-(2-((4-methoxyphenethyl)amino)pyrimidin-5-yl)-1,3,4-oxadiazol-2(3H)-one (13j)

Off-white solid (44 mg, yield: 38 %); LCMS *m*/*z* 314[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-methoxyphenethyl)pyrimidin-2-amine (14j)

Off-white solid (13.7 mg, yield: 34 %); ¹H NMR (400 MHz, DMSO-d₆) δ 14.52 (s, 1H), 8.70 (d, J = 9.6 Hz, 2H), 7.89 (t, J = 5.8 Hz, 1H), 7.58 (s, 1H), 7.15 (d, J = 8.6 Hz, 2H), 6.91 - 6.73 (m, 2H), 3.82 (d, J = 10.2 Hz, 2H), 3.71 (s, 3H), 3.67 (d, J = 9.9 Hz, 2H), 3.52 (dd, J = 14.5, 6.2 Hz, 2H), 2.79 (t, J = 7.4 Hz, 2H), 2.09 - 2.08 (m, 2H), 1.95 (t, J = 3.2 Hz, 1H); LCMS *m*/*z* 446 [M+H]⁺

### Example 31. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-methylphenethyl)pyrimidin-2-amine (14k)

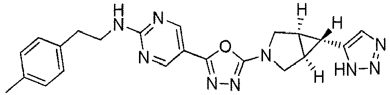

The synthesis was performed in the same manner as in Example 20, except that 2-(p-tolyl)ethan-1-amine was used instead of (4-(trifluoromethyl)phenyl)methanamine.

### Step 1: ethyl 2-((4-methylphenethyl)amino)pyrimidine-5-carboxylate (11k)

Light brown solid (167 mg, yield: 84%); LCMS *m*/*z* 286[M+H]⁺

### Step 2: 2-((4-methylphenethyl)amino)pyrimidine-5-carbohydrazide (12k)

White solid (128 mg, yield: 81%); LCMS *m*/*z* 272[M+H]⁺

### Step 3: 5-(2-((4-methylphenethyl)amino)pyrimidin-5-yl)-1,3,4-oxadiazol-2(3H)-one (13k)

White solid (72 mg, yield: 51%); ¹H NMR (400 MHz, DMSO-d₆) δ 12.40 (s, 1H), 8.62 (d, *J* = 17.7 Hz, 2H), 7.98 (t, *J* = 5.8 Hz, 1H), 7.15 - 7.00 (m, 4H), 3.58 - 3.49 (m, 2H), 2.81 (t, *J* = 7.4 Hz, 2H), 2.26 (s, 3H); LCMS *m*/*z* 298 [M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-methylphenethyl)pyrimidin-2-amine (14k)

White solid (12 mg, yield: 32 %); ¹H NMR (400 MHz, DMSO-d₆) δ 15.14 - 14.35 (m, 1H), 8.69 (s, 2H), 7.89 (t, *J* = 5.8 Hz, 1H), 7.58 (s, 1H), 7.11 (q, *J* = 8.2 Hz, 4H), 3.82 (d, *J* = 10.2 Hz, 2H), 3.67 (d, *J* = 10.2 Hz, 2H), 3.53 (dd, *J* = 14.6, 6.2 Hz, 2H), 2.81 (t, *J* = 7.1 Hz, 2H), 2.26 (s, 3H), 2.09 (s, 2H), 1.94 (t, *J* = 3.5Hz, 1H); LCMS *m*/*z* 430 [M+H]⁺

### Example 32. 5-(5-((1R,5S,6r)-6-(1H-1,2,3-Triazol-5-yl)-3-azabicyclo[3.1.0]hexan-3-yl)-1,3,4-oxadiazol-2-yl)-N-(2-(pyridin-2-yl)ethyl)pyrimidin-2-amine (30a)

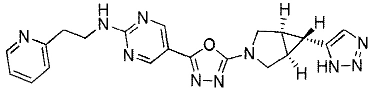

The synthesis was performed in the same manner as Example 20, except that 2-(pyridin-2-yl)ethan-1-amine was used instead of (4-(trifluoromethyl)phenyl)methanamine.

### Step 1: ethyl 2-((2-(pyridin-2-yl)ethyl)amino)pyrimidine-5-carboxylate (27a)

Light brown solid (160 mg, yield: 73%); LCMS *m*/*z* 273[M+H]⁺

### Step 2: 2-((2-(pyridin-2-yl)ethyl)amino)pyrimidine-5-carbohydrazide (28a)

Yellow solid (144 mg, yield: 95%); LCMS *m*/*z* 259[M+H]⁺

### Step 3: 5-(2-((2-(pyridin-2-yl)ethyl)amino)pyrimidin-5-yl)-1,3,4-oxadiazol-2(3H)-one (29a)

White solid (145mg, yield: 92%); ¹H NMR (400 MHz, DMSO-d₆) δ 12.33 (s, 1H), 8.62 (d, *J* = 13.9 Hz, 2H), 8.52 - 8.47 (m, 1H), 8.00 (t, *J* = 5.8 Hz, 1H), 7.69 (td, *J* = 7.6, 1.9 Hz, 1H), 7.27 (d, *J* = 7.8 Hz, 1H), 7.21 (ddd, *J =* 7.5, 4.9, 1.0 Hz, 1H), 3.75 - 3.66 (m, 2H), 3.01 (t, *J =* 7.3 Hz, 2H); LCMS *m*/*z* 285 [M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(2-(pyridin-2-yl)ethyl)pyrimidin-2-amine (30a)

Yellow solid (8.9mg, yield: 25%); ¹H NMR (400 MHz, DMSO-d₆) δ 15.04 - 14.46 (m, 1H), 8.69 (s, 2H), 8.53 - 8.48 (m, 1H), 7.91 (t, *J* = 5.9 Hz, 1H), 7.69 (td, *J* = 7.6, 1.9 Hz, 1H), 7.58 (s, 1H), 7.27 (d, *J* = 7.8 Hz, 1H), 7.24 - 7.19 (m, 1H), 3.82 (d, *J* = 10.1 Hz, 2H), 3.76 - 3.65 (m, 4H), 3.02 (t, *J* = 7.3 Hz, 1H), 2.09 (s, 2H), 1.94 (t, *J* = 3.1 Hz, 1H); LCMS *m*/*z* 417[M+H]⁺

### Example 33. 4-(2-((5-(5-((1R,5S,6r)-6-(1H-1,2,3-Triazol-5-yl)-3-azabicyclo[3.1.0]hexan-3-yl)-1,3,4-oxadiazol-2-yl)pyrimidin-2-yl)amino)ethyl)benzonitrile (14l)

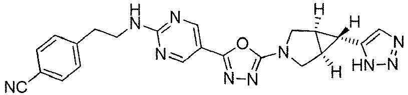

The synthesis was performed in the same manner as in Example 20, except that 4-(2-aminoethyl)benzonitrile was used instead of (4-(trifluoromethyl)phenyl)methanamine.

### Step 1: ethyl 2-((4-cyanophenethyl)amino)pyrimidine-5-carboxylate (111)

Brown solid (138 mg, yield: 83 %); ¹H NMR (400 MHz, CDCl₃) δ 8.83 (s, 2H), 7.60 (d, J = 8.1 Hz, 2H), 7.33 (d, J = 8.0 Hz, 2H), 5.61 (s, 1H), 4.36 (q, J = 7.1 Hz, 2H), 3.78 (q, J = 6.7 Hz, 2H), 3.00 (t, J = 7.0 Hz, 2H), 1.38 (t, J = 7.1 Hz, 3H); LCMS *m*/*z* 297[M+H]⁺

### Step 2: 2-((4-cyanophenethyl)amino)pyrimidine-5-carbohydrazide (121)

Off-white solid (70 mg, yield: 54 %); ¹H NMR (400 MHz, CD₃OD) δ 8.65 (s, 2H), 7.63 (d, J = 8.2 Hz, 2H), 7.43 (d, J = 8.1 Hz, 2H), 3.70 (t, J = 7.2 Hz, 2H), 3.00 (t, J = 7.1 Hz, 2H); LCMS *m*/*z* 283[M+H]⁺

### Step 3: 4-(2-((5-(5-oxo-4,5-dihydro-1,3,4-oxadiazol-2-yl)pyrimidin-2-yl)amino)ethyl)benzonitrile (131)

Off-white solid (61 mg, yield: 82 %); LCMS *m*/*z* 309[M+H]⁺

### Step 4: 4-(2-((5-(5-((1R,5S,6r)-6-(1H-1,2,3-triazol-5-yl)-3-azabicyclo[3.1.0]hexan-3-yl)-1,3,4-oxadiazol-2-yl)pyrimidin-2-yl)amino)ethyl)benzonitrile (141)

White solid (5.6mg, yield: 14%); ¹H NMR (400 MHz, DMSO-d₆) δ 14.52 (s, 1H), 8.69 (s, 2H), 7.94 (t, J = 5.7 Hz, 1H), 7.75 (d, J = 8.2 Hz, 2H), 7.58 (d, J = 1.5 Hz, 1H), 7.45 (d, J = 8.2 Hz, 2H), 3.82 (d, J = 10.2 Hz, 2H), 3.67 (d, J = 9.7 Hz, 2H), 3.61 (dd, J = 13.2, 6.8 Hz, 2H), 2.96 (t, J = 7.1 Hz, 2H), 2.09 (s, 2H), 1.95 (t, J = 3.4 Hz, 1H); LCMS *m*/*z* 441[M+H]⁺

### Example 34. 5-(5-((1R,5S,6R)-6-(1H-1,2,3-Triazol-5-yl)-3-azabicyclo[3.1.0]hexan-3-yl)-1,3,4-oxadiazol-2-yl)-N-((R)-2,3-dihydro-1H-inden-1-yl)pyrimidin-2-amine (35a)

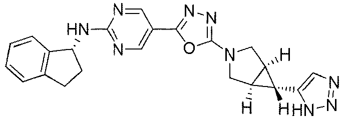

The synthesis was performed in the same manner as in Example 20, except that (R)-2,3-dihydro-1H-inden-1-amine was used instead of (4-(trifluoromethyl)phenyl)methanamine.

### Step 1: ethyl (R)-2-((2,3-dihydro-1H-inden-1-yl)amino)pyrimidine-5-carboxylate (32a)

Brown solid (175mg, yield: 82%); LCMS *m*/*z* 284[M+H]⁺

### Step 2: (R)-2-((2,3-dihydro-1H-inden-1-yl)amino)pyrimidine-5-carbohydrazide (33a)

Off-white solid (105mg, yield: 63%); LCMS *m*/*z* 270[M+H]⁺

### Step 3: (R)-5-(2-((2,3-dihydro-1H-inden-1-yl)amino)pyrimidin-5-yl)-1,3,4-oxadiazol-2(3H)-one (34a)

Off-white solid (40mg, yield: 35%); LCMS *m*/*z* 296[M+H]⁺

### Step 4: 5-(5-((1R,5S,6R)-6-(1H-1,2,3-triazol-5-yl)-3-azabicyclo[3.1.0]hexan-3-yl)-1,3,4-oxadiazol-2-yl)-N-((R)-2,3-dihydro-1H-inden-1-yl)pyrimidin-2-amine (35a)

Yellow solid (26mg, yield: 55%); ¹H NMR (400 MHz, DMSO-d₆) δ 14.66 (s, 1H), 8.75 (s, 2H), 8.23 (d, *J* = 8.5 Hz, 1H), 7.63 (s, 1H), 7.27 - 7.13 (m, 4H), 5.61 (q, *J* = 8.0 Hz, 1H), 3.83 (d, *J* = 10.2 Hz, 2H), 3.68 (d, *J* = 10.0 Hz, 2H), 2.99 (ddd, *J* = 15.7, 8.7, 3.2 Hz, 1H), 2.89 - 2.78 (m, 1H), 2.49 - 2.43 (m, 1H), 2.10 (s, 2H), 2.03 - 1.92 (m, 2H); LCMS *m*/*z* 428[M+H]⁺

### Example 35. 5-(5-((1R,5S,6S)-6-(1H-1,2,3-Triazol-5-yl)-3-azabicyclo[3.1.0]hexan-3-yl)-1,3,4-oxadiazol-2-yl)-N-((S)-2,3-dihydro-1H-inden-1-yl)pyrimidin-2-amine (35b)

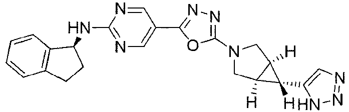

The synthesis was performed in the same manner as in Example 20, except that (S)-2,3-dihydro-1H-inden-1-amine was used instead of (4-(trifluoromethyl)phenyl)methanamine.

### Step 1: ethyl (S)-2-((2,3-dihydro-1H-inden-1-yl)amino)pyrimidine-5-carboxylate (32b)

Brown solid (151mg, yield: 71%); LCMS *m*/*z* 284[M+H]⁺

### Step 2: (S)-2-((2,3-dihydro-1H-inden-1-yl)amino)pyrimidine-5-carbohydrazide (33b)

White solid (122mg, yield: 85%); LCMS *m*/*z* 270[M+H]⁺

### Step 3: (S)-5-(2-((2,3-dihydro-1H-inden-1-yl)amino)pyrimidin-5-yl)-1,3,4-oxadiazol-2(3H)-one (34b)

Off-white solid (104mg, yield: 78%); LCMS *m*/*z* 296[M+H]⁺

### Step 4: 5-(5-((1R,5S,6S)-6-(1H-1,2,3-triazol-5-yl)-3-azabicyclo[3.1.0]hexan-3-yl)-1,3,4-oxadiazol-2-yl)-N-((S)-2,3-dihydro-1H-inden-1-yl)pyrimidin-2-amine (35b)

Yellow solid (30 mg, yield: 63 %); ¹H NMR (400 MHz, DMSO-d₆) δ 15.13 - 14.36 (m, 1H), 8.75 (s, 2H), 8.23 (d, *J* = 8.5 Hz, 1H), 7.97 - 7.40 (m, 1H), 7.29 - 7.11 (m, 4H), 5.61 (q, *J* = 8.0 Hz, 1H), 3.83 (d, *J* = 10.2 Hz, 2H), 3.68 (d, *J* = 9.9 Hz, 2H), 2.99 (ddd, *J* = 15.7, 8.7, 3.2 Hz, 1H), 2.90 - 2.77 (m, 1H), 2.48 - 2.41 (m, 1H), 2.16 - 2.06 (m, 2H), 2.01 - 1.91 (m, 2H); LCMS *m*/*z* 428[M+H]⁺

### Example 36. 5-(5-((1R,5S,6r)-6-(1H-1,2,3-Triazol-5-yl)-3-azabicyclo[3.1.0]hexan-3-yl)-1,3,4-oxadiazol-2-yl)-N-phenylpyrimidin-2-amine (30b)

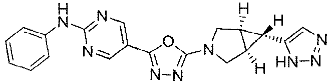

The synthesis was performed in the same manner as in Example 20, except that aniline was used instead of (4-(trifluoromethyl)phenyl)methanamine.

### Step 1: ethyl 2-(phenylamino)pyrimidine-5-carboxylate (27b)

Yellow solid (79 mg, yield: 61 %); LCMS *m*/*z* 244[M+H]⁺

### Step 2: 2-(phenylamino)pyrimidine-5-carbohydrazide (28b)

White solid (112 mg, yield: 77 %); LCMS *m*/*z* 230[M+H]⁺

### Step 3: 5-(2-(phenylamino)pyrimidin-5-yl)-1,3,4-oxadiazol-2(3H)-one (29b)

Yellow solid (56 mg, yield: 56 %); LCMS *m*/*z* 256[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-phenylpyrimidin-2-amine (30b)

Yellow solid (15 mg, yield: 35 %); ¹H NMR (400 MHz, DMSO-d₆) δ 15.17 - 14.30 (m, 1H), 10.14 (s, 1H), 8.89 (s, 2H), 7.77 (d, *J* = 7.7 Hz, 2H), 7.58 (d, *J =* 1.2 Hz, 1H), 7.36 - 7.29 (m, 2H), 7.02 (t, *J =* 7.4 Hz, 1H), 3.85 (d, *J =* 10.2 Hz, 2H), 3.69 (d, *J =* 9.9 Hz, 2H), 2.10 (s, 2H), 1.99 - 1.93 (m, 1H); LCMS *m*/*z* 388[M+H]⁺

### Example 37. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-chlorophenethyl)-N-methylpyrimidin-2-amine (39)

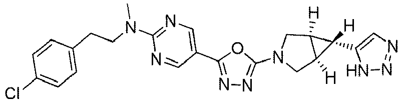

### Step 1: ethyl 2-((4-chlorophenethyl)(methyl)amino)pyrimidine-5-carboxylate (36)

Compound 11e (0.3 g, 0.981 mmol) was dissolved in DMF (9.8 mL), and NaH (60%, dispersed in mineral oil) (59 mg, 1.47 mmol) was added at 0°C and stirred for 30 minutes. After that, iodomethane (92 µL, 1.47 mmol) was added at the same temperature and stirred at room temperature for 6 hours. Distilled water was added to the reaction mixture, extracted with EtOAc, dried over Na₂SO₄, filtered, and concentrated. The obtained residue was purified by silica gel column chromatography (10-15% EtOAc in Hex) to obtain compound 36 (0.275 g, yield: 87.6%) as a white solid.
¹H NMR (400 MHz, DMSO-d₆) δ 8.76 (s, 2H), 7.34 - 7.31 (m, 2H), 7.27 - 7.24 (m, 2H), 4.26 (q, J = 7.1 Hz, 2H), 3.92 - 3.86 (m, 2H), 3.11 (s, 3H), 2.91 - 2.85 (m, 2H), 1.29 (t, J = 7.1 Hz, 3H); LCMS *m*/*z* 320[M+H]⁺

Steps 2 through 4 of Example 37 were synthesized in the same manner as Steps 2 through 4 of Example 20.

### Step 2: 2-((4-chlorophenethyl)(methyl)amino)pyrimidine-5-carbohydrazide (37)

White solid (241mg, yield: 92.3%); ¹H NMR (400 MHz, DMSO-d₆) δ 9.56 (s, 1H), 8.71 (s, 2H), 7.34 - 7.31 (m, 2H), 7.27 - 7.24 (m, 2H), 4.40 (s, 2H), 3.90 - 3.80 (m, 2H), 3.08 (s, 3H), 2.90 - 2.83 (m, 2H); LCMS m/z 306 [M+H]⁺

### Step 3: 5-(2-((4-chlorophenethyl)(methyl)amino)pyrimidin-5-yl)-1,3,4-oxadiazol-2(3H)-one (38)

White solid (250mg, yield: 95.6%); ¹H NMR (400 MHz, DMSO-d₆) δ 8.66 (s, 2H), 7.35 - 7.31 (m, 2H), 7.28 - 7.25 (m, 2H), 3.91 - 3.84 (m, 2H), 3.10 (s, 3H), 2.92 - 2.85 (m, 2H). LCMS *m*/*z* 332 [M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-chlorophenethyl)-N-methylpyrimidin-2-amine (39)

White solid (10mg, yield: 23.8%); ¹H NMR (400 MHz, DMSO-d₆) δ 8.75 (s, 2H), 7.35 - 7.32 (m, 2H), 7.27 (d, J = 8.5 Hz, 2H), 3.91 - 3.85 (m, 2H), 3.82 (d, J = 10.2 Hz, 2H), 3.67 (d, J = 9.8 Hz, 2H), 3.11 (s, 3H), 2.91 - 2.87 (m, 2H), 2.09 (s, 2H), 1.95 (s, 1H), 1.24 (s, 1H); LCMS *m*/*z* 464[M+H]⁺

### Example 38. 1-((1R,5S,6r)-6-(1H-1,2,3-Triazol-5-yl)-3-azabicyclo[3.1.0]hexan-3-yl)-4-(5-((2,3-dihydro-1H-inden-2-yl)amino)-1,3,4-thiadiazol-2-yl)butan-1-one (44b)

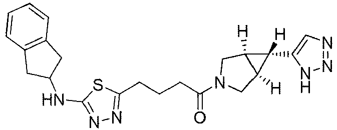

### Step 1: 2-Isothiocyanato-2,3-dihydro-1H-indene (40)

2,3-Dihydro-1H-inden-2-amine (478 mg, 3.58 mmol) and DIEA (1.83 mL, 10.7 mmol) were added to DCM (20 mL) and maintained at 0 °C under a nitrogen atmosphere. O,O'-di-2-pyridyl thiocarbonate (1.0 g, 4.3 mmol) dissolved in DCM (5 mL) was added dropwise for 5 minutes, and the reaction temperature was raised to room temperature and reacted for 12 hours. Distilled water was added to the reaction solution, extracted with DCM, and the organic layer was dried over Na₂SO₄, filtered, and concentrated under reduced pressure. The obtained residue was purified by silica gel column chromatography (100% n-hexane) to obtain compound 40 (560 mg, yield: 89%) as a white solid.
¹H NMR (400 MHz, CDCl3) δ 7.25 - 7.19 (m, 4H), 4.54 - 4.50 (m, 1H), 3.34 (dd, J = 20, 4.0 Hz, 2H), 3.18 (dd, J = 20, 4.0 Hz, 2H)

### Step 2: N-(2,3-dihydro-1H-inden-2-yl)hydrazinecarbothioamide (41)

Compound 40 (560 mg, 3.19 mmol) was dissolved in EtOH (20 mL), and hydrazine monohydrate (776 uL, 16 mmol) was added and stirred at room temperature for 12 h. After removing all the solvent, distilled water was added, and the mixture was extracted with EtOAc. The organic layer was dried over Na₂SO₄, filtered, and concentrated under reduced pressure. The obtained residue was recrystallized from (EtOAc/n-hexane) to obtain compound 41 (580 mg, yield: 87%) as a white solid.
¹H NMR (400 MHz, CDCl3) δ 7.56 (s, 1H), 7.27 - 7.16 (m, 4H), 5.21 - 5.13 (m, 1H), 3.68 (s, 2H), 3.46 (dd, J = 20, 4.0 Hz, 2H), 2.96 (dd, J = 20, 4.0 Hz, 2H), 1.56 (s, 1H); LCMS *m*/*z* 208 [M+H]⁺

### Step 3: ethyl 4-(5-((2,3-dihydro-1H-inden-2-yl)amino)-1,3,4-thiadiazol-2-yl)butanoate (42b)

Compound 41 (150 mg, 0.75 mmol) and ethyl 4-cyanobutanoate (106 mg, 0.75 mmol) were added to TFA (4.5 mL) and stirred at 80 ° C for 12 h. After all the solvent in the reaction mixture was removed, saturated aqueous sodium bicarbonate solution was added to neutralize, and extracted with DCM. The organic layer was dried over Na₂SO₄, filtered, and concentrated under reduced pressure. The obtained residue was purified by silica gel column chromatography (0-2% MeOH in DCM) to obtain compound 42b (120 mg, yield: 50%) as a white solid.
¹H NMR (400 MHz, DMSO-d₆) δ 7.78 (d, *J =* 8.0 Hz, 1H), 7.25 - 7.22 (m, 2H), 7.17 -7.13 (m, 2H), 4.43 (m, 1H), 4.07 (q, *J =* 8.0 Hz, 2H), 3.31 (dd, *J =* 24, 8.0 Hz, 2H), 2.91 - 2.81 (m, 4H), 2.37 (t, *J =* 8.0 Hz, 2H), 1.87 (t, *J =* 8.0 Hz, 2H), 1.18 (t, *J =* 8.0 Hz, 3H); LCMS *m*/*z* 332 [M+H]⁺

### Step 4: 4-(5-((2,3-dihydro-1H-inden-2-yl)amino)-1,3,4-thiadiazol-2-yl)butanoic acid (43b)

A solution of LiOH (21.7 mg, 0.91 mmol) dissolved in H₂O (390 uL) was added to a mixture of compound 42b (60 mg, 0.19 mmol) dissolved in THF/MeOH (3.5 mL/ 390 uL), and the mixture was stirred at room temperature for 3 hours. After completion of the reaction, all the solvent was removed and the pH was adjusted to 3 using 1 N HCl at 0 ° C. The resulting solid was filtered to obtain compound 43b (30 mg, yield: 55%) as a yellow solid.
¹H NMR(400 MHz, DMSO-d₆) δ 7.93 (d, *J* = 8.0 Hz, 1H), 7.25 - 7.22 (m, 2H), 7.17 -7.14 (m, 2H), 4.43 (m, 1H), 3.31 (dd, *J =* 24, 8.0 Hz, 2H), 2.91 - 2.81 (m, 4H), 2.30 (t, *J =* 8.0 Hz, 2H), 1.84 (t, *J =* 8.0 Hz, 2H); LCMS *m*/*z* 304[M+H]⁺

### Step 5: 1-((1R,5S,6r)-6-(1H-1,2,3-triazol-5-yl)-3-azabicyclo[3.1.0]hexan-3-yl)-4-(5-((2,3-dihydro-1H-inden-2-yl)amino)-1,3,4-thiadiazol-2-yl)butan-1-one (44b)

A reaction mixture of compound 43b (30 mg, 0.10 mmol), compound 4 (27.7 mg, 0.15 mmol), HATU (47 mg, 0.12 mmol), DIEA (60 uL, 0.35 mmol) and DCM (1.5 mL) was stirred at room temperature for 12 h. Distilled water was added to the reaction solution, and the mixture was extracted with DCM. The organic layer was dried over Na₂SO₄, filtered and concentrated under reduced pressure. The obtained residue was purified by silica gel column chromatography (0-25% MeOH in CH₂Cl₂) to obtain compound 44b (24 mg, yield: 56%) as a white solid.
¹H NMR (400 MHz, DMSO-d₆) δ 14.5(s, 1H), 7.85 (d, *J =* 8.0 Hz, 1H), 7.56 (s, 1H), 7.24 - 7.21 (m, 2H), 7.17 -7.14 (m, 2H), 4.43 (m, 1H), 3.76 (dd, *J* = 16, 4.0 Hz, 2H), 3.61 (dd, *J* = 12, 4.0 Hz, 1H), 3.38 (dd, *J* = 16, 4.0 Hz, 1H), 3.31 (dd, *J* = 24, 8.0 Hz, 2H), 2.91 - 2.81 (m, 4H), 2.39 - 2,22 (m, 2H), 1.98 (m, 1H), 1.92 (m, 1H), 1.85 (t, *J* = 8.0 Hz, 2H), 1.77 (m, 1H); LCMS *m*/*z* 436 [M+H]⁺

### Example 39. 1-((1R,5S,6r)-6-(1H-1,2,3-Triazol-5-yl)-3-azabicyclo[3.1.0]hexan-3-yl)-3-(5-((2,3-dihydro-1H-inden-2-yl)amino)-1,3,4-thiadiazol-2-yl)propan-1-one (44a)

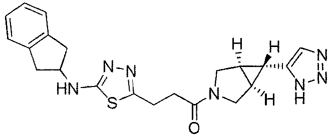

The synthesis was performed in the same manner as steps 3-5 of Example 38, except that ethyl 3-cyanopropanoate was used instead of ethyl 4-cyanobutanoate.

### Step 1: ethyl 3-(5-((2,3-dihydro-1H-inden-2-yl)amino)-1,3,4-thiadiazol-2-yl)propanoate (42a)

White solid (110mg, yield: 46%); ¹H NMR (400 MHz, CDCl3) δ 7.25 - 7.17 (m, 4H), 5.23 (s, 1H), 4.47 (s, 1H), 4.18 (q, J = 8.0 Hz, 2H), 3.42 (dd, J = 24, 8.0 Hz, 2H), 3.21 (t, J = 8.0 Hz, 2H), 2.98 (dd, J = 20, 4.0 Hz, 2H), 2.80 (t, J = 8.0 Hz, 2H), 1.27 (t, J = 8.0 Hz, 3H); LCMS *m*/*z* 318 [M+H]⁺

### Step 2: 3-(5-((2,3-dihydro-1H-inden-2-yl)amino)-1,3,4-thiadiazol-2-yl)propanoic acid (43a)

White solid (38mg, yield: 69%); ¹H NMR (400 MHz, DMSO-d₆) δ 7.85 (d, *J* = 8.0 Hz, 1H), 7.25 - 7.14 (m, 4H), 4.42 (m, 1H), 3.30 (dd, *J* = 24, 8.0 Hz, 2H), 3.02 (t, *J =* 8.0 Hz, 2H), 2.90 (dd, *J =* 20, 4.0 Hz, 2H), 2.63 (t, *J =* 8.0 Hz, 2H); LCMS *m*/*z* 290 [M+H]⁺

### Step 3: 1-((1R,5S,6r)-6-(1H-1,2,3-triazol-5-yl)-3-azabicyclo[3.1.0]hexan-3-yl)-3-(5-((2,3-dihydro-1H-inden-2-yl)amino)-1,3,4-thiadiazol-2-yl)propan-1-one (44a)

White solid (30mg, yield: 59%); ¹H NMR (400 MHz, DMSO-d₆) δ 14.6(s, 1H), 7.81 (d, *J* = 4.0 Hz, 1H), 7.24 - 7.14 (m, 4H), 4.42 (m, 1H), 3.77 - 3.74 (m, 2H), 3.64 (dd, *J* = 16, 4.0 Hz, 2H), 3.39 (dd, *J* = 16, 4.0 Hz, 2H), 3.30 (dd, *J* = 24, 8.0 Hz, 2H), 3.03 (t, *J* = 8.0 Hz, 2H), 2.90 (dd, *J* = 20, 4.0 Hz, 2H), 2.74 - 2.55 (m, 2H), 2.00(m, 1H), 1.92 (m, 1H), 1.75 (m, 1H). LCMS *m*/*z* 422[M+H]⁺

### Example 40. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-chlorophenethyl)-4-methylpyrimidin-2-amine (30c)

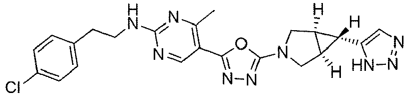

The synthesis was performed in the same manner as in Example 20, except that 2-(4-chlorophenyl)ethan-1-amine was used instead of (4-(trifluoromethyl)phenyl)methanamine and ethyl 2-chloro-4-methylpyrimidin-5-carboxylate was used instead of ethyl 2-chloropyrimidin-5-carboxylate.

### Step 1: ethyl 2-((4-chlorophenethyl)amino)-4-methylpyrimidine-5-carboxylate (27c)

Yellow solid (268 mg, yield: 86.9 %); ¹H NMR (400 MHz, CDCl₃) δ 8.80 (broad s, 1H), 7.28 (d, J = 8.4 Hz, 2H), 7.15 (d, J = 8.4 Hz, 2H), 5.41 (s, 1H), 4.32 (q, J = 7.1 Hz, 2H), 3.73 (dd, J = 13.3, 6.8 Hz, 2H), 2.89 (t, J = 7.0 Hz, 2H), 2.64 (s, 3H), 1.37 (t, J = 7.1 Hz, 3H); LCMS *m*/*z* 320[M+H]⁺

### Step 2: 2-((4-chlorophenethyl)amino)-4-methylpyrimidine-5-carbohydrazide (28c)

White solid (214 mg, yield: 83 %); ¹H NMR (400 MHz, DMSO-d₆) δ 9.33 (s, 1H), 8.24 (s, 1H), 7.47 (s, 1H), 7.33 (d, J = 8.3 Hz, 2H), 7.25 (d, J = 8.3 Hz, 2H), 4.37 (s, 2H), 3.50 (q, J = 6.8 Hz, 2H), 2.83 (t, J = 7.2 Hz, 2H), 2.38 (s, 3H); LCMS *m*/*z* 306[M+H]⁺

### Step 3: 5-(2-((4-chlorophenethyl)amino)-4-methylpyrimidin-5-yl)-1,3,4-oxadiazol-2(3H)-one (29c)

Off-white solid (41.4 mg, yield: 38 %); LCMS *m*/*z* 332[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-chlorophenethyl)-4-methylpyrimidin-2-amine (30c)

Off-white solid (20.1mg, yield: 48.5 %); ¹H NMR (400 MHz, DMSO-d₆) *δ* 15.10-14.35 (m, 1H), 8.57 (broad s, 1H), 7.87 - 7.69 (m, 1H), 7.60 - 7.46 (m, 1H), 7.33 (d, J = 8.3 Hz, 2H), 7.26 (d, J = 8.1 Hz, 2H), 3.81 (d, J = 10.1 Hz, 2H), 3.66 (d, J = 9.7 Hz, 2H), 3.61 - 3.47 (m, 2H), 2.93 - 2.78 (m, 2H), 2.55 (s, 3H), 2.08 (s, 2H), 1.95 (s, 1H); LCMS *m*/*z* 464[M+H]⁺

### Example 41. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-chlorophenethyl)pyridin-2-amine (30d)

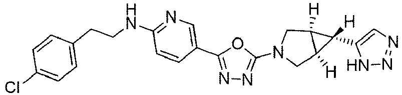

The synthesis was performed in the same manner as in Example 20, except that 2-(4-chlorophenyl)ethan-1-amine was used instead of (4-(trifluoromethyl)phenyl)methanamine and ethyl 6-chloronicotinate was used instead of ethyl 2-chloropyrimidin-5-carboxylate.

### Step 1: ethyl 6-((4-chlorophenethyl)amino)nicotinate (27d)

Off-white solid (130 mg, yield: 27.6 %); LCMS *m*/*z* 305[M+H]⁺

### Step 2: 6-((4-chlorophenethyl)amino)nicotinohydrazide (28d)

Yellow solid (60.5 mg, yield: 49 %); LCMS *m*/*z* 291[M+H]⁺

### Step 3: 5-(6-((4-chlorophenethyl)amino)pyridin-3-yl)-1,3,4-oxadiazol-2(3H)-one (29d)

Yellow solid (80.5 mg, quantitative); LCMS *m*/*z* 317[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-chlorophenethyl)pyridin-2-amine (30d)

Off-white solid (24.5 mg, yield: 61 %); ¹H NMR (400 MHz, DMSO-d₆) δ 15.17 - 14.33 (m, 1H), 8.46 (d, J = 1.8 Hz, 1H), 7.77 (dd, J = 8.9, 2.0 Hz, 1H), 7.59 (broad s, 1H), 7.34 (d, J = 8.3 Hz, 2H), 7.31 - 7.21 (m, 3H), 6.58 (d, J = 8.8 Hz, 1H), 3.80 (d, J = 10.1 Hz, 2H), 3.66 (d, J = 9.7 Hz, 2H), 3.53 (q, J = 6.4 Hz, 2H), 2.85 (t, J = 7.1 Hz, 2H), 2.09 (s, 2H), 1.99 - 1.93 (m, 1H); LCMS *m*/*z* 449[M+H]⁺

### Example 42. 5-(5-((1R,5S,6r)-6-(1H-1,2,3-Triazol-5-yl)-3-azabicyclo[3.1.0]hexan-3-yl)-1,3,4-oxadiazol-2-yl)-N-(4-chlorophenethyl)pyrazin-2-amine (30e)

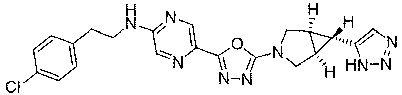

The synthesis was performed in the same manner as in Example 20, except that 2-(4-chlorophenyl)ethan-1-amine was used instead of (4-(trifluoromethyl)phenyl)methanamine and ethyl 5-chloropyrazine-2-carboxylate was used instead of ethyl 2-chloropyrimidin-5-carboxylate.

### Step 1: ethyl 5-((4-chlorophenethyl)amino)pyrazine-2-carboxylate (27e)

Brown solid (321 mg, yield: 68 %); LCMS *m*/*z* 306[M+H]⁺

### Step 2: 5-((4-chlorophenethyl)amino)pyrazine-2-carbohydrazide (28e)

White solid (224 mg, yield: 73 %); LCMS *m*/*z* 292[M+H]⁺

### Step 3: 5-(5-((4-chlorophenethyl)amino)pyrazin-2-yl)-1,3,4-oxadiazol-2(3H)-one (29e)

Off-white solid (151 mg, yield: 62 %); ¹H NMR (400 MHz, DMSO-d₆) δ 12.41 (s, 1H), 8.41 (s, 1H), 7.98 (s, 1H), 7.87 (t, J = 5.2 Hz, 1H), 7.34 (d, J = 8.1 Hz, 2H), 7.27 (d, J = 8.0 Hz, 2H), 3.57 (dd, J = 13.0, 6.6 Hz, 2H), 2.86 (t, J = 7.1 Hz, 2H); LCMS *m*/*z* 318[M+H]⁺

### Step 4: 5-(5-((1R,5S,6r)-6-(1H-1,2,3-triazol-5-yl)-3-azabicyclo[3.1.0]hexan-3-yl)-1,3,4-oxadiazol-2-yl)-N-(4-chlorophenethyl)pyrazin-2-amine (30e)

Off-white solid (24.6 mg, yield: 61 %); ¹H NMR (400 MHz, DMSO-d₆) δ 14.71 (broad s, 1H), 8.53 (d, J = 1.1 Hz, 1H), 7.98 (d, J = 1.2 Hz, 1H), 7.76 (t, J = 5.4 Hz, 1H), 7.58 (broad s, 1H), 7.35 (d, J = 8.4 Hz, 2H), 7.28 (d, J = 8.4 Hz, 2H), 3.80 (d, J = 10.1 Hz, 2H), 3.68 (d, J = 9.7 Hz, 2H), 3.56 (q, J = 6.8 Hz, 2H), 2.87 (t, J = 7.1 Hz, 2H), 2.09 (s, 2H), 2.02 - 1.92 (m, 1H); LCMS *m*/*z* 450[M+H]⁺

### Example 43. 1-((1R,5S,6r)-6-(1H-1,2,3-Triazol-5-yl)-3-azabicyclo[3.1.0]hexan-3-yl)-3-(2-((4-chlorophenethyl)amino)pyrimidin-5-yl)propan-1-one (49a)

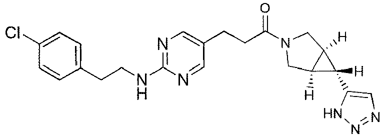

### Step 1: ethyl 3-(2-chloropyrimidin-5-yl)propanoate (46a)

Compound 21 (45a) (424 mg, 2.0 mmol) was dissolved in EtOAc (20 mL), Pd/C (300 mg) was added, and the mixture was reacted for 12 h under a hydrogen atmosphere. After completion of the reaction, the mixture was filtered through a pad of Celite, and the filtrate was concentrated to obtain compound 46a (412 mg, yield: 96%) as a white liquid.
¹H NMR (400 MHz, CDCl₃) δ 8.51 (s, 2H), 4.14 (q, *J =* 8.0 Hz, 2H), 2.94 (t, J = 8.0 Hz, 2H), 2.65 (t, *J* = 8.0 Hz, 2H),1.24 (t, *J* = 8.0 Hz, 3H); LCMS *m*/*z* 215 [M+H]⁺

### Step 2: ethyl 3-(2-((4-chlorophenethyl)amino)pyrimidin-5-yl)propanoate (47a)

A mixture of 2-(4-chlorophenyl)ethan-1-amine (348 mg, 2.23 mmol), compound 46a (400 mg, 1.86 mmol), DIEA (1.58 mL, 9.9 mmol) and n-butanol (2.5 mL) was reacted at 150 ° C for 2 h using a microwave reactor. After the reaction was completed, all the solvent was removed, distilled water was added, and extraction was performed with DCM. The organic layer was dried over Na₂SO₄, filtered and concentrated under reduced pressure. The obtained residue was purified by silica gel column chromatography (0 - 5% MeOH in CH₂Cl₂) to obtain compound 47a (68 mg, yield: 9%) as a white solid.
¹H NMR (400 MHz, DMSO-d₆) δ 8.15 (s, 2H), 7.33 (d, *J =* 8.0 Hz, 2H), 7.25 (d, *J* = 8.0 Hz, 2H), 6.98 (t, *J* = 8.0 Hz, 1H), 4.05 (q, *J* = 8.0 Hz, 2H), 3.46 (q, *J =* 8.0 Hz, 2H), 2.81 (t, *J =* 8.0 Hz, 2H), 2.63 (t, *J =* 8.0 Hz, 2H), 2.55 (t, *J =* 8.0 Hz, 2H), 1.15 (t, *J =* 8.0 Hz, 3H); LCMS *m*/*z* 334 [M+H]⁺

### Step 3: 3-(2-((4-chlorophenethyl)amino)pyrimidin-5-yl)propanoic acid (48a)

A solution of LiOH (23.3 mg, 0.97 mmol) dissolved in H₂O (387 uL) was added to a mixture of compound 47a (65 mg, 0.19 mmol) dissolved in THF/MeOH (3.4 mL/ 387 uL), and the mixture was stirred at room temperature for 3 hours. After completion of the reaction, all the solvent was removed and the pH was adjusted to 3 using 1 N HCl at 0 ° C. The resulting solid was filtered to obtain compound 48a (33 mg, 59%) as a white solid.
¹H NMR (400 MHz, DMSO-d₆) δ 8.15 (s, 2H), 7.33 (d, *J =* 8.0 Hz, 2H), 7.25 (d, *J* = 8.0 Hz, 2H), 6.96 (t, *J* = 8.0 Hz, 1H), 3.46 (q, *J =* 8.0 Hz, 2H), 2.81 (t, *J =* 8.0 Hz, 2H), 2.60 (t, *J =* 8.0 Hz, 2H), 2.47 (t, *J =* 8.0 Hz, 2H); LCMS *m*/*z* 306 [M+H]⁺

### Step 4: 1-((1R,5S,6r)-6-(1H-1,2,3-triazol-5-yl)-3-azabicyclo[3.1.0]hexan-3-yl)-3-(2-((4-chlorophenethyl)amino)pyrimidin-5-yl)propan-1-one (49a)

A reaction mixture of compound 48a (30 mg, 0.10 mmol), compound 4 (27.5 mg, 0.15 mmol), HATU (47 mg, 0.12 mmol), DIEA (60 uL, 0.35 mmol) and DCM (1.0 mL) was stirred at room temperature for 12 h. Distilled water was added to the reaction solution, and extracted with DCM. The organic layer was dried over Na₂SO₄, filtered and concentrated under reduced pressure. The obtained residue was purified by silica gel column chromatography (0-5% MeOH in CH₂Cl₂) to obtain compound 49a (40 mg, yield: 93%) as a white solid.
¹H NMR (400 MHz, DMSO-d₆) δ 14.6(s, 1H), 8.16 (s, 2H), 7.56 (s, 1H), 7.33 (d, *J* = 8.0 Hz, 2H), 7.25 (d, *J =* 8.0 Hz, 2H), 6.95 (t, *J =* 8.0 Hz, 1H), 3.72 (t, *J =* 8.0 Hz, 2H), 3.58 (dd, *J* = 16, 4.0 Hz, 1H), 3.46 (q, *J =* 8.0 Hz, 2H), 3.36 (dd, *J =* 16, 4.0 Hz, 1H),2.81 (t, *J* = 8.0 Hz, 2H), 2.60 (q, *J* = 8.0 Hz, 2H), 2.53 -2.39 (m, 2H), 2.05(m, 1H), 1.96 (m, 1H), 1.79 (m, 1H); LCMS *m*/*z* 438 [M+H]⁺

### Example 44. 1-((1R,5S,6r)-6-(1H-1,2,3-Triazol-5-yl)-3-azabicyclo[3.1.0]hexan-3-yl)-4-(2-((3,5-dichlorobenzyl)amino)pyrimidin-5-yl)butan-1-one (49b)

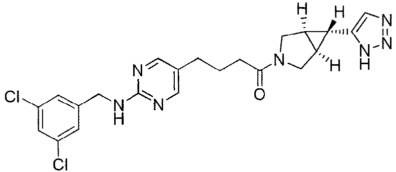

### Step 1: ethyl (E)-4-(2-chloropyrimidin-5-yl)but-3-enoate (45b)

A reaction mixture of 5-bromo-2-chloropyrimidine (400 mg, 2.1 mmol), ethyl but-3-enoate (958 mg, 8.4 mmol), Pd(OAc)₂ (19 mg, 0.084 mmol), tri(O-tolyl)phosphine (64 mg, 0.21 mmol), DIEA (2 mL) and DMF (5 mL) was reacted at 150 ° C for 4 h. After all the solvent was removed, distilled water was added, and extraction was performed with DCM. The organic layer was dried over Na₂SO₄, filtered and concentrated under reduced pressure. The obtained residue was purified by silica gel column chromatography (20 -30% EtOAc in n-hexane) to obtain compound 45b (40 mg, 8%) as a yellow solid.
¹H NMR (400 MHz, DMSO-d₆) δ 8.87 (s, 2H), 6.64 (m, 1H), 6.56 (d, *J =* 16 Hz, 1H), 4.12 (q, *J =* 8.0 Hz, 2H), 3.35 (d, *J =* 8.0 Hz, 2H), 1.21 (t, *J =* 8.0 Hz, 3H); LCMS m/z 227 [M+H]⁺

### Step 2: ethyl 4-(2-chloropyrimidin-5-yl)butanoate (46b)

The synthesis was performed in the same manner as Step 1 of Example 43.

Yellow liquid (30 mg, yield: 74%); ¹H NMR (400 MHz, DMSO-d₆) δ 8.66 (s, 2H), 4.04 (q, *J =* 8.0 Hz, 2H), 2.63 (t, *J =* 8.0 Hz, 2H), 2.32 (t, *J =* 8.0 Hz, 2H), 1.86 (m, 2H), 1.17 (t, *J =* 8.0 Hz, 3H); LCMS *m*/*z* 229 [M+H]⁺

### Step 3: ethyl 4-(2-((3,5-dichlorobenzyl)amino)pyrimidin-5-yl)butanoate (47b)

The synthesis was performed in the same manner as step 2 of Example 43, except that (3,5-dichlorophenyl)methanamine was used instead of 2-(4-chlorophenyl)ethan-1-amine.

Yellow liquid (15mg, yield: 31%); ¹H NMR (400 MHz, DMSO-d₆)δ8.14 (s, 2H), 7.58 (m, 1H), 7.43 (m, 1H), 7.33 (d, *J =* 4.0 Hz, 2H), 4.48 (d, *J =* 8.0 Hz, 2H), 4.02 (q, *J =* 8.0 Hz, 2H), 2.39 (t, *J =* 8.0 Hz, 2H), 2.27 (t, *J =* 8.0 Hz, 2H), 1.75 (m, 2H), 1.15 (t, *J =* 8.0 Hz, 3H); LCMS *m*/*z* 368[M+H]⁺

### Step 4: 4-(2-((3,5-dichlorobenzyl)amino)pyrimidin-5-yl)butanoic acid (48b)

The synthesis was performed in the same manner as Step 3 of Example 43.

White solid (7 mg, yield: 51%); ¹H NMR (400 MHz, DMSO-d₆) δ 8.15 (s, 2H), 7.57 (m, 1H), 7.42 (m, 1H), 7.33 (d, *J =* 4.0 Hz, 2H), 4.48 (d, *J =* 8.0 Hz, 2H), 2.39 (t, *J* = 8.0 Hz, 2H), 2.19 (t, *J =* 8.0 Hz, 2H), 1.72 (m, 2H); LCMS *m*/*z* 368[M+H]⁺

### Step 5: 1-((1R,5S,6r)-6-(1H-1,2,3-triazol-5-yl)-3-azabicyclo[3.1.0]hexan-3-yl)-4-(2-((3,5-dichlorobenzyl)amino)pyrimidin-5-yl)butan-1-one (49b)

The synthesis was performed in the same manner as step 4 of Example 43.

White solid (6 mg, yield: 63%); ¹H NMR (400 MHz, DMSO-d₆) δ 14.6(s, 1H), 8.15 (s, 2H), 7.56 (m, 2H), 7.42 (m, 1H), 7.33 (d, *J* = 4.0 Hz, 2H), 4.48 (d, *J* = 8.0 Hz, 2H), 3.70 (t, *J =* 8.0 Hz, 2H), 3.59 (dd, *J =* 12, 4.0 Hz, 1H), 3.35 (dd, *J =* 16, 4.0 Hz, 1H), 2.40 (t, *J =* 8.0 Hz, 2H), 2.27 - 2.15 (m, 2H), 1.97(m, 1H), 1.89 (m, 1H), 1.77 - 1.68 (m, 3H); LCMS *m*/*z* 472[M+H]⁺

### Example 45. (E)-1-((1R,5S,6r)-6-(1H-1,2,3-Triazol-5-yl)-3-azabicyclo[3.1.0]hexan-3-yl)-3-(2-((4-chlorophenethyl)amino)pyrimidin-5-yl)prop-2-en-1-one (52a)

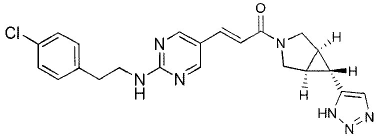

### Step 1: ethyl (E)-3-(2-((4-chlorophenethyl)amino)pyrimidin-5-yl)acrylate (50a)

A reaction mixture of 2-(4-chlorophenyl)ethan-1-amine (439 mg, 2.82 mmol), compound 21 (500 mg, 2.35 mmol), DIEA (2.0 mL, 12.5 mmol) and n-butanol (2.5 mL) were reacted in a microwave reactor at 150 ° C for 2 h. After completion of the reaction, the solvent was completely removed, distilled water was added, and the resulting solid was filtered to obtain compound 50a (500 mg, yield 53%) as a brown solid.
¹H NMR (400 MHz, DMSO-d₆) δ 8.64 (s, 2H), 7.78 (t, *J* = 8.0 Hz, 1H), 7.49 (d, *J* = 16 Hz, 1H), 7.34 (d, *J =* 8.0 Hz, 2H), 7.26 (d, *J =* 8.0 Hz, 2H), 6.52 (d, *J =* 16 Hz, 1H), 4.17 (q, *J =* 8.0 Hz, 2H), 3.55 (q, *J =* 8.0 Hz, 2H), 2.84 (t, *J =* 8.0 Hz, 2H), 1.24 (t, *J =* 8.0 Hz, 3H); LCMS *m*/*z* 332[M+H]⁺

### Step 2: (E)-3-(2-((4-chlorophenethyl)amino)pyrimidin-5-yl)acrylic acid (51a)

The synthesis was performed in the same manner as step 3 of Example 43.

White solid (120 mg, yield: 88%); ¹H NMR (400 MHz, DMSO-d₆) δ 8.60 (s, 2H), 7.73 (t, *J =* 8.0 Hz, 1H), 7.42 (d, *J =* 16 Hz, 1H), 7.34 (d, *J =* 8.0 Hz, 2H), 7.26 (d, *J =* 8.0 Hz, 2H), 6.42 (d, *J* = 16 Hz, 1H), 3.54 (q, *J =* 8.0 Hz, 2H), 2.84 (t, *J =* 8.0 Hz, 2H); LCMS *m*/*z* 304[M+H]⁺

### Step 3: (E)-1-((1R,5S,6r)-6-(1H-1,2,3-triazol-5-yl)-3-azabicyclo[3.1.0]hexan-3-yl)-3-(2-((4-chlorophenethyl)amino)pyrimidin-5-yl)prop-2-en-1-one (52a)

The synthesis was performed in the same manner as step 4 of Example 43.

White solid (17 mg, yield: 32%); ¹H NMR (400 MHz, DMSO-d₆) δ 14.6(s, 1H), 8.64 (s, 2H), 7.67 (t, *J* = 8.0 Hz, 1H), 7.61 (s, 1H), 7.34 - 7.24 (m, 5H), 6.87 (d, *J* = 16 Hz, 1H), 4.09 (d, *J =* 12 Hz, 1H), 3.88 (d, *J =* 12 Hz, 1H), 3.73 (m, 1H), 3.56 - 3.45 (m, 3H), 2.84 (t, *J =* 8.0 Hz, 2H), 2.05(m, 1H), 1.96 (m, 1H), 1.78 (m, 1H); LCMS *m*/*z* 436[M+H]⁺

### Example 46. (E)-1-((1R,5S,6r)-6-(1H-1,2,3-Triazol-5-yl)-3-azabicyclo[3.1.0]hexan-3-yl)-3-(2-((2,3-dihydro-1H-inden-2-yl)amino)pyrimidin-5-yl)prop-2-en-1-one (52b)

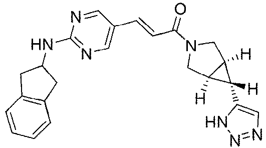

### Step 1: ethyl (E)-3-(2-((2,3-dihydro-1H-inden-2-yl)amino)pyrimidin-5-yl)acrylate (50b)

The synthesis was performed in the same manner as in Step 1 of Example 45, except that 2,3-dihydro-1H-inden-2-amine was used instead of 2-(4-chlorophenyl)ethan-1-amine.

Brown solid (260 mg, yield: 84%);¹H NMR (400 MHz, DMSO-d₆) δ 8.68 (s, 2H), 8.06 (d, *J =* 8.0 Hz, 1H), 7.51 (d, *J* = 16 Hz, 1H), 7.22 - 7.19 (m, 2H), 7.16 - 7.13 (m, 2H), 6.54 (d, *J* = 16 Hz, 1H), 4.69 - 4.64 (m, 1H), 4.18 (q, *J* = 8.0 Hz, 2H), 3.27 (dd, *J* = 16, 4.0 Hz, 2H), 2.90 (dd, *J =* 16, 4.0 Hz, 2H), 1.25 (t, *J =* 8.0 Hz, 3H); LCMS *m*/*z* 310 [M+H]⁺

### Step 2: (E)-3-(2-((2,3-dihydro-1H-inden-2-yl)amino)pyrimidin-5-yl)acrylic acid(51b)

The synthesis was performed in the same manner as step 3 of Example 43.

White solid (120 mg, yield: 89%); ¹H NMR (400 MHz, DMSO-d₆) δ 8.64 (s, 2H), 8.01 (d, *J =* 8.0 Hz, 1H), 7.45 (d, *J* = 16 Hz, 1H), 7.22 - 7.19 (m, 2H), 7.16 - 7.13 (m, 2H), 6.44 (d, *J =* 16 Hz, 1H), 4.69 - 4.64 (m, 1H), 3.29 (dd, *J =* 16, 4.0 Hz, 2H), 2.94 (dd, *J* = 16, 4.0 Hz, 2H); LCMS *m*/*z* 282[M+H]⁺

### Step 3: (E)-1-((1R,5S,6r)-6-(1H-1,2,3-triazol-5-yl)-3-azabicyclo[3.1.0]hexan-3-yl)-3-(2-((2,3-dihydro-1H-inden-2-yl)amino)pyrimidin-5-yl)prop-2-en-1-one (52b)

The synthesis was performed in the same manner as step 4 of Example 43.

Yellow solid (18 mg, yield: 36%); ¹H NMR (400 MHz, DMSO-d₆) δ 14.5(s, 1H), 8.66 (s, 2H), 7.94 (d, *J* = 8.0 Hz, 1H), 7.59 (s, 1H), 7.36 (d, *J* = 16 Hz, 1H), 7.22 - 7.19 (m, 2H), 7.16 - 7.13 (m, 2H), 6.89 (d, *J =* 16 Hz, 1H), 4.71 -4.62 (m, 1H), 4.09 (d, *J =* 12 Hz, 1H), 3.89 (d, *J* = 12 Hz, 1H), 3.75 (dd, *J* = 16, 4.0 Hz, 1H), 3.49 (dd, *J =* 16, 4.0 Hz, 1H), 3.29 (dd, *J =* 16, 4.0 Hz, 2H), 2.94 (dd, *J =* 16, 4.0 Hz, 2H), 2.05(m, 1H), 1.96 (m, 1H), 1.79 (m, 1H); LCMS *m*/*z* 414[M+H]⁺

### Example 47. N-(4-Chlorophenethyl)-5-(5-((1R,5S,6r)-6-(1-methyl-1H-1,2,3-triazol-5-yl)-3-azabicyclo[3.1.0]hexan-3-yl)-1,3,4-oxadiazol-2-yl)pyrimidin-2-amine (55)

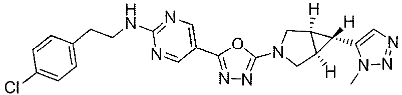

### Step 1: tert-butyl (1R,5S,6r)-6-(1-methyl-1H-1,2,3-triazol-5-yl)-3-azabicyclo[3.1.0]hexane-3-carboxylate (53)

Compound 3 (0.150 g, 0.599 mmol) was dissolved in DMF (6.0 mL), and NaH (60%, dispersed in mineral oil) (28.8 mg, 0.719 mmol) was added at 0 °C and stirred for 30 minutes. After that, iodomethane (CH ₃ I) (37 µL, 0.599 mmol) was added at the same temperature and stirred at room temperature for 15 hours. Distilled water was added to the reaction mixture, extracted with EtOAc, and washed several times with brine. The organic layer was dried over Na₂SO₄, filtered and concentrated. The obtained residue was purified by silica gel column chromatography (0-1% MeOH in DCM) to obtain compound 53 (52 mg, 32.8%) as a yellow oil.
¹H NMR (400 MHz, DMSO-d₆) δ 7.51 (s, 1H), 4.03 (s, 3H), 3.56 (d, *J* = 11.0 Hz, 2H), 1.87 (t, *J* = 2.7 Hz, 2H), 1.68 (t, *J* = 3.5 Hz, 1H), 1.39 (s, 9H)

### Step 2: (1R,5S,6r)-6-(1-methyl-1H-1,2,3-triazol-5-yl)-3-azabicyclo[3.1.0]hexane hydrochloride (54)

To a mixture of compound 53 (50 mg, 0.189 mmol) dissolved in 1,4-dioxane (0.7 mL), 4 M HCl (in dioxane, 0.47 mL, 1.89 mmol) was slowly added and stirred at room temperature for 3 hours. The reaction mixture was concentrated under reduced pressure to obtain compound 54 (30 mg, 79.0%) as a white solid, which was used in the next reaction without further purification.
¹H NMR (400 MHz, DMSO-d₆) δ 9.00 (s, 1H), 7.54 (s, 1H), 4.05 (s, 3H), 3.42 (d, J = 11.7 Hz, 2H), 3.33 (d, J = 13.0 Hz, 2H), 2.18 (t, J = 3.7 Hz, 1H), 2.06 (d, J = 2.5 Hz, 2H)

### Step 3: N-(4-chlorophenethyl)-5-(5-((1R,5S,6r)-6-(1-methyl-1H-1,2,3-triazol-5-yl)-3-azabicyclo[3.1.0]hexan-3-yl)-1,3,4-oxadiazol-2-yl)pyrimidin-2-amine (55)

A mixture of compound 13e (38 mg, 0.120 mmol) and compound 54 (28.8 mg, 0.144 mmol) dissolved in DMF (0.6 mL) was cooled to 0 °C, DIEA (61 µL, 0.359 mmol) was added, and the mixture was stirred for 30 min. BOP reagent (63.9 mg, 0.144 mmol) was added at the same temperature, and the mixture was stirred at room temperature for 15 h. Distilled water was added to the reaction mixture, extracted with EtOAc, and washed several times with brine. The organic layer was dried over Na₂SO₄, filtered and concentrated. The resulting residue was purified by silica gel column chromatography (0-1% MeOH in DCM) to obtain compound 55 (26 mg, 46.9%) as a white solid.
¹H NMR (400 MHz, DMSO-d₆) δ 8.69 (s, 2H), 7.92 (t, J = 5.7 Hz, 1H), 7.54 (s, 1H), 7.35 - 7.32 (m, 2H), 7.26 (d, J = 8.5 Hz, 2H), 4.05 (s, 3H), 3.81 (d, J = 10.2 Hz, 2H), 3.66 (d, J = 10.3 Hz, 2H), 3.56 (dd, J = 13.7, 6.5 Hz, 2H), 2.86 (t, J = 7.2 Hz, 2H), 2.07 (s, 2H), 1.91 (d, J = 2.0 Hz, 1H); LCMS *m*/*z* 464 [M+H]⁺

### Example 48. N-(4-Chlorophenethyl)-5-(5-((1R,5S,6r)-6-(4-methyl-1H-1,2,3-triazol-5-yl)-3-azabicyclo[3.1.0]hexan-3-yl)-1,3,4-oxadiazol-2-yl)pyrimidin-2-amine (59)

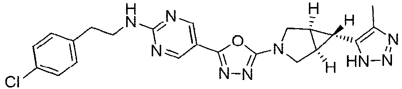

### Step 1: tert-butyl (1R,5S,6s)-6-(prop-1-yn-1-yl)-3-azabicyclo[3.1.0]hexane-3-carboxylate (56)

Compound 2 (400 mg, 1.93 mmol) was dissolved in THF (6 mL), and the mixture was maintained at -78 °C under a nitrogen atmosphere. 2.5 M n-BuLi (0.77 mL, 1.93 mmol) was added dropwise over 10 minutes, and then stirred for 30 minutes. The reaction temperature was raised to room temperature, and CH₃I (181 uL, 2.9 mmol) was added, and the reaction was carried out for 12 hours. Distilled water was added to the reaction mixture, and then extracted with EtOAc. The organic layer was dried over Na₂SO₄, filtered, and concentrated under reduced pressure. The obtained residue was purified by silica gel column chromatography (10% EtOAc in n-hexane) to obtain compound 56 (390 mg, yield: 91%) as a white solid.
¹H NMR (400 MHz, CDCl₃) δ 3,63 - 3.52 (m, 2H), 3.35 - 3.33 (m, 2H), 1.83 - 1.81 (m, 2H), 1.76 (s, 3H), 1.43 (s, 9H), 1.12 - 1.09 (m, 1H)

### Step 2: tert-butyl (1R,5S,6r)-6-(4-methyl-1H-1,2,3-triazol-5-yl)-3-azabicyclo[3.1.0]hexane-3-carboxylate (57)

A mixture of compound 56 (200 mg, 0.9 mmol) and TMSN₃ (180 uL, 1.39 mmol) was reacted in a microwave reactor at 200 ° C for 7 h. After the solvent was concentrated under reduced pressure, the resulting residue was purified by silica gel column chromatography (0 - 5% MeOH in DCM) to give compound 57 (32 mg, 13%) as a yellow solid.
¹H NMR (400 MHz, CDCl₃) δ 10.99 (s, 1H), 3.79 - 3.67 (m, 2H), 3.48 - 3.46 (m, 2H), 2.32 (s, 3H), 2.03 - 1.95 (m, 2H), 1.63 -1.61 (m, 1H), 1.47 (s, 9H)

### Step 3: (1R,5S,6r)-6-(4-methyl-1H-1,2,3-triazol-5-yl)-3-azabicyclo[3.1.0]hexane hydrochloride (58)

To a reaction mixture of compound 57 (30 mg, 0.11 mmol) dissolved in 1,4-dioxane (1 mL), 4M HCl in dioxane (282 uL, 1.1 mmol) was added. After reacting at room temperature for 3 hours, the solvent was concentrated to obtain compound 58 (20 mg, yield: 91%) as a brown solid, which was used in the next reaction without further purification.
¹H NMR (400 MHz, CD₃OD)δ 3.66 - 3.57 (m, 4H), 2.40 (s, 3H), 2.35 (m, 2H), 2.15 (m, 1H)

### Step 4: N-(4-chlorophenethyl)-5-(5-((1R,5S,6r)-6-(4-methyl-1H-1,2,3-triazol-5-yl)-3-azabicyclo[3.1.0]hexan-3-yl)-1,3,4-oxadiazol-2-yl)pyrimidin-2-amine (59)

Compound 58 (20 mg, 0.1 mmol) was added to the reaction mixture in which compound 13e (25 mg, 0.08 mmol) was dissolved in DMF (1.5 mL) and cooled to 0°C. BOP reagent (44 mg, 0.1 mmol) and DIPEA (87 µL, 0.5 mmol) were added, and the mixture was stirred at room temperature for 5 h. Distilled water was added to the reaction mixture, extracted with EtOAc, and the organic layer was dried over Na₂SO₄, filtered, and concentrated under reduced pressure. The obtained residue was purified by silica gel column chromatography (0-5% MeOH in DCM) to obtain compound 59 (13 mg, yield: 35%) as a yellow solid.
¹ H NMR (400 MHz, DMSO-d₆) δ 14.1(s, 1H), 8.69 (s, 2H), 7.91 (m, 1H), 7.35 (d, *J* = 8.0 Hz, 2H), 7.27 (d, *J* = 8.0 Hz, 2H), 3.83 (d, *J* = 8.0 Hz, 2H), 3.68 (d, *J* = 8.0 Hz, 2H), 3.58 (q, *J* = 8.0 Hz, 2H), 2.86 (t, *J* = 8.0 Hz, 2H), 2.23 (s, 3H), 2.08(m, 2H), 1.84 (m, 1H); LCMS *m*/*z* 464 [M+H]⁺

### Example 49. 5-(3-((1R,5S,6r)-6-(1H-1,2,3-Triazol-5-yl)-3-azabicyclo[3.1.0]hexan-3-yl)-4,5-dihydroisoxazol-5-yl)-N-(4-chlorophenethyl)pyrimidin-2-amine (63a)

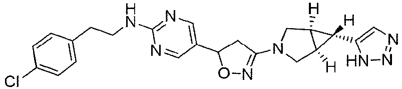

### Step 1: N-(4-chlorophenethyl)-5-vinylpyrimidin-2-amine (61a)

A reaction mixture of 2-(4-chlorophenyl)ethan-1-amine (0.3 g, 1.93 mmol), 2-chloro-5-vinylpyrimidine (0.542 g, 3.86 mmol), DIPEA (6.6 mL, 38.6 mmol), and n-butanol (3.9 mL) was reacted in a microwave reactor at 150 °C for 2 h. The reaction mixture was concentrated under reduced pressure, and the obtained residue was purified by silica gel column chromatography (10-30% EA in Hex) to obtain compound 61a (0.315 g, 62.9%) as a white solid.
¹H NMR (400 MHz, DMSO-d₆) δ 8.41 (s, 2H), 7.35 - 7.31 (m, 3H), 7.27 - 7.23 (m, 2H), 6.56 - 6.46 (m, 1H), 5.69 (dd, J = 17.8, 0.9 Hz, 1H), 5.08 (dd, J = 11.2, 0.9 Hz, 1H), 3.54 - 3.43 (m, 2H), 2.83 (t, J = 7.3 Hz, 2H); LCMS *m*/*z* 260[M+H]⁺

### Step 2: 5-(3-bromo-4,5-dihydroisoxazol-5-yl)-N-(4-chlorophenethyl)pyrimidin-2-amine (62a)

1,1-Dibromoformaldoxime (0.484 g, 2.39 mmol) was dissolved in DMF (3.0 mL), cooled to -10 °C, and compound 61a (0.31 g, 1.18 mmol) was added. A solution of KHCO₃ (0.299 g, 2.98 mmol) in H₂O (3.0 mL) was slowly added to the reaction mixture, and the mixture was stirred at room temperature for 1 h. After completion of the reaction, distilled water was added, extracted with EtOAc, dried over Na₂SO₄, filtered, and concentrated. The obtained residue was purified by silica gel column chromatography (0-1% MeOH in DCM) to give compound 62a (0.41 g, 90.0%) as a yellow solid.
¹H NMR (400 MHz, DMSO-d₆) δ 8.33 (s, 2H), 7.43 (t, J = 5.7 Hz, 1H), 7.34 - 7.31 (m, 2H), 7.25 (d, J = 8.4 Hz, 2H), 5.55 (t, J = 10.4 Hz, 1H), 3.61 (dd, J = 17.5, 10.6 Hz, 1H), 3.51 - 3.48 (m, 2H), 3.47 - 3.42 (m, 1H), 2.82 (t, J = 7.2 Hz, 2H); LCMS *m*/*z* 381[M+H]⁺

### Step 3: 5-(3-((1R,5S,6r)-6-(1H-1,2,3-triazol-5-yl)-3-azabicyclo[3.1.0]hexan-3-yl)-4,5-dihydroisoxazol-5-yl)-N-(4-chlorophenethyl)pyrimidin-2-amine (63a)

A reaction mixture of compound 62a (80 mg, 0.210 mmol), compound 4 (47.0 mg, 0.252 mmol), DIEA (356 µL, 2.10 mmol), and t-butanol (4.2 mL) was reacted in a microwave reactor at 200 °C for 3 h. The reaction mixture was filtered and concentrated, and the obtained residue was purified by silica gel column chromatography (0-1% MeOH in DCM) to obtain compound 63a (9.9 mg, 10.5%) as a yellow solid.
¹H NMR (400 MHz, DMSO-d₆) δ 8.29 (s, 2H), 7.33 (d, *J* = 8.4 Hz, 2H), 7.25 (d, *J* = 8.5 Hz, 2H), 5.25 - 5.19 (m, 1H), 4.04 (tdd, *J* = 7.1, 4.8, 2.7 Hz, 1H), 3.49 (s, 2H), 3.40 (t, *J* = 9.2 Hz, 2H), 3.36 - 3.31 (m, 1H), 3.17 (d, *J* = 5.3 Hz, 2H), 2.86 - 2.81 (m, 2H), 1.98 (d, *J* = 5.5 Hz, 2H), 1.92 - 1.87 (m, 2H); LCMS *m*/*z* 451[M+H]⁺

### Example 50. 5-(3-((1R,5S,6r)-6-(1H-1,2,3-Triazol-5-yl)-3-azabicyclo[3.1.0]hexan-3-yl)-4,5-dihydroisoxazol-5-yl)-N-(2,3-dihydro-1H-inden-2-yl)pyrimidin-2-amine (63b)

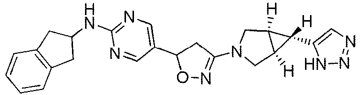

The synthesis was performed in the same manner as Example 49, except that 2,3-dihydro-1H-inden-2-amine was used instead of 2-(4-chlorophenyl)ethan-1-amine.

### Step 1: N-(2,3-dihydro-1H-inden-2-yl)-5-vinylpyrimidin-2-amine (61b)

Yellow solid (281 mg, yield: 78.9%); ¹H NMR (400 MHz, DMSO-d₆) δ 8.45 (s, 2H), 7.59 (d, *J* = 6.8 Hz, 1H), 7.20 (dt, *J* = 7.2, 3.5 Hz, 2H), 7.16 - 7.12 (m, 2H), 6.54 (dd, *J* = 17.8, 11.2 Hz, 1H), 5.71 (dd, *J* = 17.8, 0.8 Hz, 1H), 5.10 (dd, *J =* 11.2, 0.8 Hz, 1H), 4.67 - 4.57 (m, 1H), 3.24 (dd, *J* = 15.8, 7.6 Hz, 2H), 2.89 (dd, *J* = 15.7, 6.9 Hz, 2H); LCMS *m*/*z* 238[M+H]⁺

### Step 2: 5-(3-bromo-4,5-dihydroisoxazol-5-yl)-N-(2,3-dihydro-1H-inden-2-yl)pyrimidin-2-amine (62b)

White solid (249 mg, yield: 59%); ¹H NMR (400 MHz, DMSO-d₆) δ 8.36 (s, 2H), 7.70 (d, *J* = 6.9 Hz, 1H), 7.20 (dt, *J* = 7.1, 3.5 Hz, 2H), 7.15 - 7.12 (m, 2H), 5.56 (t, *J =* 10.4 Hz, 1H), 4.61 (dt, *J* = 14.2, 7.1 Hz, 1H), 3.62 (dd, *J* = 17.5, 10.6 Hz, 1H), 3.51 - 3.44 (m, 1H), 3.26 - 3.21 (m, 2H), 2.91 (d, *J* = 6.9 Hz, 2H); LCMS *m*/*z* 359[M+H]⁺

### Step 3: 5-(3-((1R,5S,6r)-6-(1H-1,2,3-triazol-5-yl)-3-azabicyclo[3.1.0]hexan-3-yl)-4,5-dihydroisoxazol-5-yl)-N-(2,3-dihydro-1H-inden-2-yl)pyrimidin-2-amine (63b)

Yellow solid (4.3 mg, yield: 24%); ¹H NMR (400 MHz, DMSO-d₆) δ 8.33 (s, 2H), 7.54 (d, *J* = 6.9 Hz, 1H), 7.21 (dd, *J* = 5.3, 3.3 Hz, 2H), 7.15 - 7.12 (m, 2H), 5.24 (t, *J* = 9.3 Hz, 1H), 4.61 (dd, *J* = 14.4, 7.2 Hz, 1H), 3.51 (d, *J* = 10.2 Hz, 2H), 3.40 (t, *J* = 9.4 Hz, 2H), 3.34 (dd, *J* = 15.8, 9.2 Hz, 1H), 3.27 - 3.25 (m, 2H), 3.23 - 3.20 (m, 2H), 3.18 - 3.13 (m, 1H), 2.88 (d, *J* = 8.9 Hz, 2H), 1.98 (s, 2H), 1.90 (d, *J* = 3.5 Hz, 1H); LCMS *m*/*z* 429[M+H]⁺

### Example 51. 2-((1R,5S,6s)-3-(5-(2-((4-Chlorophenethyl)amino)pyrimidin-5-yl)-1,3,4-oxadiazol-2-yl)-3-azabicyclo[3.1.0]hexan-6-yl)acetic acid (68)

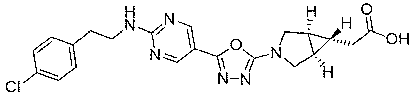

### Step 1: tert-butyl (1R,5S,6s)-6-(2-methoxy-2-oxoethyl)-3-azabicyclo[3.1.0]hexane-3-carboxylate (65)

2-((1R,5S,6s)-3-(tert-butoxycarbonyl)-3-azabicyclo[3.1.0]hexan-6-yl)acetic acid (0.1 g, 0.414 mmol) was dissolved in DMF (2.1 mL), and K₂CO₃ (57.3 mg, 0.414 mmol) and CH₃I (31 µL, 0.497 mmol) were added sequentially, and the mixture was stirred at room temperature for 15 h. Distilled water was added to the reaction mixture, extracted with EtOAc, and washed several times with brine. The organic layer was dried over Na₂SO₄, filtered and concentrated to give compound 65 (96 mg, yield: 96.7%) as a yellow oil.
¹H NMR (400 MHz, DMSO-d₆) δ 3.60 (s, 3H), 3.43 - 3.38 (m, 2H), 3.22 (d, J = 13.6 Hz, 2H), 2.31 - 2.28 (m, 2H), 1.41 - 1.38 (m, 2H), 1.38 - 1.36 (m, 9H), 0.69 - 0.64 (m, 1H)

### Step 2: methyl 2-((1R,5S,6s)-3-azabicyclo[3.1.0]hexan-6-yl)acetate hydrochloride (66)

Compound 65 (95 mg, 0.372 mmol) was dissolved in 1,4-dioxane (1.4 mL), 4 M HCl (in dioxane, 0.93 mL, 37.2 mmol) was slowly added, and the mixture was stirred at room temperature for 5 hours. The reaction mixture was concentrated under reduced pressure to obtain compound 66 (71 mg, quantitative) as a yellow solid, which was used in the next reaction without further purification.
¹H NMR (400 MHz, DMSO-d₆) δ 9.17 (s, 1H), 3.60 (s, 3H), 3.24 (d, J = 3.0 Hz, 4H), 2.32 (d, J = 7.0 Hz, 2H), 1.62 (s, 2H), 1.20 - 1.15 (m, 1H)

### Step 3: methyl 2-((1R,5S,6s)-3-(5-(2-((4-chlorophenethyl)amino)pyrimidin-5-yl)-1,3,4-oxadiazol-2-yl)-3-azabicyclo[3.1.0]hexan-6-yl)acetate (67)

Compound 13e (80 mg, 0.252 mmol) and compound 66 (57.9 mg, 0.302 mmol) were dissolved in DMF (1.3 mL). The reaction mixture was cooled to 0°C, DIEA (128 µL, 0.756 mmol) was added, and stirred for 30 minutes. BOP reagent (134 mg, 0.302 mmol) was added at the same temperature, and the mixture was stirred at room temperature for 15 hours. Distilled water was added to the reaction mixture, extracted with EtOAc, and washed several times with brine. The organic layer was dried over Na₂SO₄, filtered, and concentrated. The resulting residue was purified by silica gel column chromatography (0-1% MeOH in DCM) to give compound 67 (80 mg, yield: 69.8%) as a yellow solid.
¹H NMR (400 MHz, DMSO-d₆) δ 8.73 - 8.62 (m, 2H), 7.93 - 7.86 (m, 1H), 7.35 - 7.31 (m, 2H), 7.27 - 7.24 (m, 2H), 3.64 (d, *J* = 10.0 Hz, 2H), 3.61 (s, 3H), 3.54 (t, *J* = 5.9 Hz, 4H), 2.85 (t, *J* = 7.2 Hz, 2H), 2.36 (d, *J* = 7.1 Hz, 2H), 1.61 (s, 2H), 0.91 - 0.83 (m, 1H); LCMS *m*/*z* 455[M+H]⁺

### Step 4: 2-((1R,5S,6s)-3-(5-(2-((4-chlorophenethyl)amino)pyrimidin-5-yl)-1,3,4-oxadiazol-2-yl)-3-azabicyclo[3.1.0]hexan-6-yl)acetic acid (68)

Compound 67 (40 mg, 0.088 mmol) was dissolved in THF (0.44 mL), 1 N NaOH (220 µL, 0.220 mmol) was added, and the mixture was stirred at room temperature for 1.5 h. The reaction mixture was concentrated, EtOAc was added, and the mixture was stirred for 1 h. The resulting solid was filtered to obtain compound 68 (30 mg, 77.4%) as a yellow solid.
¹H NMR (400 MHz, DMSO-d₆) δ 8.67 (s, 2H), 7.89 (t, J = 5.8 Hz, 1H), 7.33 (d, J = 8.4 Hz, 2H), 7.26 (d, J = 8.4 Hz, 2H), 3.63 - 3.47 (m, 6H), 2.89 - 2.81 (m, 2H), 1.87 (d, J = 6.7 Hz, 2H), 1.44 (s, 2H), 0.81 (tt, J = 7.3, 3.8 Hz, 1H); LCMS *m*/*z* 441[M+H]⁺

### Test example 1. Evaluation of the inhibitory activity against human Autotaxin protein

### (1) Experimental method

Solutions of each synthesized compound (80 uM, 100% dimethyl sulfoxide) were sequentially diluted 5-fold with dimethyl sulfoxide to prepare 6 concentrations. Each concentration of the compound was diluted 2-fold with 1x test buffer (50 mM Tris-Cl (pH 8.0), 5 mM KCl, 1 mM CaCl₂, 1 mM MgCl₂, 140 mM NaCl, deionized water, 1 mg/mL BSA), and 1 uL (1.25% dimethylsulfoxide) of the diluted mixture was dispensed into each well of a 96-well clear round-bottom plate. 9 uL of 1x test buffer was added, and 20 uL of 240 nM human autotaxin protein (buffer: 50 mM Tris-HCl, pH 8.0, with 150 mM sodium chloride and 20% glycerol) was added. 10 uL of sonicated 360 uM 18:1 LysoPC (diluted with 1x assay buffer) was added. The reaction was performed for 2 hours in a shaking incubator at 37 °C, and a secondary reaction mixture (choline assay kit, KA1662) (65 uL of 1x assay buffer: 1 uL of choline oxidase: 1 uL of dye probe) was prepared. 60 uL of the secondary reaction mixture was added to the plate where the reaction was performed, and incubated for 30 minutes in a shaker. The absorbance value was measured at a wavelength of 570 nm using a SpectraMax iD3 microplate reader. The percentage inhibition activity value (% inhibition) was calculated by the formula: (1 - absorbance value _{test group} / absorbance value _{control group}) X 100.

### (2) Results

The activity inhibition rates measured as above are shown in Table 1 below.

**[Table 1]**

| **Example** | **Compound number** | **Inhibition rate (%, at 500nM)** |
|---|---|---|
| 1 | 9a | 63.6 |
| 2 | 9b | 88.5 |
| 3 | 9c | 73.1 |
| 4 | 9d | 81.5 |
| 5 | 9e | 86.4 |
| 6 | 9f | 68.9 |
| 7 | 9g | 92.0 |
| 8 | 9h | 97.3 |
| 9 | 9i | 40.6 |
| 10 | 9j | 91.6 |
| 11 | 14a | 85.3 |
| 12 | 14b | 82.9 |
| 13 | 14c | 86.0 |
| 14 | 14d | 85.3 |
| 15 | 14e | 94.5 |
| 16 | 19a | 98.6 |
| 17 | 19b | 96.7 |
| 18 | 19c | 87.6 |
| 19 | 25 | 91.9 |
| 20 | 9k | 88.5 |
| 21 | 91 | 97.6 |
| 22 | 9m | 98.4 |
| 23 | 9n | 82.2 |
| 24 | 9o | 66.3 |
| 25 | 9p | 95.2 |
| 26 | 14f | 98.4 |
| 27 | 14g | 98.6 |
| 28 | 14h | 96.4 |
| 29 | 14i | 97.0 |
| 30 | 14j | 95.7 |
| 31 | 14k | 97.0 |
| 32 | 30a | 48.7 |
| 33 | 14l | 93.7 |
| 34 | 35a | 90.8 |
| 35 | 35b | 96.8 |
| 36 | 30b | 65.2 |
| 37 | 39 | 91.7 |
| 38 | 44b | 25.5 |
| 39 | 44a | 59.7 |
| 40 | 30c | 92.3 |
| 41 | 30d | 95.8 |
| 42 | 30e | 68.4 |
| 43 | 49a | 84.8 |
| 44 | 49b | 58.6 |
| 45 | 52a | 98.7 |
| 46 | 52b | 99.4 |
| 47 | 55 | 13.9 |
| 48 | 59 | 34.7 |
| 49 | 63a | 76.4 |
| 50 | 63b | 95.5 |
| 51 | 68 | 3.5 |

All of the synthesized compounds Example 1 to Example 51 exhibited excellent inhibitory activity against human Autotaxin protein.

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. An azabicycle derivative compound represented by the following Formula 1, a hydrate thereof, a solvate thereof, or a pharmaceutically acceptable salt thereof, wherein: 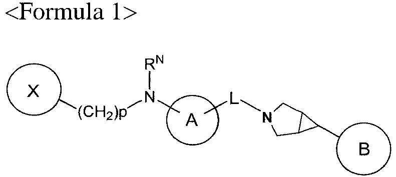
X is C₅₋₇ aryl; 5- or 6-membered heteroaryl having 1 to 3 N; or a fused bicyclic ring in which C₅₋₇ aryl ring is fused with a 5- or 6-membered cycloalkyl ring having 0 to 3 O, wherein X is optionally substituted with one or more independent R^{x} ,
wherein R ^{x} is C₁₋₄ alkyl, C₁₋₄ alkoxy, cyano, halogen or C₁₋₄ haloalkyl,
p is an integer from 0 to 3,
R^{N} is hydrogen or C₁₋₄ alkyl,
A is a 5- or 6-membered heteroaryl having 1 to 3 heteroatoms selected from N and S, wherein A is optionally substituted with one or more independent R^{A},
wherein R^{A} is C₁₋₄ alkyl,
L is -(CH₂)ₘC(O)-, -CHCHC(O)-, or a 5- or 6-membered aromatic or non-aromatic heterocycle having 1 to 4 heteroatoms selected from N and O, wherein m is an integer from 1 to 5, and
B is -(CH₂)ₙC(O)OH, or a 5- or 6-membered heteroaryl having 1 to 4 N, wherein n is an integer from 1 to 3, wherein B is optionally substituted with one or more independent R^{B},
wherein R^{B} is C₁₋₄ alkyl.

2. The compound, a hydrate thereof, a solvate thereof or a pharmaceutically acceptable salt thereof of Claim 1, wherein X is phenyl, pyridinyl, dihydroindenyl or benzodioxolyl.

3. The compound, a hydrate thereof, a solvate thereof or a pharmaceutically acceptable salt thereof of Claim 1, wherein R^{x} is methyl, methoxy, cyano, fluoro, chloro, bromo, difluoromethyl or trifluoromethyl.

4. The compound, a hydrate thereof, a solvate thereof or a pharmaceutically acceptable salt thereof of Claim 1, wherein p is 0, 1 or 2.

5. The compound, a hydrate thereof, a solvate thereof or a pharmaceutically acceptable salt thereof of Claim 1, wherein R^{N} is hydrogen or methyl.

6. The compound, a hydrate thereof, a solvate thereof or a pharmaceutically acceptable salt thereof of Claim 1, wherein A is pyridinyl, pyrimidinyl, pyrazinyl or thiadiazolyl.

7. The compound, a hydrate thereof, a solvate thereof or a pharmaceutically acceptable salt thereof of Claim 1, wherein R^{A} is methyl.

8. The compound, a hydrate thereof, a solvate thereof or a pharmaceutically acceptable salt thereof of Claim 1, wherein L is -(CH₂)₂C(O)-, -(CH₂)₃C(O)-, -CHCHC(O)-, dihydroisoxazolyl or oxadiazolyl.

9. The compound, a hydrate thereof, a solvate thereof or a pharmaceutically acceptable salt thereof of Claim 1, wherein B is -CH₂C(O)OH or triazolyl.

10. The compound, a hydrate thereof, a solvate thereof or a pharmaceutically acceptable salt thereof of Claim 1, wherein R^{B} is methyl.

11. The compound, a hydrate thereof, a solvate thereof or a pharmaceutically acceptable salt thereof of Claim 1wherein the compound represented by the Formula 1 is any one selected from the group consisting of:
[1] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-benzylpyrimidin-2-amine,
[2] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-chlorobenzyl)pyrimidin-2-amine,
[3] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorobenzyl)pyrimidin-2-amine,
[4] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,4-difluorobenzyl)pyrimidin-2-amine,
[5] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-difluorobenzyl)pyrimidin-2-amine,
[6] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,4-dichlorobenzyl)pyrimidin-2-amine,
[7] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-dichlorobenzyl)pyrimidin-2-amine,
[8] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-dibromobenzyl)pyrimidin-2-amine,
[9] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(benzo[*d*][1,3]dioxol-5-ylmethyl)pyrimidin-2-amine,
[10] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-bis(trifluoromethyl)benzyl)pyrimidin-2-amine,
[11] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-phenethylpyrimidin-2-amine,
[12] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-fluorophenethyl)pyrimidin-2-amine,
[13] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-fluorophenethyl)pyrimidin-2-amine,
[14] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-chlorophenethyl)pyrimidin-2-amine,
[15] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)pyrimidin-2-amine,
[16] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2,3-dihydro-1*H*-inden-2-yl)pyrimidin-2-amine,
[17] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(5,6-difluoro-2,3-dihydro-1*H*-inden-2-yl)pyrimidin-2-amine,
[18] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(5-bromo-2,3-dihydro-1*H*-inden-2-yl)pyrimidin-2-amine,
[19] 1-((1*R*,5*S*,6*r*)-6-(1*H*-1,2,3-Triazol-5-yl)-3-azabicyclo[3.1.0]hexan-3-yl)-3-(2-((2,3-dihydro-1*H*-inden-2-yl)amino)pyrimidin-5-yl)propan-1-one,
[20] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-(trifluoromethyl)benzyl)pyrimidin-2-amine,
[21] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3-(trifluoromethyl)benzyl)pyrimidin-2-amine,
[22] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2-(trifluoromethyl)benzyl)pyrimidin-2-amine,
[23] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-(difluoromethyl)benzyl)pyrimidin-2-amine,
[24] 4-(((5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)pyrimidin-2-yl)amino)methyl)benzonitrile,
[25] 3-(((5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)pyrimidin-2-yl)amino)methyl)benzonitrile,
[26] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-(trifluoromethyl)phenethyl)pyrimidin-2-amine,
[27] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-bromophenethyl)pyrimidin-2-amine,
[28] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2-chlorophenethyl)pyrimidin-2-amine,
[29] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(3,5-dichlorophenethyl)pyrimidin-2-amine,
[30] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-methoxyphenethyl)pyrimidin-2-amine,
[31] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-methylphenethyl)pyrimidin-2-amine,
[32] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(2-(pyridin-2-yl)ethyl)pyrimidin-2-amine,
[33] 4-(2-((5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)pyrimidin-2-yl)amino)ethyl)benzonitrile,
[34] 5-(5-((1*R*,5*S*,6*R*)-6-(1*H*-1,2,3-Triazol-5-yl)-3-azabicyclo[3.1.0]hexan-3-yl)-1,3,4-oxadiazol-2-yl)-*N*-((R)-2,3-dihydro-1*H*-inden-1-yl)pyrimidin-2-amine,
[35] 5-(5-((1*R*,5*S*,6*S*)-6-(1*H*-1,2,3-Triazol-5-yl)-3-azabicyclo[3.1.0]hexan-3-yl)-1,3,4-oxadiazol-2-yl)-*N*-((*S*)-2,3-dihydro-1*H*-inden-1-yl)pyrimidin-2-amine,
[36] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-phenylpyrimidin-2-amine,
[37] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)-*N*-methylpyrimidin-2-amine,
[38] 1-((1*R*,5*S*,6*r*)-6-(1*H*-1,2,3-Triazol-5-yl)-3-azabicyclo[3.1.0]hexan-3-yl)-4-(5-((2,3-dihydro-1*H*-inden-2-yl)amino)-1,3,4-thiadiazol-2-yl)butan-1-one,
[39] 1-((1*R*,5*S*,6*r*)-6-(1*H*-1,2,3-Triazol-5-yl)-3-azabicyclo[3.1.0]hexan-3-yl)-3-(5-((2,3-dihydro-1*H*-inden-2-yl)amino)-1,3,4-thiadiazol-2-yl)propan-1-one,
[40] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)-4-methylpyrimidin-2-amine,
[41] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)pyridin-2-amine,
[42] 5-(5-((1*R*,5*S*,6*r*)-6-(1*H*-1,2,3-Triazol-5-yl)-3-azabicyclo[3.1.0]hexan-3-yl)-1,3,4-oxadiazol-2-yl)-*N*-(4-chlorophenethyl)pyrazin-2-amine,
[43] 1-((1*R*,5*S*,6*r*)-6-(1*H*-1,2,3-Triazol-5-yl)-3-azabicyclo[3.1.0]hexan-3-yl)-3-(2-((4-chlorophenethyl)amino)pyrimidin-5-yl)propan-1-one,
[44] 1-((1*R*,5*S*,6*r*)-6-(1*H*-1,2,3-Triazol-5-yl)-3-azabicyclo[3.1.0]hexan-3-yl)-4-(2-((3,5-dichlorobenzyl)amino)pyrimidin-5-yl)butan-1-one,
[45] (*E*)-1-((1*R*,5*S*,6*r*)-6-(1*H*-1,2,3-Triazol-5-yl)-3-azabicyclo[3.1.0]hexan-3-yl)-3-(2-((4-chlorophenethyl)amino)pyrimidin-5-yl)prop-2-en-1-one,
[46] (*E*)-1-((1*R*,5*S*,6*r*)-6-(1*H*-1,2,3-Triazol-5-yl)-3-azabicyclo[3.1.0]hexan-3-yl)-3-(2-((2,3-dihydro-1*H*-inden-2-yl)amino)pyrimidin-5-yl)prop-2-en-1-one,
[47] *N*-(4-Chlorophenethyl)-5-(5-((1*R*,5*S*,6*r*)-6-(1-methyl-1*H*-1,2,3-triazol-5-yl)-3-azabicyclo[3.1.0]hexan-3-yl)-1,3,4-oxadiazol-2-yl)pyrimidin-2-amine,
[48] *N*-(4-Chlorophenethyl)-5-(5-((1*R*,5*S*,6*r*)-6-(4-methyl-1*H*-1,2,3-triazol-5-yl)-3-azabicyclo[3.1.0]hexan-3-yl)-1,3,4-oxadiazol-2-yl)pyrimidin-2-amine,
[49] 5-(3-((1*R*,5*S*,6*r*)-6-(1*H*-1,2,3-Triazol-5-yl)-3-azabicyclo[3.1.0]hexan-3-yl)-4,5-dihydroisoxazol-5-yl)-*N*-(4-chlorophenethyl)pyrimidin-2-amine,
[50] 5-(3-((1*R*,5*S*,6*r*)-6-(1*H*-1,2,3-Triazol-5-yl)-3-azabicyclo[3.1.0]hexan-3-yl)-4,5-dihydroisoxazol-5-yl)-*N*-(2,3-dihydro-1*H*-inden-2-yl)pyrimidin-2-amine, and
[51] 2-((1*R*,5*S*,6*s*)-3-(5-(2-((4-Chlorophenethyl)amino)pyrimidin-5-yl)-1,3,4-oxadiazol-2-yl)-3-azabicyclo[3.1.0]hexan-6-yl)acetic acid.

12. A pharmaceutical composition for preventing or treating an autotaxin-related disease, comprising an azabicycle derivative compound, a hydrate thereof, a solvate thereof, or a pharmaceutically acceptable salt thereof of any one of Claims 1 to 11, as an active ingredient.

13. The pharmaceutical composition of Claim 12, wherein the autotaxin-related disease is selected from the group consisting of fibrotic diseases, inflammatory diseases, autoimmune diseases, respiratory diseases, cardiovascular diseases, metabolic diseases, cancer and cancer metastasis, ocular diseases, cholestatic form and other forms of chronic pruritus, and acute or chronic organ transplant rejection.

14. A pharmaceutical composition comprising the azabicycle derivative compound, a hydrate thereof, a solvate thereof or a pharmaceutically acceptable salt thereof of any one of Claims 1 to 11 as an active ingredient, for preventing or treating one or more disease selected from the group consisting of a fibrotic disease selected from idiopathic pulmonary fibrosis (IPF), interstitial lung disease, liver fibrosis, liver cirrhosis, non-alcoholic steatohepatitis, radiation-induced fibrosis, renal fibrosis, cutaneous fibrosis, glomerulosclerosis, myocardial and vascular fibrosis; an inflammatory disease selected from rheumatoid arthritis, osteoarthritis, atopic dermatitis, inflammatory bowel disease, inflammatory airway disease, chronic obstructive pulmonary disease (COPD) and asthma; an autoimmune disease selected from multiple sclerosis and scleroderma; a respiratory disease selected from asbestos-induced pulmonary fibrosis and acute respiratory distress syndrome (ARDS); a cardiovascular disease selected from arteriosclerosis, myocardial infarction, arterial and pulmonary hypertension, cardiac arrhythmia, stroke and other vascular damage; a metabolic disease selected from obesity and diabetes; cancer and cancer metastasis selected from breast cancer, ovarian cancer, lung cancer, prostate cancer, mesothelioma, glioma, liver carcinoma, gastrointestinal cancer, pancreatic cancer, and its progression and metastatic invasion; an ocular disease selected from proliferative and non-proliferative retinopathy, diabetic retinopathy, dry and wet age-related macular degeneration (AMD), macular edema, central artery/venous occlusion, traumatic injury, and glaucoma; cholestatic form and other forms of chronic pruritus; and acute or chronic organ transplant rejection.

15. A pharmaceutical composition comprising the azabicycle derivative compound, a hydrate thereof, a solvate thereof or a pharmaceutically acceptable salt thereof of any one of Claims 1 to 11, and a pharmaceutically acceptable additive.
